# EUROPEAN PATENT APPLICATION

(11) **EP 1 219 635 A2**
(43) Date of publication of application: **03.07.2002**
(21) Application number: 01130295.7
(22) Date of filing: 21.12.2001
(51) Int. Cl.: C07K 14/295, C12N 15/63, A61K 38/00, A61P 31/04

(54) **Chlamydia pneumoniae antigenes**

(30) Priority: 21.12.2000 US 256941 P
(71) Applicant: SHIRE BIOCHEM INC., Laval, Quebec H7V 4A7 (CA)
(72) Inventor: Couture, France, St-David, Quebec G6W 7M2 (CA); Hamel, Josée, Sillery, Quebec G1T 1N6 (CA); Brodeur, Bernard R., Sillery, Quebec G1T 1N6 (CA); Martin, Denis, St-Augustin-de-Desmaures, Quebec G3A 1E9 (CA)
(74) Representative: HOFFMANN - EITLE

(57) **Abstract**

Chlamydia pneumoniae polypeptides and polynucleotides encoding them are disclosed. Said polypeptides are antigenic and therefore useful components for the prophylaxis, diagnosis or therapy of Chlamydial infection in animals. Also disclosed are recombinant methods of producing the protein antigens as well as diagnostic assays for detecting Chlamydia bacterial infection, particularly C. pneumoniae.

### Annex to the application documents - subsequently filed sequences listing

SEQUENCE LISTING

<110> Shire BioChem Inc.

<120> Novel Chlamydia antigens and coresponding DNA fragments

<130> 91287m3

<140> 01130295.7
<141> 2001-12-21

<150> US 60/256941
<151> 2000-12-21

<160> 76

<170> PatentIn Ver. 2.1

<210> 1
<211> 777
<212> DNA
<213> Chlamydia pneumoniae

<220>
<221> CDS
<222> (1)..(777)

<400> 1
atg aac aga cgg tgg aat tta gtt tta gca aca gta gct ctg gca ctc 48
Met Asn Arg Arg Trp Asn Leu Val Leu Ala Thr Val Ala Leu Ala Leu
1               5                   10                  15

tcc gtc gct tct tgt gac gta cgg tct aag gat aaa gac aag gat cag 96
Ser Val Ala Ser Cys Asp Val Arg Ser Lys Asp Lys Asp Lys Asp Gln
            20                  25                  30

ggg tcg tta gtg gaa tat aaa gat aac aaa gat acc aat gac ata gaa 144
Gly Ser Leu Val Glu Tyr Lys Asp Asn Lys Asp Thr Asn Asp Ile Glu
        35                  40                  45

tta tcc gat aat caa aag tta tcc aga aca ttt ggt cat tta tta gca 192
Leu Ser Asp Asn Gln Lys Leu Ser Arg Thr Phe Gly His Leu Leu Ala
    50                  55                  60

cgc caa tta cgc aag tca gaa gat atg ttt ttt gat att gca gaa gtg 240
Arg Gln Leu Arg Lys Ser Glu Asp Met Phe Phe Asp Ile Ala Glu Val
65                  70                  75                  80

gct aag ggg ttg cag gcg gaa ttg gtt tgt aaa agt gct cct tta aca    288
Ala Lys Gly Leu Gln Ala Glu Leu Val Cys Lys Ser Ala Pro Leu Thr
                85                  90                  95

gaa aca gag tat gaa gaa aaa atg gct gaa gta cag aag ttg gtt ttt    336
Glu Thr Glu Tyr Glu Glu Lys Met Ala Glu Val Gln Lys Leu Val Phe
            100                 105                 110

gaa aaa aaa tca aaa gaa aat ctt tca ttg gca gaa aaa ttc tta aaa    384
Glu Lys Lys Ser Lys Glu Asn Leu Ser Leu Ala Glu Lys Phe Leu Lys
        115                 120                 125

gaa aat agc aag aac gct ggt gtt gtt gaa gtg caa cca agt aaa ttg    432
Glu Asn Ser Lys Asn Ala Gly Val Val Glu Val Gln Pro Ser Lys Leu
    130                 135                 140

caa tac aaa att att aaa gaa ggt gca ggg aaa gca att tca ggt aaa    480
Gln Tyr Lys Ile Ile Lys Glu Gly Ala Gly Lys Ala Ile Ser Gly Lys
145                 150                 155                 160

cct tca gct cta ttg cac tac aag ggt tcc ttc atc aat ggc caa gta    528
Pro Ser Ala Leu Leu His Tyr Lys Gly Ser Phe Ile Asn Gly Gln Val
                165                 170                 175

ttt agc agt tca gaa ggc aac aat gag cct atc ttg ctt cct cta ggc    576
Phe Ser Ser Ser Glu Gly Asn Asn Glu Pro Ile Leu Leu Pro Leu Gly
            180                 185                 190

caa aca att cct ggt ttt gct tta ggt atg cag ggc atg aaa gaa gga    624
Gln Thr Ile Pro Gly Phe Ala Leu Gly Met Gln Gly Met Lys Glu Gly
        195                 200                 205

gaa act cga gtt ctc tac atc cat cct gat ctt gct tac gga acc gca    672
Glu Thr Arg Val Leu Tyr Ile His Pro Asp Leu Ala Tyr Gly Thr Ala
    210                 215                 220

gga caa ctt cct cca aac tct tta tta att ttt gaa att aac ttg att    720
Gly Gln Leu Pro Pro Asn Ser Leu Leu Ile Phe Glu Ile Asn Leu Ile
225                 230                 235                 240

cag gct tca gca gat gaa gtt gct gct gta ccc caa gaa gga aat caa    768
Gln Ala Ser Ala Asp Glu Val Ala Ala Val Pro Gln Glu Gly Asn Gln
                245                 250                 255

ggt gaa tga                                                        777
Gly Glu

<210> 2
<211> 258
<212> PRT
<213> Chlamydia pneumoniae

<400> 2
Met Asn Arg Arg Trp Asn Leu Val Leu Ala Thr Val Ala Leu Ala Leu
1               5                   10                  15
Ser Val Ala Ser Cys Asp Val Arg Ser Lys Asp Lys Asp Lys Asp Gln
            20                  25                  30
Gly Ser Leu Val Glu Tyr Lys Asp Asn Lys Asp Thr Asn Asp Ile Glu
        35                  40                  45
Leu Ser Asp Asn Gln Lys Leu Ser Arg Thr Phe Gly His Leu Leu Ala
    50                  55                  60
Arg Gln Leu Arg Lys Ser Glu Asp Met Phe Phe Asp Ile Ala Glu Val
65                  70                  75                  80
Ala Lys Gly Leu Gln Ala Glu Leu Val Cys Lys Ser Ala Pro Leu Thr
                85                  90                  95
Glu Thr Glu Tyr Glu Glu Lys Met Ala Glu Val Gln Lys Leu Val Phe
            100                 105                 110
Glu Lys Lys Ser Lys Glu Asn Leu Ser Leu Ala Glu Lys Phe Leu Lys
        115                 120                 125
Glu Asn Ser Lys Asn Ala Gly Val Val Glu Val Gln Pro Ser Lys Leu
    130                 135                 140
Gln Tyr Lys Ile Ile Lys Glu Gly Ala Gly Lys Ala Ile Ser Gly Lys
145                 150                 155                 160
Pro Ser Ala Leu Leu His Tyr Lys Gly Ser Phe Ile Asn Gly Gln Val
                165                 170                 175
Phe Ser Ser Ser Glu Gly Asn Asn Glu Pro Ile Leu Leu Pro Leu Gly
            180                 185                 190
Gln Thr Ile Pro Gly Phe Ala Leu Gly Met Gln Gly Met Lys Glu Gly
        195                 200                 205
Glu Thr Arg Val Leu Tyr Ile His Pro Asp Leu Ala Tyr Gly Thr Ala
    210                 215                 220
Gly Gln Leu Pro Pro Asn Ser Leu Leu Ile Phe Glu Ile Asn Leu Ile
225                 230                 235                 240
Gln Ala Ser Ala Asp Glu Val Ala Ala Val Pro Gln Glu Gly Asn Gln
                245                 250                 255
Gly Glu

<210> 3
<211> 741
<212> DNA
<213> Chlamydia pneumoniae

<220>

<221> CDS
<222> (1)..(741)

<400> 3
atg aaa atc acc aca gtc aaa aca cca aaa ata tat cct tat gat gac    48
Met Lys Ile Thr Thr Val Lys Thr Pro Lys Ile Tyr Pro Tyr Asp Asp
1               5                   10                  15

cta tat tct att cta gag tct tca ttg cct aag tta aac gaa cgc tct    96
Leu Tyr Ser Ile Leu Glu Ser Ser Leu Pro Lys Leu Asn Glu Arg Ser
            20                  25                  30

att gtt gtg att acg tct aag ata gtc tct tta tgt gaa ggt gct gtt    144
Ile Val Val Ile Thr Ser Lys Ile Val Ser Leu Cys Glu Gly Ala Val
        35                  40                  45

gta gaa ctt gag aag gtt tct aaa gat gaa tta ata aag caa gaa gca    192
Val Glu Leu Glu Lys Val Ser Lys Asp Glu Leu Ile Lys Gln Glu Ala
    50                  55                  60

gat gcc tat gtt ttt gta gag aaa tac ggc ata tat cta act aag aag    240
Asp Ala Tyr Val Phe Val Glu Lys Tyr Gly Ile Tyr Leu Thr Lys Lys
65                  70                  75                  80

tgg ggg ata ctc att cct tca gcg ggg att gac gag tcc aat gtt gaa    288
Trp Gly Ile Leu Ile Pro Ser Ala Gly Ile Asp Glu Ser Asn Val Glu
                85                  90                  95

ggt tat ttt gtg ttg tat cct agg gat ttt ttg ctt tcc gtg aat act    336
Gly Tyr Phe Val Leu Tyr Pro Arg Asp Phe Leu Leu Ser Val Asn Thr
            100                 105                 110

cta ggg gat tgg tta agg aat ttc tat cat ctc gag cat tgc gga atc    384
Leu Gly Asp Trp Leu Arg Asn Phe Tyr His Leu Glu His Cys Gly Ile
        115                 120                 125

att ata tcg gat agt cat acg act ccg ttg cgt cgg gga act atg ggt    432
Ile Ile Ser Asp Ser His Thr Thr Pro Leu Arg Arg Gly Thr Met Gly
    130                 135                 140

tta ggc tta tgt tgg aat ggt ttt ttc cct tta tat aat tat gta gga    480
Leu Gly Leu Cys Trp Asn Gly Phe Phe Pro Leu Tyr Asn Tyr Val Gly
145                 150                 155                 160

aaa cca gat tgt ttt ggt cgt gct ttg aag atg act tat agc aat tta    528
Lys Pro Asp Cys Phe Gly Arg Ala Leu Lys Met Thr Tyr Ser Asn Leu
                165                 170                 175

tta gat ggt tta tcg gca gct gcg gtt ctt tgt atg gga gag gga gac    576
Leu Asp Gly Leu Ser Ala Ala Ala Val Leu Cys Met Gly Glu Gly Asp
            180                 185                 190

gag cag act ccc att gct att ata gag gaa gct ccc aag att acc ttc    624
Glu Gln Thr Pro Ile Ala Ile Ile Glu Glu Ala Pro Lys Ile Thr Phe
        195                 200                 205

cat tct tct cca act aca tta caa gat atg agc act tta gca atc gct    672
His Ser Ser Pro Thr Thr Leu Gln Asp Met Ser Thr Leu Ala Ile Ala
    210                 215                 220

gag gat gaa gat tta tat ggt cct ctg cta caa tct atg gca tgg gaa    720
Glu Asp Glu Asp Leu Tyr Gly Pro Leu Leu Gln Ser Met Ala Trp Glu
225                 230                 235                 240

act ccc gca cca acc tcc tga                                        741
Thr Pro Ala Pro Thr Ser
                245

<210> 4
<211> 246
<212> PRT
<213> Chlamydia pneumoniae

<400> 4
Met Lys Ile Thr Thr Val Lys Thr Pro Lys Ile Tyr Pro Tyr Asp Asp
  1               5                  10                  15
Leu Tyr Ser Ile Leu Glu Ser Ser Leu Pro Lys Leu Asn Glu Arg Ser
             20                  25                  30
Ile Val Val Ile Thr Ser Lys Ile Val Ser Leu Cys Glu Gly Ala Val
         35                  40                  45
Val Glu Leu Glu Lys Val Ser Lys Asp Glu Leu Ile Lys Gln Glu Ala
     50                  55                  60
Asp Ala Tyr Val Phe Val Glu Lys Tyr Gly Ile Tyr Leu Thr Lys Lys
 65                  70                  75                  80
Trp Gly Ile Leu Ile Pro Ser Ala Gly Ile Asp Glu Ser Asn Val Glu
                 85                  90                  95
Gly Tyr Phe Val Leu Tyr Pro Arg Asp Phe Leu Leu Ser Val Asn Thr
            100                 105                 110
Leu Gly Asp Trp Leu Arg Asn Phe Tyr His Leu Glu His Cys Gly Ile
        115                 120                 125
Ile Ile Ser Asp Ser His Thr Thr Pro Leu Arg Arg Gly Thr Met Gly
    130                 135                 140
Leu Gly Leu Cys Trp Asn Gly Phe Phe Pro Leu Tyr Asn Tyr Val Gly
145                 150                 155                 160
Lys Pro Asp Cys Phe Gly Arg Ala Leu Lys Met Thr Tyr Ser Asn Leu

165                 170                 175
Leu Asp Gly Leu Ser Ala Ala Ala Val Leu Cys Met Gly Glu Gly Asp
            180                 185                 190
Glu Gln Thr Pro Ile Ala Ile Ile Glu Glu Ala Pro Lys Ile Thr Phe
        195                 200                 205
His Ser Ser Pro Thr Thr Leu Gln Asp Met Ser Thr Leu Ala Ile Ala
    210                 215                 220
Glu Asp Glu Asp Leu Tyr Gly Pro Leu Leu Gln Ser Met Ala Trp Glu
225                 230                 235                 240
Thr Pro Ala Pro Thr Ser
                245

<210> 5
<211> 1584
<212> DNA
<213> Chlamydia pneumoniae

<220>
<221> CDS
<222> (1)..(1584)

<400> 5
atg ctc cgt ttc ttc gct gta ttt ata tca act ctt tgg ctc att acc      48
Met Leu Arg Phe Phe Ala Val Phe Ile Ser Thr Leu Trp Leu Ile Thr
  1               5                  10                  15

tca gga tgt tcc cca tcc caa tcc tct aaa gga att ttt gtg gta aat      96
Ser Gly Cys Ser Pro Ser Gln Ser Ser Lys Gly Ile Phe Val Val Asn
             20                  25                  30

atg aag gaa atg cca cgc tcc ttg gat cct gga aaa act cgt ctc att     144
Met Lys Glu Met Pro Arg Ser Leu Asp Pro Gly Lys Thr Arg Leu Ile
         35                  40                  45

gca gac caa act cta atg cgt cat cta tat gaa gga ctc gtc gaa gaa     192
Ala Asp Gln Thr Leu Met Arg His Leu Tyr Glu Gly Leu Val Glu Glu
     50                  55                  60

cat tcc caa aat gga gag att aaa cca gcc ctt gca gaa agc tac acc     240
His Ser Gln Asn Gly Glu Ile Lys Pro Ala Leu Ala Glu Ser Tyr Thr
 65                  70                  75                  80

atc tcc gaa gac ggg act cgg tac aca ttt aaa atc aaa aac atc ctt     288
Ile Ser Glu Asp Gly Thr Arg Tyr Thr Phe Lys Ile Lys Asn Ile Leu
                 85                  90                  95

tgg agt aac gga gac cct ctg aca gct caa gac ttt gtc tcc tct tgg 336
Trp Ser Asn Gly Asp Pro Leu Thr Ala Gln Asp Phe Val Ser Ser Trp
            100                 105                 110

aag gaa atc cta aag gaa gat gcg tcc tcc gta tat ctc tat gcg ttt 384
Lys Glu Ile Leu Lys Glu Asp Ala Ser Ser Val Tyr Leu Tyr Ala Phe
        115                 120                 125

tta cct atc aaa aat gct cgg gca atc ttt gat gat act gag tct cca 432
Leu Pro Ile Lys Asn Ala Arg Ala Ile Phe Asp Asp Thr Glu Ser Pro
    130                 135                 140

gaa aat cta gga gtc cga gct tta gat aag cgt cat ctc gaa att cag 480
Glu Asn Leu Gly Val Arg Ala Leu Asp Lys Arg His Leu Glu Ile Gln
145                 150                 155                 160

tta gaa act ccc tgc gcg cat ttc cta cat ttc ttg act ctt cct att 528
Leu Glu Thr Pro Cys Ala His Phe Leu His Phe Leu Thr Leu Pro Ile
                165                 170                 175

ttt ttc cct gtt cat gaa act ctg cga aac tat agc acc tct ttt gaa 576
Phe Phe Pro Val His Glu Thr Leu Arg Asn Tyr Ser Thr Ser Phe Glu
            180                 185                 190

gag atg ccc att acc tgc ggt gct ttc cgc cct gtg tct cta gaa aaa 624
Glu Met Pro Ile Thr Cys Gly Ala Phe Arg Pro Val Ser Leu Glu Lys
        195                 200                 205

ggc ctg aga ctc cat cta gag aaa aac cct atg tac cat aat aaa agc 672
Gly Leu Arg Leu His Leu Glu Lys Asn Pro Met Tyr His Asn Lys Ser
    210                 215                 220

cgt gtg aaa cta cat aaa att att gta cag ttt atc tca aac gct aac 720
Arg Val Lys Leu His Lys Ile Ile Val Gln Phe Ile Ser Asn Ala Asn
225                 230                 235                 240

act gca gcc att cta ttc aaa cat aag aaa tta gat tgg caa gga cct 768
Thr Ala Ala Ile Leu Phe Lys His Lys Lys Leu Asp Trp Gln Gly Pro
                245                 250                 255

cct tgg gga gaa cct atc cct cca gaa atc tca gct tct cta cat caa 816
Pro Trp Gly Glu Pro Ile Pro Pro Glu Ile Ser Ala Ser Leu His Gln
            260                 265                 270

gat gac cag ctc ttt tct ctt ccg ggc gct tcg act aca tgg tta ctc 864
Asp Asp Gln Leu Phe Ser Leu Pro Gly Ala Ser Thr Thr Trp Leu Leu
        275                 280                 285

ttt aat ata caa aaa aaa cct tgg aac aat gct aaa tta cgc aag gca 912
Phe Asn Ile Gln Lys Lys Pro Trp Asn Asn Ala Lys Leu Arg Lys Ala
    290                 295                 300

ttg agc ctt gca ata gac aaa gat atg tta acc aaa gtg gta tac caa 960
Leu Ser Leu Ala Ile Asp Lys Asp Met Leu Thr Lys Val Val Tyr Gln
305                 310                 315                 320

ggt ctt gca gaa cct aca gat cat atc cta cat cca aga ctt tat cca 1008
Gly Leu Ala Glu Pro Thr Asp His Ile Leu His Pro Arg Leu Tyr Pro
                325                 330                 335

ggg acc tat ccc gaa cgg aaa aga caa aac gaa aga att ctt gag gct 1056
Gly Thr Tyr Pro Glu Arg Lys Arg Gln Asn Glu Arg Ile Leu Glu Ala
            340                 345                 350

caa caa ctc ttt gaa gaa gct cta gac gaa ctt caa atg aca cgc gaa 1104
Gln Gln Leu Phe Glu Glu Ala Leu Asp Glu Leu Gln Met Thr Arg Glu
        355                 360                 365

gat cta gaa aag gaa act ttg act ttc tca acc ttt tct ttt tct tac 1152
Asp Leu Glu Lys Glu Thr Leu Thr Phe Ser Thr Phe Ser Phe Ser Tyr
    370                 375                 380

gga agg att tgc caa atg cta aga gaa caa tgg aag aaa gtc tta aaa 1200
Gly Arg Ile Cys Gln Met Leu Arg Glu Gln Trp Lys Lys Val Leu Lys
385                 390                 395                 400

ttt act atc cct ata gta ggc caa gag ttt ttc aca ata caa aaa aac 1248
Phe Thr Ile Pro Ile Val Gly Gln Glu Phe Phe Thr Ile Gln Lys Asn
                405                 410                 415

ttc cta gag ggg aac tat tcc cta acc gtg aac caa tgg acc gca gca 1296
Phe Leu Glu Gly Asn Tyr Ser Leu Thr Val Asn Gln Trp Thr Ala Ala
            420                 425                 430

ttt att gat ccg atg tct tat ctc atg atc ttt gcc aat cct gga gga 1344
Phe Ile Asp Pro Met Ser Tyr Leu Met Ile Phe Ala Asn Pro Gly Gly
        435                 440                 445

att tcc ccc tat cac ctc caa gat tca cac ttt caa act ctt ctc ata 1392
Ile Ser Pro Tyr His Leu Gln Asp Ser His Phe Gln Thr Leu Leu Ile
    450                 455                 460

aag atc act caa gaa cat aaa aaa cac cta cga aat cag ctt att att 1440
Lys Ile Thr Gln Glu His Lys Lys His Leu Arg Asn Gln Leu Ile Ile
465                 470                 475                 480

gaa gcc ctt gac tat tta gaa cac tgt cac att ctc gaa cca cta tgt    1488
Glu Ala Leu Asp Tyr Leu Glu His Cys His Ile Leu Glu Pro Leu Cys
                485                 490                 495

cat cca aat ctt cga att gct ttg aac aaa aac att aaa aac ttt aat    1536
His Pro Asn Leu Arg Ile Ala Leu Asn Lys Asn Ile Lys Asn Phe Asn
            500                 505                 510

ctt ttt gtt cga cga act tca gac ttt cgt ttt ata gaa aaa cta tag    1584
Leu Phe Val Arg Arg Thr Ser Asp Phe Arg Phe Ile Glu Lys Leu
        515                 520                 525

<210> 6
<211> 527
<212> PRT
<213> Chlamydia pneumoniae

<400> 6
Met Leu Arg Phe Phe Ala Val Phe Ile Ser Thr Leu Trp Leu Ile Thr
  1               5                  10                  15
Ser Gly Cys Ser Pro Ser Gln Ser Ser Lys Gly Ile Phe Val Val Asn
             20                  25                  30
Met Lys Glu Met Pro Arg Ser Leu Asp Pro Gly Lys Thr Arg Leu Ile
         35                  40                  45
Ala Asp Gln Thr Leu Met Arg His Leu Tyr Glu Gly Leu Val Glu Glu
     50                  55                  60
His Ser Gln Asn Gly Glu Ile Lys Pro Ala Leu Ala Glu Ser Tyr Thr
 65                  70                  75                  80
Ile Ser Glu Asp Gly Thr Arg Tyr Thr Phe Lys Ile Lys Asn Ile Leu
                 85                  90                  95
Trp Ser Asn Gly Asp Pro Leu Thr Ala Gln Asp Phe Val Ser Ser Trp
            100                 105                 110
Lys Glu Ile Leu Lys Glu Asp Ala Ser Ser Val Tyr Leu Tyr Ala Phe
        115                 120                 125
Leu Pro Ile Lys Asn Ala Arg Ala Ile Phe Asp Asp Thr Glu Ser Pro
    130                 135                 140
Glu Asn Leu Gly Val Arg Ala Leu Asp Lys Arg His Leu Glu Ile Gln
145                 150                 155                 160
Leu Glu Thr Pro Cys Ala His Phe Leu His Phe Leu Thr Leu Pro Ile
                165                 170                 175
Phe Phe Pro Val His Glu Thr Leu Arg Asn Tyr Ser Thr Ser Phe Glu
            180                 185                 190
Glu Met Pro Ile Thr Cys Gly Ala Phe Arg Pro Val Ser Leu Glu Lys
        195                 200                 205
Gly Leu Arg Leu His Leu Glu Lys Asn Pro Met Tyr His Asn Lys Ser
    210                 215                 220
Arg Val Lys Leu His Lys Ile Ile Val Gln Phe Ile Ser Asn Ala Asn

225                 230                 235                 240
Thr Ala Ala Ile Leu Phe Lys His Lys Lys Leu Asp Trp Gln Gly Pro
                245                 250                 255
Pro Trp Gly Glu Pro Ile Pro Pro Glu Ile Ser Ala Ser Leu His Gln
            260                 265                 270
Asp Asp Gln Leu Phe Ser Leu Pro Gly Ala Ser Thr Thr Trp Leu Leu
        275                 280                 285
Phe Asn Ile Gln Lys Lys Pro Trp Asn Asn Ala Lys Leu Arg Lys Ala
    290                 295                 300
Leu Ser Leu Ala Ile Asp Lys Asp Met Leu Thr Lys Val Val Tyr Gln
305                 310                 315                 320
Gly Leu Ala Glu Pro Thr Asp His Ile Leu His Pro Arg Leu Tyr Pro
                325                 330                 335
Gly Thr Tyr Pro Glu Arg Lys Arg Gln Asn Glu Arg Ile Leu Glu Ala
            340                 345                 350
Gln Gln Leu Phe Glu Glu Ala Leu Asp Glu Leu Gln Met Thr Arg Glu
        355                 360                 365
Asp Leu Glu Lys Glu Thr Leu Thr Phe Ser Thr Phe Ser Phe Ser Tyr
    370                 375                 380
Gly Arg Ile Cys Gln Met Leu Arg Glu Gln Trp Lys Lys Val Leu Lys
385                 390                 395                 400
Phe Thr Ile Pro Ile Val Gly Gln Glu Phe Phe Thr Ile Gln Lys Asn
                405                 410                 415
Phe Leu Glu Gly Asn Tyr Ser Leu Thr Val Asn Gln Trp Thr Ala Ala
            420                 425                 430
Phe Ile Asp Pro Met Ser Tyr Leu Met Ile Phe Ala Asn Pro Gly Gly
        435                 440                 445
Ile Ser Pro Tyr His Leu Gln Asp Ser His Phe Gln Thr Leu Leu Ile
    450                 455                 460
Lys Ile Thr Gln Glu His Lys Lys His Leu Arg Asn Gln Leu Ile Ile
465                 470                 475                 480
Glu Ala Leu Asp Tyr Leu Glu His Cys His Ile Leu Glu Pro Leu Cys
                485                 490                 495
His Pro Asn Leu Arg Ile Ala Leu Asn Lys Asn Ile Lys Asn Phe Asn
            500                 505                 510
Leu Phe Val Arg Arg Thr Ser Asp Phe Arg Phe Ile Glu Lys Leu
        515                 520                 525

<210> 7
<211> 1173
<212> DNA
<213> Chlamydia pneumoniae

<220>
<221> CDS
<222> (1)..(1173)

<400> 7
atg tca tct cct gta aat aac aca ccc tca gca cca aac att cca ata    48
Met Ser Ser Pro Val Asn Asn Thr Pro Ser Ala Pro Asn Ile Pro Ile
1               5                   10                  15

cca gcg ccc acg act cca ggt att cct aca aca aaa cct cgt tct agt    96
Pro Ala Pro Thr Thr Pro Gly Ile Pro Thr Thr Lys Pro Arg Ser Ser
            20                  25                  30

ttc att gaa aag gtt atc att gta gct aag tac ata cta ttt gca att    144
Phe Ile Glu Lys Val Ile Ile Val Ala Lys Tyr Ile Leu Phe Ala Ile
        35                  40                  45

gca gcc aca tca gga gca ctc gga aca att cta ggt cta tct gga gcg    192
Ala Ala Thr Ser Gly Ala Leu Gly Thr Ile Leu Gly Leu Ser Gly Ala
    50                  55                  60

cta acc cca gga ata ggt att gcc ctt ctt gtt atc ttc ttt gtt tct    240
Leu Thr Pro Gly Ile Gly Ile Ala Leu Leu Val Ile Phe Phe Val Ser
65                  70                  75                  80

atg gtg ctt tta ggt tta atc ctt aaa gat tct ata agt gga gga gaa    288
Met Val Leu Leu Gly Leu Ile Leu Lys Asp Ser Ile Ser Gly Gly Glu
                85                  90                  95

gaa cgc agg ctc aga gaa gag gtc tct cga ttt aca agt gag aat caa    336
Glu Arg Arg Leu Arg Glu Glu Val Ser Arg Phe Thr Ser Glu Asn Gln
            100                 105                 110

cgg ttg aca gtc ata acc aca aca ctt gag act gaa gta aag gat tta    384
Arg Leu Thr Val Ile Thr Thr Thr Leu Glu Thr Glu Val Lys Asp Leu
        115                 120                 125

aaa gca gct aaa gat caa ctt aca ctt gaa atc gaa gca ttt aga aat    432
Lys Ala Ala Lys Asp Gln Leu Thr Leu Glu Ile Glu Ala Phe Arg Asn
    130                 135                 140

gaa aac ggt aat tta aaa aca act gct gag gac tta gaa gag cag gtt    480
Glu Asn Gly Asn Leu Lys Thr Thr Ala Glu Asp Leu Glu Glu Gln Val
145                 150                 155                 160

tct aaa ctt agc gaa caa tta gaa gca cta gag cga att aat caa ctt    528
Ser Lys Leu Ser Glu Gln Leu Glu Ala Leu Glu Arg Ile Asn Gln Leu
                165                 170                 175

atc caa gca aac gct gga gat gct caa gaa att tcg tct gaa cta aag    576
Ile Gln Ala Asn Ala Gly Asp Ala Gln Glu Ile Ser Ser Glu Leu Lys

180                 185                 190

aaa tta ata agc ggt tgg gat tcc aaa gtt gtt gaa cag ata aat act    624
Lys Leu Ile Ser Gly Trp Asp Ser Lys Val Val Glu Gln Ile Asn Thr
        195                 200                 205

tct att caa gca ttg aaa gtg tta ttg ggt caa gag tgg gtg caa gag    672
Ser Ile Gln Ala Leu Lys Val Leu Leu Gly Gln Glu Trp Val Gln Glu
    210                 215                 220

gct caa aca cac gtt aaa gca atg caa gag caa att caa gca ttg caa    720
Ala Gln Thr His Val Lys Ala Met Gln Glu Gln Ile Gln Ala Leu Gln
225                 230                 235                 240

gct gaa att cta gga atg cac aat caa tct aca gca ttg caa aag tca    768
Ala Glu Ile Leu Gly Met His Asn Gln Ser Thr Ala Leu Gln Lys Ser
                245                 250                 255

gtt gag aat cta tta gta caa gat caa gct cta aca aga gta gta ggt    816
Val Glu Asn Leu Leu Val Gln Asp Gln Ala Leu Thr Arg Val Val Gly
            260                 265                 270

gag ttg tta gag tct gag aac aag cta agc caa gct tgt tct gcg cta    864
Glu Leu Leu Glu Ser Glu Asn Lys Leu Ser Gln Ala Cys Ser Ala Leu
        275                 280                 285

cgt caa gaa ata gaa aag ttg gcc caa cat gaa aca tct ttg caa caa    912
Arg Gln Glu Ile Glu Lys Leu Ala Gln His Glu Thr Ser Leu Gln Gln
    290                 295                 300

cgt att gat gcg atg cta gcc caa gag caa aat ttg gca gag cag gtc    960
Arg Ile Asp Ala Met Leu Ala Gln Glu Gln Asn Leu Ala Glu Gln Val
305                 310                 315                 320

aca gcc ctt gaa aaa atg aaa caa gaa gct cag aag gct gag tcc gag    1008
Thr Ala Leu Glu Lys Met Lys Gln Glu Ala Gln Lys Ala Glu Ser Glu
                325                 330                 335

ttc att gct tgt gta cgt gat cga act ttc gga cgt cgt gaa aca cct    1056
Phe Ile Ala Cys Val Arg Asp Arg Thr Phe Gly Arg Arg Glu Thr Pro
            340                 345                 350

cca cca aca aca cct gta gtt gaa ggt gat gaa agt caa gaa gaa gac    1104
Pro Pro Thr Thr Pro Val Val Glu Gly Asp Glu Ser Gln Glu Glu Asp
        355                 360                 365

gaa gga ggt act ccc cca gta tca caa cca tct tca ccc gta gat aga    1152
Glu Gly Gly Thr Pro Pro Val Ser Gln Pro Ser Ser Pro Val Asp Arg

370                 375                 380

gca aca gga gat ggt cag taa                                          1173
Ala Thr Gly Asp Gly Gln
385                 390

<210> 8
<211> 390
<212> PRT
<213> Chlamydia pneumoniae

<400> 8
Met Ser Ser Pro Val Asn Asn Thr Pro Ser Ala Pro Asn Ile Pro Ile
1               5                   10                  15
Pro Ala Pro Thr Thr Pro Gly Ile Pro Thr Thr Lys Pro Arg Ser Ser
            20                  25                  30
Phe Ile Glu Lys Val Ile Ile Val Ala Lys Tyr Ile Leu Phe Ala Ile
        35                  40                  45
Ala Ala Thr Ser Gly Ala Leu Gly Thr Ile Leu Gly Leu Ser Gly Ala
    50                  55                  60
Leu Thr Pro Gly Ile Gly Ile Ala Leu Leu Val Ile Phe Phe Val Ser
65                  70                  75                  80
Met Val Leu Leu Gly Leu Ile Leu Lys Asp Ser Ile Ser Gly Gly Glu
                85                  90                  95
Glu Arg Arg Leu Arg Glu Glu Val Ser Arg Phe Thr Ser Glu Asn Gln
            100                 105                 110
Arg Leu Thr Val Ile Thr Thr Thr Leu Glu Thr Glu Val Lys Asp Leu
        115                 120                 125
Lys Ala Ala Lys Asp Gln Leu Thr Leu Glu Ile Glu Ala Phe Arg Asn
    130                 135                 140
Glu Asn Gly Asn Leu Lys Thr Thr Ala Glu Asp Leu Glu Glu Gln Val
145                 150                 155                 160
Ser Lys Leu Ser Glu Gln Leu Glu Ala Leu Glu Arg Ile Asn Gln Leu
                165                 170                 175
Ile Gln Ala Asn Ala Gly Asp Ala Gln Glu Ile Ser Ser Glu Leu Lys
            180                 185                 190
Lys Leu Ile Ser Gly Trp Asp Ser Lys Val Val Glu Gln Ile Asn Thr
        195                 200                 205
Ser Ile Gln Ala Leu Lys Val Leu Leu Gly Gln Glu Trp Val Gln Glu
    210                 215                 220
Ala Gln Thr His Val Lys Ala Met Gln Glu Gln Ile Gln Ala Leu Gln
225                 230                 235                 240
Ala Glu Ile Leu Gly Met His Asn Gln Ser Thr Ala Leu Gln Lys Ser
                245                 250                 255
Val Glu Asn Leu Leu Val Gln Asp Gln Ala Leu Thr Arg Val Val Gly
            260                 265                 270
Glu Leu Leu Glu Ser Glu Asn Lys Leu Ser Gln Ala Cys Ser Ala Leu

275                 280                 285
Arg Gln Glu Ile Glu Lys Leu Ala Gln His Glu Thr Ser Leu Gln Gln
    290                 295                 300
Arg Ile Asp Ala Met Leu Ala Gln Glu Gln Asn Leu Ala Glu Gln Val
305                 310                 315                 320
Thr Ala Leu Glu Lys Met Lys Gln Glu Ala Gln Lys Ala Glu Ser Glu
                325                 330                 335
Phe Ile Ala Cys Val Arg Asp Arg Thr Phe Gly Arg Arg Glu Thr Pro
            340                 345                 350
Pro Pro Thr Thr Pro Val Val Glu Gly Asp Glu Ser Gln Glu Glu Asp
        355                 360                 365
Glu Gly Gly Thr Pro Pro Val Ser Gln Pro Ser Ser Pro Val Asp Arg
    370                 375                 380
Ala Thr Gly Asp Gly Gln
385                 390

<210> 9
<211> 1980
<212> DNA
<213> Chlamydia pneumoniae

<220>
<221> CDS
<222> (1)..(1980)

<400> 9
atg aaa cta ctt ctg aaa gcg gtc ctg agg cat aaa aat cat ctc gtt     48
Met Lys Leu Leu Leu Lys Ala Val Leu Arg His Lys Asn His Leu Val
  1               5                  10                  15

ata tta ggc tgt tct cta ctc gca att tta gga ctt acc ttt tca tct     96
Ile Leu Gly Cys Ser Leu Leu Ala Ile Leu Gly Leu Thr Phe Ser Ser
             20                  25                  30

cag atg gag att ttt tct tta ggg atg att gct aaa aca ggc ccc gac    144
Gln Met Glu Ile Phe Ser Leu Gly Met Ile Ala Lys Thr Gly Pro Asp
         35                  40                  45

gcc ttt tta ctt ttt gga cgt aag gaa tct gga aaa ctt gta aag gtt    192
Ala Phe Leu Leu Phe Gly Arg Lys Glu Ser Gly Lys Leu Val Lys Val
     50                  55                  60

tca gaa cta agt cag aaa gat att tta gag aat tgg cag gca att agt    240
Ser Glu Leu Ser Gln Lys Asp Ile Leu Glu Asn Trp Gln Ala Ile Ser
 65                  70                  75                  80

aag gat tca gag aca ctt aca gtc tct gat gcc acg aca tac atc gcc 288
Lys Asp Ser Glu Thr Leu Thr Val Ser Asp Ala Thr Thr Tyr Ile Ala
                85                  90                  95

gaa cat ggg aaa agc aca gcc tct ctg acg agc aag ctc tct aag ttt 336
Glu His Gly Lys Ser Thr Ala Ser Leu Thr Ser Lys Leu Ser Lys Phe
            100                 105                 110

gtc cgt aac tac atc gat gtg agc cgc ttt cga gga ctg gca atc ttc 384
Val Arg Asn Tyr Ile Asp Val Ser Arg Phe Arg Gly Leu Ala Ile Phe
        115                 120                 125

tta atc tgc gtt gct att ttt aaa gca gtc acc tta ttt ttc caa cgt 432
Leu Ile Cys Val Ala Ile Phe Lys Ala Val Thr Leu Phe Phe Gln Arg
    130                 135                 140

ttc ctt ggg caa gtc gtt gct ata cgg gta agc cga gac tta cgt cag 480
Phe Leu Gly Gln Val Val Ala Ile Arg Val Ser Arg Asp Leu Arg Gln
145                 150                 155                 160

gac tac ttt aag gcc cta caa caa ctc ccc atg acc ttc ttc cat gat 528
Asp Tyr Phe Lys Ala Leu Gln Gln Leu Pro Met Thr Phe Phe His Asp
                165                 170                 175

cat gat atc ggt aat tta agt aat cgt gtc atg aca gat tct gca agc 576
His Asp Ile Gly Asn Leu Ser Asn Arg Val Met Thr Asp Ser Ala Ser
            180                 185                 190

att gcc tta gca gta aac tct tta atg att aac tac att caa gcc cca 624
Ile Ala Leu Ala Val Asn Ser Leu Met Ile Asn Tyr Ile Gln Ala Pro
        195                 200                 205

att acc ttc ata ttg aca ttg gga gtc tgt ctg tcg att tca tgg aag 672
Ile Thr Phe Ile Leu Thr Leu Gly Val Cys Leu Ser Ile Ser Trp Lys
    210                 215                 220

ttt tca att ctt att tgt gtt gcc ttt cct atc ttt atc ctt ccc att 720
Phe Ser Ile Leu Ile Cys Val Ala Phe Pro Ile Phe Ile Leu Pro Ile
225                 230                 235                 240

gtc gtg atc gct aga aag atc aaa aat tta gca aaa cgt att caa aag 768
Val Val Ile Ala Arg Lys Ile Lys Asn Leu Ala Lys Arg Ile Gln Lys
                245                 250                 255

agt cag gat tca ttt tcc tcc gtt ctt tat gat ttt ctt gct ggg gtt 816
Ser Gln Asp Ser Phe Ser Ser Val Leu Tyr Asp Phe Leu Ala Gly Val
            260                 265                 270

atg aca gta aaa gtc ttt cgt aca gaa aaa ttt gcc ttc aca aaa tat 864
Met Thr Val Lys Val Phe Arg Thr Glu Lys Phe Ala Phe Thr Lys Tyr
        275                 280                 285

tgt gag cat aac aat aag att tct gct tta gag gag aaa agt gct gct 912
Cys Glu His Asn Asn Lys Ile Ser Ala Leu Glu Glu Lys Ser Ala Ala
    290                 295                 300

tac ggt ttg ctt cca cga ccc ctc ctg cat acc ata gct tct tta ttt 960
Tyr Gly Leu Leu Pro Arg Pro Leu Leu His Thr Ile Ala Ser Leu Phe
305                 310                 315                 320

ttt gct ttt gtc gtc gtt atc gga att tat aaa ttt gct att cct ccc 1008
Phe Ala Phe Val Val Val Ile Gly Ile Tyr Lys Phe Ala Ile Pro Pro
                325                 330                 335

gaa gaa ctt atc gta ttt tgt ggt ttg ctc tac cta atc tac gac cct 1056
Glu Glu Leu Ile Val Phe Cys Gly Leu Leu Tyr Leu Ile Tyr Asp Pro
            340                 345                 350

att aag aag ttc ggg gat gaa aat acc tcc atc atg agg gga tgt gct 1104
Ile Lys Lys Phe Gly Asp Glu Asn Thr Ser Ile Met Arg Gly Cys Ala
        355                 360                 365

gct gcg gag aga ttt tat gaa gtc ttg aat cac ccc gat ctt cat agt 1152
Ala Ala Glu Arg Phe Tyr Glu Val Leu Asn His Pro Asp Leu His Ser
    370                 375                 380

caa aaa gaa aga gaa atc gag ttc ctt gga ctt tct aat aca atc aca 1200
Gln Lys Glu Arg Glu Ile Glu Phe Leu Gly Leu Ser Asn Thr Ile Thr
385                 390                 395                 400

ttc gag aat gtt tcc ttc ggc tat cag gaa gat aag cac atc ctc aaa 1248
Phe Glu Asn Val Ser Phe Gly Tyr Gln Glu Asp Lys His Ile Leu Lys
                405                 410                 415

aat cta agc ttt acc tta cat aaa ggc gaa gct cta ggc att gta gga 1296
Asn Leu Ser Phe Thr Leu His Lys Gly Glu Ala Leu Gly Ile Val Gly
            420                 425                 430

cct aca gga tct gga aaa aca aca ctt gtt aaa tta ctt cct agg ctc 1344
Pro Thr Gly Ser Gly Lys Thr Thr Leu Val Lys Leu Leu Pro Arg Leu
        435                 440                 445

tac gaa gtc tcc caa gga aag att ctt atc gac tct ctt cct att acg 1392
Tyr Glu Val Ser Gln Gly Lys Ile Leu Ile Asp Ser Leu Pro Ile Thr
    450                 455                 460

gaa tat aac aaa ggg tcc tta agg aat cac atc gcc tgt gta tta cag    1440
Glu Tyr Asn Lys Gly Ser Leu Arg Asn His Ile Ala Cys Val Leu Gln
465                 470                 475                 480

aat cct ttc tta ttc tat gat act gta tgg aat aac ctt acc tgt ggt    1488
Asn Pro Phe Leu Phe Tyr Asp Thr Val Trp Asn Asn Leu Thr Cys Gly
                485                 490                 495

aag gat atg gag gag gag gct gtt tta gaa gct cta aaa cgt gcc tac    1536
Lys Asp Met Glu Glu Glu Ala Val Leu Glu Ala Leu Lys Arg Ala Tyr
            500                 505                 510

gct gat gag ttt att tta aag ctc cct aaa gga gtc cat agc gtg ctc    1584
Ala Asp Glu Phe Ile Leu Lys Leu Pro Lys Gly Val His Ser Val Leu
        515                 520                 525

gaa gaa tct ggg aag aat ctc tca gga gga cag cag caa cgt ttg gca    1632
Glu Glu Ser Gly Lys Asn Leu Ser Gly Gly Gln Gln Gln Arg Leu Ala
    530                 535                 540

ata gca cgt gct ctg ttg aaa aac gcc tcc atc tta att tta gat gag    1680
Ile Ala Arg Ala Leu Leu Lys Asn Ala Ser Ile Leu Ile Leu Asp Glu
545                 550                 555                 560

gca acg tca gct cta gat gcc att agt gaa aat tac att aag aat atc    1728
Ala Thr Ser Ala Leu Asp Ala Ile Ser Glu Asn Tyr Ile Lys Asn Ile
                565                 570                 575

att gga gag ctt aaa gga cag tgc aca caa atc att att gcc cac aag    1776
Ile Gly Glu Leu Lys Gly Gln Cys Thr Gln Ile Ile Ile Ala His Lys
            580                 585                 590

ctg acc act ctt gaa cat gta gat cgc gtg ctc tac ata gaa aat ggt    1824
Leu Thr Thr Leu Glu His Val Asp Arg Val Leu Tyr Ile Glu Asn Gly
        595                 600                 605

caa aaa att gcc gaa ggc aca aaa gaa gaa ctc tta cag acg tgt cct    1872
Gln Lys Ile Ala Glu Gly Thr Lys Glu Glu Leu Leu Gln Thr Cys Pro
    610                 615                 620

gaa ttt tta aaa atg tgg gag ctc tca gga act aaa gaa tat aac agg    1920
Glu Phe Leu Lys Met Trp Glu Leu Ser Gly Thr Lys Glu Tyr Asn Arg
625                 630                 635                 640

gtc ttt gtt cct gat cac aaa tta gtc gca aat cct acg gac atg gca    1968
Val Phe Val Pro Asp His Lys Leu Val Ala Asn Pro Thr Asp Met Ala
                645                 650                 655

ata aca act tag                                                    1980
Ile Thr Thr
            660



<210> 10
<211> 659
<212> PRT
<213> Chlamydia pneumoniae

<400> 10
Met Lys Leu Leu Leu Lys Ala Val Leu Arg His Lys Asn His Leu Val
1               5                   10                  15
Ile Leu Gly Cys Ser Leu Leu Ala Ile Leu Gly Leu Thr Phe Ser Ser
            20                  25                  30
Gln Met Glu Ile Phe Ser Leu Gly Met Ile Ala Lys Thr Gly Pro Asp
        35                  40                  45
Ala Phe Leu Leu Phe Gly Arg Lys Glu Ser Gly Lys Leu Val Lys Val
    50                  55                  60
Ser Glu Leu Ser Gln Lys Asp Ile Leu Glu Asn Trp Gln Ala Ile Ser
65                  70                  75                  80
Lys Asp Ser Glu Thr Leu Thr Val Ser Asp Ala Thr Thr Tyr Ile Ala
                85                  90                  95
Glu His Gly Lys Ser Thr Ala Ser Leu Thr Ser Lys Leu Ser Lys Phe
            100                 105                 110
Val Arg Asn Tyr Ile Asp Val Ser Arg Phe Arg Gly Leu Ala Ile Phe
        115                 120                 125
Leu Ile Cys Val Ala Ile Phe Lys Ala Val Thr Leu Phe Phe Gln Arg
    130                 135                 140
Phe Leu Gly Gln Val Val Ala Ile Arg Val Ser Arg Asp Leu Arg Gln
145                 150                 155                 160
Asp Tyr Phe Lys Ala Leu Gln Gln Leu Pro Met Thr Phe Phe His Asp
                165                 170                 175
His Asp Ile Gly Asn Leu Ser Asn Arg Val Met Thr Asp Ser Ala Ser
            180                 185                 190
Ile Ala Leu Ala Val Asn Ser Leu Met Ile Asn Tyr Ile Gln Ala Pro
        195                 200                 205
Ile Thr Phe Ile Leu Thr Leu Gly Val Cys Leu Ser Ile Ser Trp Lys
    210                 215                 220
Phe Ser Ile Leu Ile Cys Val Ala Phe Pro Ile Phe Ile Leu Pro Ile
225                 230                 235                 240
Val Val Ile Ala Arg Lys Ile Lys Asn Leu Ala Lys Arg Ile Gln Lys
                245                 250                 255
Ser Gln Asp Ser Phe Ser Ser Val Leu Tyr Asp Phe Leu Ala Gly Val
            260                 265                 270
Met Thr Val Lys Val Phe Arg Thr Glu Lys Phe Ala Phe Thr Lys Tyr
        275                 280                 285
Cys Glu His Asn Asn Lys Ile Ser Ala Leu Glu Glu Lys Ser Ala Ala

290                 295                 300
Tyr Gly Leu Leu Pro Arg Pro Leu Leu His Thr Ile Ala Ser Leu Phe
305                 310                 315                 320
Phe Ala Phe Val Val Val Ile Gly Ile Tyr Lys Phe Ala Ile Pro Pro
                325                 330                 335
Glu Glu Leu Ile Val Phe Cys Gly Leu Leu Tyr Leu Ile Tyr Asp Pro
            340                 345                 350
Ile Lys Lys Phe Gly Asp Glu Asn Thr Ser Ile Met Arg Gly Cys Ala
        355                 360                 365
Ala Ala Glu Arg Phe Tyr Glu Val Leu Asn His Pro Asp Leu His Ser
    370                 375                 380
Gln Lys Glu Arg Glu Ile Glu Phe Leu Gly Leu Ser Asn Thr Ile Thr
385                 390                 395                 400
Phe Glu Asn Val Ser Phe Gly Tyr Gln Glu Asp Lys His Ile Leu Lys
                405                 410                 415
Asn Leu Ser Phe Thr Leu His Lys Gly Glu Ala Leu Gly Ile Val Gly
            420                 425                 430
Pro Thr Gly Ser Gly Lys Thr Thr Leu Val Lys Leu Leu Pro Arg Leu
        435                 440                 445
Tyr Glu Val Ser Gln Gly Lys Ile Leu Ile Asp Ser Leu Pro Ile Thr
    450                 455                 460
Glu Tyr Asn Lys Gly Ser Leu Arg Asn His Ile Ala Cys Val Leu Gln
465                 470                 475                 480
Asn Pro Phe Leu Phe Tyr Asp Thr Val Trp Asn Asn Leu Thr Cys Gly
                485                 490                 495
Lys Asp Met Glu Glu Glu Ala Val Leu Glu Ala Leu Lys Arg Ala Tyr
            500                 505                 510
Ala Asp Glu Phe Ile Leu Lys Leu Pro Lys Gly Val His Ser Val Leu
        515                 520                 525
Glu Glu Ser Gly Lys Asn Leu Ser Gly Gly Gln Gln Gln Arg Leu Ala
    530                 535                 540
Ile Ala Arg Ala Leu Leu Lys Asn Ala Ser Ile Leu Ile Leu Asp Glu
545                 550                 555                 560
Ala Thr Ser Ala Leu Asp Ala Ile Ser Glu Asn Tyr Ile Lys Asn Ile
                565                 570                 575
Ile Gly Glu Leu Lys Gly Gln Cys Thr Gln Ile Ile Ile Ala His Lys
            580                 585                 590
Leu Thr Thr Leu Glu His Val Asp Arg Val Leu Tyr Ile Glu Asn Gly
        595                 600                 605
Gln Lys Ile Ala Glu Gly Thr Lys Glu Glu Leu Leu Gln Thr Cys Pro
    610                 615                 620
Glu Phe Leu Lys Met Trp Glu Leu Ser Gly Thr Lys Glu Tyr Asn Arg
625                 630                 635                 640
Val Phe Val Pro Asp His Lys Leu Val Ala Asn Pro Thr Asp Met Ala
                645                 650                 655
Ile Thr Thr

<210> 11
<211> 450
<212> DNA
<213> Chlamydia pneumoniae

<220>
<221> CDS
<222> (1)..(450)

<400> 11
atg gct gtt caa tct ata aaa gaa gcc gta aca tca gcc gca aca tca     48
Met Ala Val Gln Ser Ile Lys Glu Ala Val Thr Ser Ala Ala Thr Ser
1               5                   10                  15

gta gga tgt gta aac tgt tct aga gag gct ata cca gca ttt aat aca     96
Val Gly Cys Val Asn Cys Ser Arg Glu Ala Ile Pro Ala Phe Asn Thr
            20                  25                  30

gag gag aga gca acg agt att gct aga tct gtt ata gca gct atc att     144
Glu Glu Arg Ala Thr Ser Ile Ala Arg Ser Val Ile Ala Ala Ile Ile
        35                  40                  45

gct gtt gta gct atc tcc tta ctc gga cta ggt ctt gta gtt ctt gct     192
Ala Val Val Ala Ile Ser Leu Leu Gly Leu Gly Leu Val Val Leu Ala
    50                  55                  60

ggt tgc tgt cct tta gga atg gct gcg ggt gct ata aca atg ctg ctg     240
Gly Cys Cys Pro Leu Gly Met Ala Ala Gly Ala Ile Thr Met Leu Leu
65                  70                  75                  80

ggt gta gca tta tta gct tgg gca ata ctg att act ttg aga ctg ctt     288
Gly Val Ala Leu Leu Ala Trp Ala Ile Leu Ile Thr Leu Arg Leu Leu
                85                  90                  95

aat ata cct aag gct gaa ata ccg agt cca ggg aac aac ggt gag cct     336
Asn Ile Pro Lys Ala Glu Ile Pro Ser Pro Gly Asn Asn Gly Glu Pro
            100                 105                 110

aat gaa aga aat tca gca act cct cct cta gag ggt ggt gtt gca gga     384
Asn Glu Arg Asn Ser Ala Thr Pro Pro Leu Glu Gly Gly Val Ala Gly
        115                 120                 125

gaa gcc ggt cgc ggc ggg ggg tca cct tta acc caa ctt gat ctc aat     432
Glu Ala Gly Arg Gly Gly Gly Ser Pro Leu Thr Gln Leu Asp Leu Asn
    130                 135                 140

tca ggg gcg gga agt tag                                             450

Ser Gly Ala Gly Ser
145                 150

<210> 12
<211> 149
<212> PRT
<213> Chlamydia pneumoniae

<400> 12
Met Ala Val Gln Ser Ile Lys Glu Ala Val Thr Ser Ala Ala Thr Ser
1               5                   10                  15
Val Gly Cys Val Asn Cys Ser Arg Glu Ala Ile Pro Ala Phe Asn Thr
            20                  25                  30
Glu Glu Arg Ala Thr Ser Ile Ala Arg Ser Val Ile Ala Ala Ile Ile
        35                  40                  45
Ala Val Val Ala Ile Ser Leu Leu Gly Leu Gly Leu Val Val Leu Ala
    50                  55                  60
Gly Cys Cys Pro Leu Gly Met Ala Ala Gly Ala Ile Thr Met Leu Leu
65                  70                  75                  80
Gly Val Ala Leu Leu Ala Trp Ala Ile Leu Ile Thr Leu Arg Leu Leu
                85                  90                  95
Asn Ile Pro Lys Ala Glu Ile Pro Ser Pro Gly Asn Asn Gly Glu Pro
            100                 105                 110
Asn Glu Arg Asn Ser Ala Thr Pro Pro Leu Glu Gly Gly Val Ala Gly
        115                 120                 125
Glu Ala Gly Arg Gly Gly Gly Ser Pro Leu Thr Gln Leu Asp Leu Asn
    130                 135                 140
Ser Gly Ala Gly Ser
145

<210> 13
<211> 486
<212> DNA
<213> Chlamydia pneumoniae

<220>
<221> CDS
<222> (1)..(486)

<400> 13
atg agc agt tcg gaa gtt gtt ttc cag aca gtt cat ggc ctt ggc ttt     48
Met Ser Ser Ser Glu Val Val Phe Gln Thr Val His Gly Leu Gly Phe
1               5                   10                  15

ggt gga ttg tct tca aaa agt gtt gtc cct ttt aag aaa agt ctt tcg     96

Gly Gly Leu Ser Ser Lys Ser Val Val Pro Phe Lys Lys Ser Leu Ser
            20                  25                  30

gat gcg ccc cgt gtt gtg tgc tcg att tta gtt ttg act ctg ggg ttg 144
Asp Ala Pro Arg Val Val Cys Ser Ile Leu Val Leu Thr Leu Gly Leu
        35                  40                  45

gga gcg ctt gtt tgt ggt att gcc att act tgt tgg tgt gtc ccg gga 192
Gly Ala Leu Val Cys Gly Ile Ala Ile Thr Cys Trp Cys Val Pro Gly
    50                  55                  60

gtt att tta atg ggg gga att tgc gct ata gtt tta ggt gca att tct 240
Val Ile Leu Met Gly Gly Ile Cys Ala Ile Val Leu Gly Ala Ile Ser
65                  70                  75                  80

tta gct tta agt cta ttt tgg ttg tgg ggt tta ttt tct aat tgt tgt 288
Leu Ala Leu Ser Leu Phe Trp Leu Trp Gly Leu Phe Ser Asn Cys Cys
                85                  90                  95

ggt tct aag aga gtt tta ccg ggt gag gga ttg cta cgg gat aag ctt 336
Gly Ser Lys Arg Val Leu Pro Gly Glu Gly Leu Leu Arg Asp Lys Leu
            100                 105                 110

tta gat ggt gga ttt tca aga gcg gca cct tca gga atg gga ctt ccg 384
Leu Asp Gly Gly Phe Ser Arg Ala Ala Pro Ser Gly Met Gly Leu Pro
        115                 120                 125

ggt gat gga tct cca aga gcg tca acg cca tct tgc cta gag gaa ctt 432
Gly Asp Gly Ser Pro Arg Ala Ser Thr Pro Ser Cys Leu Glu Glu Leu
    130                 135                 140

caa gca gag ata cag gca gtt act caa gct atc gat cag atg tca gat 480
Gln Ala Glu Ile Gln Ala Val Thr Gln Ala Ile Asp Gln Met Ser Asp
145                 150                 155                 160

gat tga 486
Asp

<210> 14
<211> 161
<212> PRT
<213> Chlamydia pneumoniae

<400> 14
Met Ser Ser Ser Glu Val Val Phe Gln Thr Val His Gly Leu Gly Phe
1               5                   10                  15
Gly Gly Leu Ser Ser Lys Ser Val Val Pro Phe Lys Lys Ser Leu Ser

20                   25                   30
Asp Ala Pro Arg Val Val Cys Ser Ile Leu Val Leu Thr Leu Gly Leu
         35                  40                  45
Gly Ala Leu Val Cys Gly Ile Ala Ile Thr Cys Trp Cys Val Pro Gly
     50                  55                  60
Val Ile Leu Met Gly Gly Ile Cys Ala Ile Val Leu Gly Ala Ile Ser
 65                  70                  75                  80
Leu Ala Leu Ser Leu Phe Trp Leu Trp Gly Leu Phe Ser Asn Cys Cys
                 85                  90                  95
Gly Ser Lys Arg Val Leu Pro Gly Glu Gly Leu Leu Arg Asp Lys Leu
            100                 105                 110
Leu Asp Gly Gly Phe Ser Arg Ala Ala Pro Ser Gly Met Gly Leu Pro
        115                 120                 125
Gly Asp Gly Ser Pro Arg Ala Ser Thr Pro Ser Cys Leu Glu Glu Leu
    130                 135                 140
Gln Ala Glu Ile Gln Ala Val Thr Gln Ala Ile Asp Gln Met Ser Asp
145                 150                 155                 160
Asp

<210> 15
<211> 1170
<212> DNA
<213> Chlamydia pneumoniae

<220>
<221> CDS
<222> (1)..(1170)

<400> 15
atg gga act cct ata tct ggc aat gat ggt gac cgt aat acg ata tca    48
Met Gly Thr Pro Ile Ser Gly Asn Asp Gly Asp Arg Asn Thr Ile Ser
  1               5                  10                  15

gat cct tta gaa gaa agt gcc gca gaa gag ggg gat tca gat tta gag    96
Asp Pro Leu Glu Glu Ser Ala Ala Glu Glu Gly Asp Ser Asp Leu Glu
             20                  25                  30

gat cgg gta tcg gaa agt gct acc caa gtg ata gaa acc ata gcg gat   144
Asp Arg Val Ser Glu Ser Ala Thr Gln Val Ile Glu Thr Ile Ala Asp
         35                  40                  45

acg ggg atc cca gaa gca act cca tct gaa ggt acg aat agt gat tta   192
Thr Gly Ile Pro Glu Ala Thr Pro Ser Glu Gly Thr Asn Ser Asp Leu
     50                  55                  60

aat agc gac ctt gta gat aga gta gaa tat gaa gct cgc gga agc ctg   240

Asn Ser Asp Leu Val Asp Arg Val Glu Tyr Glu Ala Arg Gly Ser Leu
65                  70                  75                  80

tta act acg atg ctt gcg cgg att cgt aaa gca gta tcg cag att tgg     288
Leu Thr Thr Met Leu Ala Arg Ile Arg Lys Ala Val Ser Gln Ile Trp
                85                  90                  95

atg cat gtt aaa aca aaa cgc cat cca aaa gaa cag gga gtg cgt tcc     336
Met His Val Lys Thr Lys Arg His Pro Lys Glu Gln Gly Val Arg Ser
            100                 105                 110

ttg ggg gac atc cct tgt gat ctt ctc aaa gca acg cga ctc cct aaa     384
Leu Gly Asp Ile Pro Cys Asp Leu Leu Lys Ala Thr Arg Leu Pro Lys
        115                 120                 125

gaa act gcg gaa cct cca tac ttt tat gct tta gaa aca gca cta gct     432
Glu Thr Ala Glu Pro Pro Tyr Phe Tyr Ala Leu Glu Thr Ala Leu Ala
    130                 135                 140

tca tgc cga agc ttt ttc ttt cat gta ttt tta agg cta ttc act ctt     480
Ser Cys Arg Ser Phe Phe Phe His Val Phe Leu Arg Leu Phe Thr Leu
145                 150                 155                 160

tta cgt cgt caa cac cca gag gct cct tta gac ctt tgt ggt aca gat     528
Leu Arg Arg Gln His Pro Glu Ala Pro Leu Asp Leu Cys Gly Thr Asp
                165                 170                 175

cct ata agt cca gaa gct gca gtt gca ttt gct tta atc tta cgt tct     576
Pro Ile Ser Pro Glu Ala Ala Val Ala Phe Ala Leu Ile Leu Arg Ser
            180                 185                 190

tgc tgc aag tgg gta gct acg gac gca gtt caa gaa gga ctg cct tta     624
Cys Cys Lys Trp Val Ala Thr Asp Ala Val Gln Glu Gly Leu Pro Leu
        195                 200                 205

gaa gtc atc gaa gag gca gga atg tat aat gcg ttt tct tta gaa gct     672
Glu Val Ile Glu Glu Ala Gly Met Tyr Asn Ala Phe Ser Leu Glu Ala
    210                 215                 220

aca aca aca gta gaa gaa gtc tcc aaa agg ctc tcc gaa cta tta tac     720
Thr Thr Thr Val Glu Glu Val Ser Lys Arg Leu Ser Glu Leu Leu Tyr
225                 230                 235                 240

tca gac aaa cgt att gat ggg tta gct aac gtc cgt ggg att act aag     768
Ser Asp Lys Arg Ile Asp Gly Leu Ala Asn Val Arg Gly Ile Thr Lys
                245                 250                 255

ata att acc tct cct tat tta gga gct ggg caa tgc gtc agc gtt gtt     816

Ile Ile Thr Ser Pro Tyr Leu Gly Ala Gly Gln Cys Val Ser Val Val
            260                 265                 270

gac aac cta aaa aca tat gat ctt ggt cgt aac tat act cag gta ctt     864
Asp Asn Leu Lys Thr Tyr Asp Leu Gly Arg Asn Tyr Thr Gln Val Leu
        275                 280                 285

gcc tgt gcc tcc caa att gat gag ttt gcc gat aaa gga gag aac gaa     912
Ala Cys Ala Ser Gln Ile Asp Glu Phe Ala Asp Lys Gly Glu Asn Glu
    290                 295                 300

gct ctc gtt atg aaa gac atc ctc tat tta gta cgt caa gat cgt agc     960
Ala Leu Val Met Lys Asp Ile Leu Tyr Leu Val Arg Gln Asp Arg Ser
305                 310                 315                 320

aaa gag ctc gga gac ttt tta atg atg tgg tca gaa gag cac gcc tca     1008
Lys Glu Leu Gly Asp Phe Leu Met Met Trp Ser Glu Glu His Ala Ser
                325                 330                 335

gaa gta aac tat gat gtc gtt ctc gct atc tta gaa gta aat ctc cct     1056
Glu Val Asn Tyr Asp Val Val Leu Ala Ile Leu Glu Val Asn Leu Pro
            340                 345                 350

atc tta gaa gaa gac tat cgc tcg cat ccc cta gca tat cag aag aaa     1104
Ile Leu Glu Glu Asp Tyr Arg Ser His Pro Leu Ala Tyr Gln Lys Lys
        355                 360                 365

tta aac tat gtg atc tgt cag ttt ttc tgt agc gag cgt ctg aca tca     1152
Leu Asn Tyr Val Ile Cys Gln Phe Phe Cys Ser Glu Arg Leu Thr Ser
    370                 375                 380

ata gag ccc aaa gac tag                                             1170
Ile Glu Pro Lys Asp
385                 390

<210> 16
<211> 389
<212> PRT
<213> Chlamydia pneumoniae

<400> 16
Met Gly Thr Pro Ile Ser Gly Asn Asp Gly Asp Arg Asn Thr Ile Ser
  1               5                  10                  15
Asp Pro Leu Glu Glu Ser Ala Ala Glu Glu Gly Asp Ser Asp Leu Glu
             20                  25                  30
Asp Arg Val Ser Glu Ser Ala Thr Gln Val Ile Glu Thr Ile Ala Asp
         35                  40                  45

Thr Gly Ile Pro Glu Ala Thr Pro Ser Glu Gly Thr Asn Ser Asp Leu
    50                  55                  60
Asn Ser Asp Leu Val Asp Arg Val Glu Tyr Glu Ala Arg Gly Ser Leu
65                  70                  75                  80
Leu Thr Thr Met Leu Ala Arg Ile Arg Lys Ala Val Ser Gln Ile Trp
                85                  90                  95
Met His Val Lys Thr Lys Arg His Pro Lys Glu Gln Gly Val Arg Ser
            100                 105                 110
Leu Gly Asp Ile Pro Cys Asp Leu Leu Lys Ala Thr Arg Leu Pro Lys
        115                 120                 125
Glu Thr Ala Glu Pro Pro Tyr Phe Tyr Ala Leu Glu Thr Ala Leu Ala
    130                 135                 140
Ser Cys Arg Ser Phe Phe Phe His Val Phe Leu Arg Leu Phe Thr Leu
145                 150                 155                 160
Leu Arg Arg Gln His Pro Glu Ala Pro Leu Asp Leu Cys Gly Thr Asp
                165                 170                 175
Pro Ile Ser Pro Glu Ala Ala Val Ala Phe Ala Leu Ile Leu Arg Ser
            180                 185                 190
Cys Cys Lys Trp Val Ala Thr Asp Ala Val Gln Glu Gly Leu Pro Leu
        195                 200                 205
Glu Val Ile Glu Glu Ala Gly Met Tyr Asn Ala Phe Ser Leu Glu Ala
    210                 215                 220
Thr Thr Thr Val Glu Glu Val Ser Lys Arg Leu Ser Glu Leu Leu Tyr
225                 230                 235                 240
Ser Asp Lys Arg Ile Asp Gly Leu Ala Asn Val Arg Gly Ile Thr Lys
                245                 250                 255
Ile Ile Thr Ser Pro Tyr Leu Gly Ala Gly Gln Cys Val Ser Val Val
            260                 265                 270
Asp Asn Leu Lys Thr Tyr Asp Leu Gly Arg Asn Tyr Thr Gln Val Leu
        275                 280                 285
Ala Cys Ala Ser Gln Ile Asp Glu Phe Ala Asp Lys Gly Glu Asn Glu
    290                 295                 300
Ala Leu Val Met Lys Asp Ile Leu Tyr Leu Val Arg Gln Asp Arg Ser
305                 310                 315                 320
Lys Glu Leu Gly Asp Phe Leu Met Met Trp Ser Glu Glu His Ala Ser
                325                 330                 335
Glu Val Asn Tyr Asp Val Val Leu Ala Ile Leu Glu Val Asn Leu Pro
            340                 345                 350
Ile Leu Glu Glu Asp Tyr Arg Ser His Pro Leu Ala Tyr Gln Lys Lys
        355                 360                 365
Leu Asn Tyr Val Ile Cys Gln Phe Phe Cys Ser Glu Arg Leu Thr Ser
    370                 375                 380
Ile Glu Pro Lys Asp
385

<210> 17

<211> 1035
<212> DNA
<213> Chlamydia pneumoniae

<220>
<221> CDS
<222> (1)..(1035)

<400> 17
atg ctc acc cta ggc ttg gaa agt tct tgc gat gag act gcc tgc gct    48
Met Leu Thr Leu Gly Leu Glu Ser Ser Cys Asp Glu Thr Ala Cys Ala
1               5                   10                  15

ata gtt aat gag gat aag cag ata tta gca aat att att gcc tct caa    96
Ile Val Asn Glu Asp Lys Gln Ile Leu Ala Asn Ile Ile Ala Ser Gln
            20                  25                  30

gat atc cat gca tcc tat ggc gga gtc gtt cct gaa ctt gct tca aga    144
Asp Ile His Ala Ser Tyr Gly Gly Val Val Pro Glu Leu Ala Ser Arg
        35                  40                  45

gca cat ctc cat atc ttc cca caa gtg ata aat aaa gct cta caa cag    192
Ala His Leu His Ile Phe Pro Gln Val Ile Asn Lys Ala Leu Gln Gln
    50                  55                  60

gcc aac tta ttg atc gaa gat atg gat ctg att gca gta acg caa act    240
Ala Asn Leu Leu Ile Glu Asp Met Asp Leu Ile Ala Val Thr Gln Thr
65                  70                  75                  80

cca ggg ttg ata ggt tct cta tca gta gga gtg cat ttt ggt aaa ggc    288
Pro Gly Leu Ile Gly Ser Leu Ser Val Gly Val His Phe Gly Lys Gly
                85                  90                  95

att gcc ata gga gca aaa aaa tcc ttg att gga gtc aat cac gtc gaa    336
Ile Ala Ile Gly Ala Lys Lys Ser Leu Ile Gly Val Asn His Val Glu
            100                 105                 110

gct cat ctc tat gct gcc tat atg gca gcg caa aac gtg caa ttc cct    384
Ala His Leu Tyr Ala Ala Tyr Met Ala Ala Gln Asn Val Gln Phe Pro
        115                 120                 125

gct tta ggt ctt gtg gtc tct gga gct cat acc gca gcg ttt ttt ata    432
Ala Leu Gly Leu Val Val Ser Gly Ala His Thr Ala Ala Phe Phe Ile
    130                 135                 140

gaa aat cct aca tcc tat aaa ctc ata gga aaa act cga gat gat gct    480
Glu Asn Pro Thr Ser Tyr Lys Leu Ile Gly Lys Thr Arg Asp Asp Ala
145                 150                 155                 160

ata gga gaa act ttt gat aaa gta gga cgc ttt cta gga tta cca tac    528
Ile Gly Glu Thr Phe Asp Lys Val Gly Arg Phe Leu Gly Leu Pro Tyr
                165                 170                 175

cct gca ggc cca tta att gaa aaa ctc gct tta gaa ggc tct gag gac    576
Pro Ala Gly Pro Leu Ile Glu Lys Leu Ala Leu Glu Gly Ser Glu Asp
            180                 185                 190

agt tat cct ttt agt cca gct aaa gtc cca aac tat gac ttt tca ttc    624
Ser Tyr Pro Phe Ser Pro Ala Lys Val Pro Asn Tyr Asp Phe Ser Phe
        195                 200                 205

agc ggt ctt aaa aca gct gtt ctc tac gca atc aaa gga aat aat agt    672
Ser Gly Leu Lys Thr Ala Val Leu Tyr Ala Ile Lys Gly Asn Asn Ser
    210                 215                 220

agc ccc cgc tct cct gct cca gag ata tct tta gaa aaa caa aga gat    720
Ser Pro Arg Ser Pro Ala Pro Glu Ile Ser Leu Glu Lys Gln Arg Asp
225                 230                 235                 240

atc gct gct tca ttt caa aaa gcg gcc tgc act act att gca caa aaa    768
Ile Ala Ala Ser Phe Gln Lys Ala Ala Cys Thr Thr Ile Ala Gln Lys
                245                 250                 255

ctt ccc act att ata aaa gaa ttt tcg tgc cga tct ata ctt att gga    816
Leu Pro Thr Ile Ile Lys Glu Phe Ser Cys Arg Ser Ile Leu Ile Gly
            260                 265                 270

ggt ggc gta gcc att aat gaa tac ttt aga tcc gca ata caa act gcg    864
Gly Gly Val Ala Ile Asn Glu Tyr Phe Arg Ser Ala Ile Gln Thr Ala
        275                 280                 285

tgt aat cta cct gta tac ttc ccc cct gct aaa cta tgc tca gat aat    912
Cys Asn Leu Pro Val Tyr Phe Pro Pro Ala Lys Leu Cys Ser Asp Asn
    290                 295                 300

gct gct atg att gca ggt cta ggg gga gaa aat ttt caa aaa aac tct    960
Ala Ala Met Ile Ala Gly Leu Gly Gly Glu Asn Phe Gln Lys Asn Ser
305                 310                 315                 320

agt att ccg gaa att cgt ata tgc gca aga tat cag tgg gaa tct gta    1008
Ser Ile Pro Glu Ile Arg Ile Cys Ala Arg Tyr Gln Trp Glu Ser Val
                325                 330                 335

tca cca ttc tcc tta gcc tct ccg tag                                1035
Ser Pro Phe Ser Leu Ala Ser Pro
            340                 345

<210> 18
<211> 344
<212> PRT
<213> Chlamydia pneumoniae

<400> 18
Met Leu Thr Leu Gly Leu Glu Ser Ser Cys Asp Glu Thr Ala Cys Ala
1               5                   10                  15
Ile Val Asn Glu Asp Lys Gln Ile Leu Ala Asn Ile Ile Ala Ser Gln
            20                  25                  30
Asp Ile His Ala Ser Tyr Gly Gly Val Val Pro Glu Leu Ala Ser Arg
        35                  40                  45
Ala His Leu His Ile Phe Pro Gln Val Ile Asn Lys Ala Leu Gln Gln
    50                  55                  60
Ala Asn Leu Leu Ile Glu Asp Met Asp Leu Ile Ala Val Thr Gln Thr
65                  70                  75                  80
Pro Gly Leu Ile Gly Ser Leu Ser Val Gly Val His Phe Gly Lys Gly
                85                  90                  95
Ile Ala Ile Gly Ala Lys Lys Ser Leu Ile Gly Val Asn His Val Glu
            100                 105                 110
Ala His Leu Tyr Ala Ala Tyr Met Ala Ala Gln Asn Val Gln Phe Pro
        115                 120                 125
Ala Leu Gly Leu Val Val Ser Gly Ala His Thr Ala Ala Phe Phe Ile
    130                 135                 140
Glu Asn Pro Thr Ser Tyr Lys Leu Ile Gly Lys Thr Arg Asp Asp Ala
145                 150                 155                 160
Ile Gly Glu Thr Phe Asp Lys Val Gly Arg Phe Leu Gly Leu Pro Tyr
                165                 170                 175
Pro Ala Gly Pro Leu Ile Glu Lys Leu Ala Leu Glu Gly Ser Glu Asp
            180                 185                 190
Ser Tyr Pro Phe Ser Pro Ala Lys Val Pro Asn Tyr Asp Phe Ser Phe
        195                 200                 205
Ser Gly Leu Lys Thr Ala Val Leu Tyr Ala Ile Lys Gly Asn Asn Ser
    210                 215                 220
Ser Pro Arg Ser Pro Ala Pro Glu Ile Ser Leu Glu Lys Gln Arg Asp
225                 230                 235                 240
Ile Ala Ala Ser Phe Gln Lys Ala Ala Cys Thr Thr Ile Ala Gln Lys
                245                 250                 255
Leu Pro Thr Ile Ile Lys Glu Phe Ser Cys Arg Ser Ile Leu Ile Gly
            260                 265                 270
Gly Gly Val Ala Ile Asn Glu Tyr Phe Arg Ser Ala Ile Gln Thr Ala
        275                 280                 285
Cys Asn Leu Pro Val Tyr Phe Pro Pro Ala Lys Leu Cys Ser Asp Asn
    290                 295                 300
Ala Ala Met Ile Ala Gly Leu Gly Gly Glu Asn Phe Gln Lys Asn Ser
305                 310                 315                 320

Ser Ile Pro Glu Ile Arg Ile Cys Ala Arg Tyr Gln Trp Glu Ser Val
                325                 330                 335
Ser Pro Phe Ser Leu Ala Ser Pro
            340

<210> 19
<211> 1214
<212> DNA
<213> Chlamydia pneumoniae

<220>
<221> CDS
<222> (1)..(1212)

<400> 19
atg ggg ctg caa tcc agg tta caa cat tgt ata gaa gtg tcc cag aat 48
Met Gly Leu Gln Ser Arg Leu Gln His Cys Ile Glu Val Ser Gln Asn
1               5                   10                  15

tcg aac ttt gat tca caa gta aaa cag ttt atc tat gcg tgc caa gat 96
Ser Asn Phe Asp Ser Gln Val Lys Gln Phe Ile Tyr Ala Cys Gln Asp
            20                  25                  30

aag aca tta agg cag tct gta ctc aag att ttc cgc tac cat cct tta 144
Lys Thr Leu Arg Gln Ser Val Leu Lys Ile Phe Arg Tyr His Pro Leu
        35                  40                  45

cta aaa att cat gat att gct cgg gcc gtc tat ctt ttg atg gcc tta 192
Leu Lys Ile His Asp Ile Ala Arg Ala Val Tyr Leu Leu Met Ala Leu
    50                  55                  60

gaa gaa ggc gag gat tta ggc tta agc ttt tta aat gta cag cag tac 240
Glu Glu Gly Glu Asp Leu Gly Leu Ser Phe Leu Asn Val Gln Gln Tyr
65                  70                  75                  80

cct tca ggt gct gta gaa ctg ttt tct tgt ggg gga ttt cct tgg aaa 288
Pro Ser Gly Ala Val Glu Leu Phe Ser Cys Gly Gly Phe Pro Trp Lys
                85                  90                  95

gga tta cct tat cct gca gaa cat gcg gaa ttt ggc cta ctc ctg tta 336
Gly Leu Pro Tyr Pro Ala Glu His Ala Glu Phe Gly Leu Leu Leu Leu
            100                 105                 110

cag atc gca gag ttt tat gaa gag agt cag gca tac gtc tct aaa atg 384
Gln Ile Ala Glu Phe Tyr Glu Glu Ser Gln Ala Tyr Val Ser Lys Met
        115                 120                 125

agt cat ttt caa cag gca ctc ttt gat cac caa ggg agc gtc ttt ccc 432
Ser His Phe Gln Gln Ala Leu Phe Asp His Gln Gly Ser Val Phe Pro
    130                 135                 140

tct ctc tgg agc cag gag aac tct cga ctc cta aaa gaa aag aca act 480
Ser Leu Trp Ser Gln Glu Asn Ser Arg Leu Leu Lys Glu Lys Thr Thr
145                 150                 155                 160

ctt agc caa tcg ttt ctc ttc caa tta gga atg caa att cac cca gaa 528
Leu Ser Gln Ser Phe Leu Phe Gln Leu Gly Met Gln Ile His Pro Glu
                165                 170                 175

tac agt ctt gag gat cct gca cta ggg ttc tgg atg caa aga acg cgt 576
Tyr Ser Leu Glu Asp Pro Ala Leu Gly Phe Trp Met Gln Arg Thr Arg
            180                 185                 190

tct tca tcc gct ttt gta gcc gct tca gga tgt caa agt agc ttg gga 624
Ser Ser Ser Ala Phe Val Ala Ala Ser Gly Cys Gln Ser Ser Leu Gly
        195                 200                 205

gcg tat tcc tca ggg gat gtc ggt gtt atc gct tat gga cct tgc tct 672
Ala Tyr Ser Ser Gly Asp Val Gly Val Ile Ala Tyr Gly Pro Cys Ser
    210                 215                 220

gga gac att agt gat tgt tat tat ttt gga tgt tgt gga atc gct aaa 720
Gly Asp Ile Ser Asp Cys Tyr Tyr Phe Gly Cys Cys Gly Ile Ala Lys
225                 230                 235                 240

gag ttc gtg tgc caa aaa tct cac caa act aca gag att tct ttt ctc 768
Glu Phe Val Cys Gln Lys Ser His Gln Thr Thr Glu Ile Ser Phe Leu
                245                 250                 255

acc tct aca gga aag cct cat ccc aga aat acg gga ttt tcc tac ctt 816
Thr Ser Thr Gly Lys Pro His Pro Arg Asn Thr Gly Phe Ser Tyr Leu
            260                 265                 270

cga gat tcc tat gta cat ctg ccg atc cgc tgt aag atc act att tcc 864
Arg Asp Ser Tyr Val His Leu Pro Ile Arg Cys Lys Ile Thr Ile Ser
        275                 280                 285

gac aag caa tat cgc gtg cac gct gcg ttg gct gag gcc acc tct gcc 912
Asp Lys Gln Tyr Arg Val His Ala Ala Leu Ala Glu Ala Thr Ser Ala
    290                 295                 300

atg acg ttt tct att ttc tgt aag ggg aag aat tgt cag gtt gtt gac 960
Met Thr Phe Ser Ile Phe Cys Lys Gly Lys Asn Cys Gln Val Val Asp
305                 310                 315                 320

ggc cct cgc ttg cgc tcc tgt tcc cta gat tct tat aaa ggt ccc gga 1008
Gly Pro Arg Leu Arg Ser Cys Ser Leu Asp Ser Tyr Lys Gly Pro Gly
                325                 330                 335

aac gac att atg att ctt ggg gaa aat gac gca atc aac att gtt tct 1056
Asn Asp Ile Met Ile Leu Gly Glu Asn Asp Ala Ile Asn Ile Val Ser
            340                 345                 350

gca agt ccc tat atg gaa att ttt gct ttg caa ggc aaa gaa aaa ttt 1104
Ala Ser Pro Tyr Met Glu Ile Phe Ala Leu Gln Gly Lys Glu Lys Phe
        355                 360                 365

tgg aat gca gac ttt ttg att aat att cct tac aaa gaa gag ggc gtc 1152
Trp Asn Ala Asp Phe Leu Ile Asn Ile Pro Tyr Lys Glu Glu Gly Val
    370                 375                 380

atg tta att ttt gaa aaa aaa gtg acc tct gag aaa gga aga ttc ttt 1200
Met Leu Ile Phe Glu Lys Lys Val Thr Ser Glu Lys Gly Arg Phe Phe
385                 390                 395                 400

acg aag atg aat ta 1214
Thr Lys Met Asn

<210> 20
<211> 404
<212> PRT
<213> Chlamydia pneumoniae

<400> 20
Met Gly Leu Gln Ser Arg Leu Gln His Cys Ile Glu Val Ser Gln Asn
1               5                   10                  15

Ser Asn Phe Asp Ser Gln Val Lys Gln Phe Ile Tyr Ala Cys Gln Asp
            20                  25                  30

Lys Thr Leu Arg Gln Ser Val Leu Lys Ile Phe Arg Tyr His Pro Leu
        35                  40                  45

Leu Lys Ile His Asp Ile Ala Arg Ala Val Tyr Leu Leu Met Ala Leu
    50                  55                  60

Glu Glu Gly Glu Asp Leu Gly Leu Ser Phe Leu Asn Val Gln Gln Tyr
65                  70                  75                  80

Pro Ser Gly Ala Val Glu Leu Phe Ser Cys Gly Gly Phe Pro Trp Lys
                85                  90                  95

Gly Leu Pro Tyr Pro Ala Glu His Ala Glu Phe Gly Leu Leu Leu Leu
            100                 105                 110

Gln Ile Ala Glu Phe Tyr Glu Glu Ser Gln Ala Tyr Val Ser Lys Met
        115                 120                 125

Ser His Phe Gln Gln Ala Leu Phe Asp His Gln Gly Ser Val Phe Pro
    130                 135                 140

Ser Leu Trp Ser Gln Glu Asn Ser Arg Leu Leu Lys Glu Lys Thr Thr
145                 150                 155                 160

Leu Ser Gln Ser Phe Leu Phe Gln Leu Gly Met Gln Ile His Pro Glu
                165                 170                 175

Tyr Ser Leu Glu Asp Pro Ala Leu Gly Phe Trp Met Gln Arg Thr Arg
            180                 185                 190

Ser Ser Ser Ala Phe Val Ala Ala Ser Gly Cys Gln Ser Ser Leu Gly
        195                 200                 205

Ala Tyr Ser Ser Gly Asp Val Gly Val Ile Ala Tyr Gly Pro Cys Ser
    210                 215                 220

Gly Asp Ile Ser Asp Cys Tyr Tyr Phe Gly Cys Cys Gly Ile Ala Lys
225                 230                 235                 240

Glu Phe Val Cys Gln Lys Ser His Gln Thr Thr Glu Ile Ser Phe Leu
                245                 250                 255

Thr Ser Thr Gly Lys Pro His Pro Arg Asn Thr Gly Phe Ser Tyr Leu
            260                 265                 270

Arg Asp Ser Tyr Val His Leu Pro Ile Arg Cys Lys Ile Thr Ile Ser
        275                 280                 285

Asp Lys Gln Tyr Arg Val His Ala Ala Leu Ala Glu Ala Thr Ser Ala
    290                 295                 300

Met Thr Phe Ser Ile Phe Cys Lys Gly Lys Asn Cys Gln Val Val Asp
305                 310                 315                 320

Gly Pro Arg Leu Arg Ser Cys Ser Leu Asp Ser Tyr Lys Gly Pro Gly
                325                 330                 335

Asn Asp Ile Met Ile Leu Gly Glu Asn Asp Ala Ile Asn Ile Val Ser
            340                 345                 350

Ala Ser Pro Tyr Met Glu Ile Phe Ala Leu Gln Gly Lys Glu Lys Phe
        355                 360                 365

Trp Asn Ala Asp Phe Leu Ile Asn Ile Pro Tyr Lys Glu Glu Gly Val
    370                 375                 380

Met Leu Ile Phe Glu Lys Lys Val Thr Ser Glu Lys Gly Arg Phe Phe
385                 390                 395                 400

Thr Lys Met Asn

<210> 21
<211> 1149
<212> DNA
<213> Chlamydia pneumoniae

<220>
<221> CDS
<222> (1)..(1149)

<400> 21
atg gga atc aat cct tcg ggt aat aga tca cca gat gat gta tgg gtt      48
Met Gly Ile Asn Pro Ser Gly Asn Arg Ser Pro Asp Asp Val Trp Val
  1               5                  10                  15

aga gga gct caa ggc gat agc tcc agt acc caa ggt aca gga gct aca      96
Arg Gly Ala Gln Gly Asp Ser Ser Ser Thr Gln Gly Thr Gly Ala Thr
             20                  25                  30

aac tca aat ctt ggt gct cac aac gta act aca tca acc tca cag ccg     144
Asn Ser Asn Leu Gly Ala His Asn Val Thr Thr Ser Thr Ser Gln Pro
         35                  40                  45

caa gtt gct tct aaa gca aag cag tta tgg cag acg gta agg gag ttc     192
Gln Val Ala Ser Lys Ala Lys Gln Leu Trp Gln Thr Val Arg Glu Phe
     50                  55                  60

ttt tta ggg aag aaa tca ccc gat tct tct cag ggt gct tcg gga cct     240
Phe Leu Gly Lys Lys Ser Pro Asp Ser Ser Gln Gly Ala Ser Gly Pro
 65                  70                  75                  80

gca atg caa agt cct tca gga cct aca ata cgg cct acg cgt ccg gca     288
Ala Met Gln Ser Pro Ser Gly Pro Thr Ile Arg Pro Thr Arg Pro Ala
                 85                  90                  95

cct cca cct cct aca acg ggt ggg gct aat gcg aaa cgt ccc gca acg 336
Pro Pro Pro Pro Thr Thr Gly Gly Ala Asn Ala Lys Arg Pro Ala Thr
            100                 105                 110

cat ggg aaa ggt cga gca cct caa cct cct acg gcg ggg tct tct tca 384
His Gly Lys Gly Arg Ala Pro Gln Pro Pro Thr Ala Gly Ser Ser Ser
        115                 120                 125

gga tca gag caa cct act gcc atg agt tct gaa gtc gct aaa ctt gtg 432
Gly Ser Glu Gln Pro Thr Ala Met Ser Ser Glu Val Ala Lys Leu Val
    130                 135                 140

agt gaa tta aaa gat gca gtc cat agt cat gcg gag tct caa aaa gta 480
Ser Glu Leu Lys Asp Ala Val His Ser His Ala Glu Ser Gln Lys Val
145                 150                 155                 160

ctt aaa aag gta tct caa gag cta caa aca aag tgg acg gat tgg gaa 528
Leu Lys Lys Val Ser Gln Glu Leu Gln Thr Lys Trp Thr Asp Trp Glu
                165                 170                 175

aat aat agg ggt cca gac tat ctt ttg cat ggt tat cgt gtc att gct 576
Asn Asn Arg Gly Pro Asp Tyr Leu Leu His Gly Tyr Arg Val Ile Ala
            180                 185                 190

cga gct ttg cag caa aca tac aca gaa caa tct atg ctt atc gaa ggg 624
Arg Ala Leu Gln Gln Thr Tyr Thr Glu Gln Ser Met Leu Ile Glu Gly
        195                 200                 205

act tca tct aca gga cca gtt ccg caa gca gtg act gta gct aag gat 672
Thr Ser Ser Thr Gly Pro Val Pro Gln Ala Val Thr Val Ala Lys Asp
    210                 215                 220

gct gta act cag aca gtt aga ggc gca att aag aat tta gaa aat cct 720
Ala Val Thr Gln Thr Val Arg Gly Ala Ile Lys Asn Leu Glu Asn Pro
225                 230                 235                 240

aag cca ggt aat gat cct gat ggt gta ctc atg caa gtg gtt ata agc 768
Lys Pro Gly Asn Asp Pro Asp Gly Val Leu Met Gln Val Val Ile Ser
                245                 250                 255

tta ggt atc gaa gga cct aca tta gac cca gga gaa tct atc caa aac 816
Leu Gly Ile Glu Gly Pro Thr Leu Asp Pro Gly Glu Ser Ile Gln Asn
            260                 265                 270

ttt tta gaa act agg gtt tcg gat ttc ggt gga gat gat agc gac ata 864
Phe Leu Glu Thr Arg Val Ser Asp Phe Gly Gly Asp Asp Ser Asp Ile
        275                 280                 285

gat tat aca agt gat ata gct cga tta ggg tca gct tta gat cgg gta     912
Asp Tyr Thr Ser Asp Ile Ala Arg Leu Gly Ser Ala Leu Asp Arg Val
    290                 295                 300

cgc gaa aat cat cct aat gag atg cct aga ata tgg ata gca tta gca     960
Arg Glu Asn His Pro Asn Glu Met Pro Arg Ile Trp Ile Ala Leu Ala
305                 310                 315                 320

cga gaa ctc ggt gcg gct gta cac tct cat gct act tcc gtc cga atc     1008
Arg Glu Leu Gly Ala Ala Val His Ser His Ala Thr Ser Val Arg Ile
                325                 330                 335

gca aat gca gga aag aat cac act cgt gac gtt gtg cga atg gcc aat     1056
Ala Asn Ala Gly Lys Asn His Thr Arg Asp Val Val Arg Met Ala Asn
            340                 345                 350

gag tcg agt aga cta ctt caa ggt atg aaa gtg tta tcg gtc gga gct     1104
Glu Ser Ser Arg Leu Leu Gln Gly Met Lys Val Leu Ser Val Gly Ala
        355                 360                 365

tgg gcg aat aca atg aca gtt tta atc ggg gat ctt ttt gaa taa         1149
Trp Ala Asn Thr Met Thr Val Leu Ile Gly Asp Leu Phe Glu
    370                 375                 380

<210> 22
<211> 382
<212> PRT
<213> Chlamydia pneumoniae

<400> 22
Met Gly Ile Asn Pro Ser Gly Asn Arg Ser Pro Asp Asp Val Trp Val
1               5                   10                  15
Arg Gly Ala Gln Gly Asp Ser Ser Ser Thr Gln Gly Thr Gly Ala Thr
            20                  25                  30
Asn Ser Asn Leu Gly Ala His Asn Val Thr Thr Ser Thr Ser Gln Pro
        35                  40                  45
Gln Val Ala Ser Lys Ala Lys Gln Leu Trp Gln Thr Val Arg Glu Phe
    50                  55                  60
Phe Leu Gly Lys Lys Ser Pro Asp Ser Ser Gln Gly Ala Ser Gly Pro
65                  70                  75                  80
Ala Met Gln Ser Pro Ser Gly Pro Thr Ile Arg Pro Thr Arg Pro Ala
                85                  90                  95
Pro Pro Pro Pro Thr Thr Gly Gly Ala Asn Ala Lys Arg Pro Ala Thr
            100                 105                 110
His Gly Lys Gly Arg Ala Pro Gln Pro Pro Thr Ala Gly Ser Ser Ser
        115                 120                 125

Gly Ser Glu Gln Pro Thr Ala Met Ser Ser Glu Val Ala Lys Leu Val
    130                 135                 140
Ser Glu Leu Lys Asp Ala Val His Ser His Ala Glu Ser Gln Lys Val
145                 150                 155                 160
Leu Lys Lys Val Ser Gln Glu Leu Gln Thr Lys Trp Thr Asp Trp Glu
                165                 170                 175
Asn Asn Arg Gly Pro Asp Tyr Leu Leu His Gly Tyr Arg Val Ile Ala
            180                 185                 190
Arg Ala Leu Gln Gln Thr Tyr Thr Glu Gln Ser Met Leu Ile Glu Gly
        195                 200                 205
Thr Ser Ser Thr Gly Pro Val Pro Gln Ala Val Thr Val Ala Lys Asp
    210                 215                 220
Ala Val Thr Gln Thr Val Arg Gly Ala Ile Lys Asn Leu Glu Asn Pro
225                 230                 235                 240
Lys Pro Gly Asn Asp Pro Asp Gly Val Leu Met Gln Val Val Ile Ser
                245                 250                 255
Leu Gly Ile Glu Gly Pro Thr Leu Asp Pro Gly Glu Ser Ile Gln Asn
            260                 265                 270
Phe Leu Glu Thr Arg Val Ser Asp Phe Gly Gly Asp Asp Ser Asp Ile
        275                 280                 285
Asp Tyr Thr Ser Asp Ile Ala Arg Leu Gly Ser Ala Leu Asp Arg Val
    290                 295                 300
Arg Glu Asn His Pro Asn Glu Met Pro Arg Ile Trp Ile Ala Leu Ala
305                 310                 315                 320
Arg Glu Leu Gly Ala Ala Val His Ser His Ala Thr Ser Val Arg Ile
                325                 330                 335
Ala Asn Ala Gly Lys Asn His Thr Arg Asp Val Val Arg Met Ala Asn
            340                 345                 350
Glu Ser Ser Arg Leu Leu Gln Gly Met Lys Val Leu Ser Val Gly Ala
        355                 360                 365
Trp Ala Asn Thr Met Thr Val Leu Ile Gly Asp Leu Phe Glu
    370                 375                 380

<210> 23
<211> 1122
<212> DNA
<213> Chlamydia pneumoniae

<220>
<221> CDS
<222> (1)..(1122)

<400> 23
atg tca ata gct att gca agg gaa caa tac gca gct ata ttg gat atg      48
Met Ser Ile Ala Ile Ala Arg Glu Gln Tyr Ala Ala Ile Leu Asp Met
  1               5                  10                  15

cat cct aaa cct tcg atc gcc atg ttt tct tcg gag cag gcg aga act 96
His Pro Lys Pro Ser Ile Ala Met Phe Ser Ser Glu Gln Ala Arg Thr
            20                  25                  30

tct tgg gag aaa cga cag gct cat cct tac ctt tat cgt ctt ctt gag 144
Ser Trp Glu Lys Arg Gln Ala His Pro Tyr Leu Tyr Arg Leu Leu Glu
        35                  40                  45

atc ata tgg ggt gtt gtg aaa ttt ctt ctc ggc tta atc ttc ttt att 192
Ile Ile Trp Gly Val Val Lys Phe Leu Leu Gly Leu Ile Phe Phe Ile
    50                  55                  60

ccc ttg ggt ctt ttc tgg gtc ctt cag aag ata tgt cag aat ttt att 240
Pro Leu Gly Leu Phe Trp Val Leu Gln Lys Ile Cys Gln Asn Phe Ile
65                  70                  75                  80

ctt ctt ggt gca gga ggg tgg att ttt aga ccc ata tgc agg gac tct 288
Leu Leu Gly Ala Gly Gly Trp Ile Phe Arg Pro Ile Cys Arg Asp Ser
                85                  90                  95

aat tta ttg cga caa gct tac gcc gcg cgt ctt ttc tcc gct tca ttc 336
Asn Leu Leu Arg Gln Ala Tyr Ala Ala Arg Leu Phe Ser Ala Ser Phe
            100                 105                 110

caa gat cat gtc tcc tct gtg cga agg gtt tgc tta cag tat gac gag 384
Gln Asp His Val Ser Ser Val Arg Arg Val Cys Leu Gln Tyr Asp Glu
        115                 120                 125

gtc ttt att gac gga ttg gag tta cgt ctt ccc aat gct aag cca gat 432
Val Phe Ile Asp Gly Leu Glu Leu Arg Leu Pro Asn Ala Lys Pro Asp
    130                 135                 140

cga tgg atg tta atc tcc aat gga aac tcc gat tgc tta gag tat agg 480
Arg Trp Met Leu Ile Ser Asn Gly Asn Ser Asp Cys Leu Glu Tyr Arg
145                 150                 155                 160

aca gtg ctg caa ggg gaa aag gac tgg ata ttc cgt att gct gaa gag 528
Thr Val Leu Gln Gly Glu Lys Asp Trp Ile Phe Arg Ile Ala Glu Glu
                165                 170                 175

tct caa tcc aac att tta atc ttc aat tac cca gga gtc atg aag agc 576
Ser Gln Ser Asn Ile Leu Ile Phe Asn Tyr Pro Gly Val Met Lys Ser
            180                 185                 190

caa ggg aat ata aca aga aac aat gta gtc aaa tct tat caa gca tgc 624
Gln Gly Asn Ile Thr Arg Asn Asn Val Val Lys Ser Tyr Gln Ala Cys
        195                 200                 205

gta cgc tat ctt aga gat gaa ccc gca gga cct cag gcg cgt caa atc     672
Val Arg Tyr Leu Arg Asp Glu Pro Ala Gly Pro Gln Ala Arg Gln Ile
    210                 215                 220

gtt gct tat ggc tat tct tta gga gct agt gtt caa gcc gaa gca tta     720
Val Ala Tyr Gly Tyr Ser Leu Gly Ala Ser Val Gln Ala Glu Ala Leu
225                 230                 235                 240

agt aaa gag atc gca gac gga agt gat agc gtc cgt tgg ttt gtc gtt     768
Ser Lys Glu Ile Ala Asp Gly Ser Asp Ser Val Arg Trp Phe Val Val
                245                 250                 255

aaa gat cga gga gct cgc tct aca gga gcc gtt gct aaa cag ttt att     816
Lys Asp Arg Gly Ala Arg Ser Thr Gly Ala Val Ala Lys Gln Phe Ile
            260                 265                 270

gga agt cta gga gtt tgg ctg gcg aat ctt acc cat tgg aat att aat     864
Gly Ser Leu Gly Val Trp Leu Ala Asn Leu Thr His Trp Asn Ile Asn
        275                 280                 285

tct gaa aag aga agc aag gac ttg cat tgc cca gaa ctc ttt att tat     912
Ser Glu Lys Arg Ser Lys Asp Leu His Cys Pro Glu Leu Phe Ile Tyr
    290                 295                 300

ggc aag gat tcc caa ggt aat ctt atc ggg gat gga ttg ttc aaa aaa     960
Gly Lys Asp Ser Gln Gly Asn Leu Ile Gly Asp Gly Leu Phe Lys Lys
305                 310                 315                 320

gag acg tgc ttc gca gca cca ttt tta gat cct aaa aac ttg gaa gag    1008
Glu Thr Cys Phe Ala Ala Pro Phe Leu Asp Pro Lys Asn Leu Glu Glu
                325                 330                 335

tgt tca ggg aag aaa atc cct gta gct cag acc ggt cta aga cac gat    1056
Cys Ser Gly Lys Lys Ile Pro Val Ala Gln Thr Gly Leu Arg His Asp
            340                 345                 350

cat atc ctt tcc gat gat gtg att aaa gaa gtt gca ggt cat att caa    1104
His Ile Leu Ser Asp Asp Val Ile Lys Glu Val Ala Gly His Ile Gln
        355                 360                 365

aga cat ttc gat aat tag                                            1122
Arg His Phe Asp Asn
    370

<210> 24
<211> 373

<212> PRT
<213> Chlamydia pneumoniae

<400> 24
Met Ser Ile Ala Ile Ala Arg Glu Gln Tyr Ala Ala Ile Leu Asp Met
1               5                   10                  15
His Pro Lys Pro Ser Ile Ala Met Phe Ser Ser Glu Gln Ala Arg Thr
            20                  25                  30
Ser Trp Glu Lys Arg Gln Ala His Pro Tyr Leu Tyr Arg Leu Leu Glu
        35                  40                  45
Ile Ile Trp Gly Val Val Lys Phe Leu Leu Gly Leu Ile Phe Phe Ile
    50                  55                  60
Pro Leu Gly Leu Phe Trp Val Leu Gln Lys Ile Cys Gln Asn Phe Ile
65                  70                  75                  80
Leu Leu Gly Ala Gly Gly Trp Ile Phe Arg Pro Ile Cys Arg Asp Ser
                85                  90                  95
Asn Leu Leu Arg Gln Ala Tyr Ala Ala Arg Leu Phe Ser Ala Ser Phe
            100                 105                 110
Gln Asp His Val Ser Ser Val Arg Arg Val Cys Leu Gln Tyr Asp Glu
        115                 120                 125
Val Phe Ile Asp Gly Leu Glu Leu Arg Leu Pro Asn Ala Lys Pro Asp
    130                 135                 140
Arg Trp Met Leu Ile Ser Asn Gly Asn Ser Asp Cys Leu Glu Tyr Arg
145                 150                 155                 160
Thr Val Leu Gln Gly Glu Lys Asp Trp Ile Phe Arg Ile Ala Glu Glu
                165                 170                 175
Ser Gln Ser Asn Ile Leu Ile Phe Asn Tyr Pro Gly Val Met Lys Ser
            180                 185                 190
Gln Gly Asn Ile Thr Arg Asn Asn Val Val Lys Ser Tyr Gln Ala Cys
        195                 200                 205
Val Arg Tyr Leu Arg Asp Glu Pro Ala Gly Pro Gln Ala Arg Gln Ile
    210                 215                 220
Val Ala Tyr Gly Tyr Ser Leu Gly Ala Ser Val Gln Ala Glu Ala Leu
225                 230                 235                 240
Ser Lys Glu Ile Ala Asp Gly Ser Asp Ser Val Arg Trp Phe Val Val
                245                 250                 255
Lys Asp Arg Gly Ala Arg Ser Thr Gly Ala Val Ala Lys Gln Phe Ile
            260                 265                 270
Gly Ser Leu Gly Val Trp Leu Ala Asn Leu Thr His Trp Asn Ile Asn
        275                 280                 285
Ser Glu Lys Arg Ser Lys Asp Leu His Cys Pro Glu Leu Phe Ile Tyr
    290                 295                 300
Gly Lys Asp Ser Gln Gly Asn Leu Ile Gly Asp Gly Leu Phe Lys Lys
305                 310                 315                 320
Glu Thr Cys Phe Ala Ala Pro Phe Leu Asp Pro Lys Asn Leu Glu Glu
                325                 330                 335
Cys Ser Gly Lys Lys Ile Pro Val Ala Gln Thr Gly Leu Arg His Asp
            340                 345                 350

His Ile Leu Ser Asp Asp Val Ile Lys Glu Val Ala Gly His Ile Gln
        355                 360                 365
Arg His Phe Asp Asn
    370

<210> 25
<211> 1215
<212> DNA
<213> Chlamydia pneumoniae

<220>
<221> CDS
<222> (1)..(1215)

<400> 25
atg aca gat tct aat ccc cta ccc tct tat aca gac gcc agt ctc tac    48
Met Thr Asp Ser Asn Pro Leu Pro Ser Tyr Thr Asp Ala Ser Leu Tyr
  1               5                  10                  15

aga act cct gcg aaa cat tcc tat ccg att aga ctc cct ctc aac cgt    96
Arg Thr Pro Ala Lys His Ser Tyr Pro Ile Arg Leu Pro Leu Asn Arg
             20                  25                  30

aca gat aga atc gag aaa ata ctg aaa att gtc acc ctc aca cta gcc    144
Thr Asp Arg Ile Glu Lys Ile Leu Lys Ile Val Thr Leu Thr Leu Ala
         35                  40                  45

cta gcg tgc gct ttg ggc ttt agc att gct gct ggc att ttg gct atg    192
Leu Ala Cys Ala Leu Gly Phe Ser Ile Ala Ala Gly Ile Leu Ala Met
     50                  55                  60

cct att ttt tct gcc gta gtt gtc ata aca tta gca att gct gcg gtc    240
Pro Ile Phe Ser Ala Val Val Val Ile Thr Leu Ala Ile Ala Ala Val
 65                  70                  75                  80

tca ctt tac tcc ctt tta aag aaa cct aaa tta tac gag att ctt cct    288
Ser Leu Tyr Ser Leu Leu Lys Lys Pro Lys Leu Tyr Glu Ile Leu Pro
                 85                  90                  95

caa atc gaa ccc gaa tct gag caa agt tct ctg tct ccc tct ccc cag    336
Gln Ile Glu Pro Glu Ser Glu Gln Ser Ser Leu Ser Pro Ser Pro Gln
            100                 105                 110

cct cct gag caa cag gac ctc cct ttg cag atc gat cca ctt ccc gat    384
Pro Pro Glu Gln Gln Asp Leu Pro Leu Gln Ile Asp Pro Leu Pro Asp
        115                 120                 125

ccc gaa tca ctc ccc gaa gtc tct ctt gct gat cta acc aca ccc cca 432
Pro Glu Ser Leu Pro Glu Val Ser Leu Ala Asp Leu Thr Thr Pro Pro
    130                 135                 140

gaa gaa ctt acc gct atc acg gtc act cct ggc tat gag gct ctt ctt 480
Glu Glu Leu Thr Ala Ile Thr Val Thr Pro Gly Tyr Glu Ala Leu Leu
145                 150                 155                 160

gaa caa aac tgg gat ctt ctt ccg agc tta gcc gct gta gac cca tcg 528
Glu Gln Asn Trp Asp Leu Leu Pro Ser Leu Ala Ala Val Asp Pro Ser
                165                 170                 175

ttt act aca gaa aca cct cag cag ccc tgt ttt att tgg aag ctt aaa 576
Phe Thr Thr Glu Thr Pro Gln Gln Pro Cys Phe Ile Trp Lys Leu Lys
            180                 185                 190

gac tcg aag ctt atc ttt ata tct acc tca gga gat att gca gtt cca 624
Asp Ser Lys Leu Ile Phe Ile Ser Thr Ser Gly Asp Ile Ala Val Pro
        195                 200                 205

aga atc aaa act caa ggc agg gtg atg att gtt aac gca gca aac gag 672
Arg Ile Lys Thr Gln Gly Arg Val Met Ile Val Asn Ala Ala Asn Glu
    210                 215                 220

aac atc tcc cga gaa gga ggg gga acg aat aaa gct cta tcc ctg gct 720
Asn Ile Ser Arg Glu Gly Gly Gly Thr Asn Lys Ala Leu Ser Leu Ala
225                 230                 235                 240

aca agt cta cag tgt tgg aac gca tct agg ctc cct aga gcg cac tct 768
Thr Ser Leu Gln Cys Trp Asn Ala Ser Arg Leu Pro Arg Ala His Ser
                245                 250                 255

cgt tct gga tcc caa cta cag cca gga gaa tgc cgc tca gca aaa tgg 816
Arg Ser Gly Ser Gln Leu Gln Pro Gly Glu Cys Arg Ser Ala Lys Trp
            260                 265                 270

gaa aat agt gat cac acc tca aac gac cat gtc cca ggc aaa gca cac 864
Glu Asn Ser Asp His Thr Ser Asn Asp His Val Pro Gly Lys Ala His
        275                 280                 285

ttc tta gca caa ctg ctt ggt ccc gaa gct gct aag tgt aac aac gat 912
Phe Leu Ala Gln Leu Leu Gly Pro Glu Ala Ala Lys Cys Asn Asn Asp
    290                 295                 300

cct aag caa gca ttt gaa gta agc aag aaa gcg ttt cat aac ctg ttc 960
Pro Lys Gln Ala Phe Glu Val Ser Lys Lys Ala Phe His Asn Leu Phe
305                 310                 315                 320

caa gaa gct gaa atc ata ggc gtt gat gtg att caa ctc ccc ctc att 1008
Gln Glu Ala Glu Ile Ile Gly Val Asp Val Ile Gln Leu Pro Leu Ile
                325                 330                 335

gga tgt aat cta ttt gct cca tca aga ctt cta aac ctc ggg aaa aca 1056
Gly Cys Asn Leu Phe Ala Pro Ser Arg Leu Leu Asn Leu Gly Lys Thr
            340                 345                 350

aga gca gaa tgg atc gag gct ata aaa ttg gca ctc atc aca tct ctt 1104
Arg Ala Glu Trp Ile Glu Ala Ile Lys Leu Ala Leu Ile Thr Ser Leu
        355                 360                 365

caa gat ttt gga tgg gaa caa gac aac cag gaa gag caa aaa att atc 1152
Gln Asp Phe Gly Trp Glu Gln Asp Asn Gln Glu Glu Gln Lys Ile Ile
    370                 375                 380

atc ctt aca gac aag gac cag cct ccc atc att cca ccc cgt ttc gat 1200
Ile Leu Thr Asp Lys Asp Gln Pro Pro Ile Ile Pro Pro Arg Phe Asp
385                 390                 395                 400

cta acg act ccc tag 1215
Leu Thr Thr Pro
405

<210> 26
<211> 404
<212> PRT
<213> Chlamydia pneumoniae

<400> 26
Met Thr Asp Ser Asn Pro Leu Pro Ser Tyr Thr Asp Ala Ser Leu Tyr
1               5                   10                  15
Arg Thr Pro Ala Lys His Ser Tyr Pro Ile Arg Leu Pro Leu Asn Arg
            20                  25                  30
Thr Asp Arg Ile Glu Lys Ile Leu Lys Ile Val Thr Leu Thr Leu Ala
        35                  40                  45
Leu Ala Cys Ala Leu Gly Phe Ser Ile Ala Ala Gly Ile Leu Ala Met
    50                  55                  60
Pro Ile Phe Ser Ala Val Val Val Ile Thr Leu Ala Ile Ala Ala Val
65                  70                  75                  80
Ser Leu Tyr Ser Leu Leu Lys Lys Pro Lys Leu Tyr Glu Ile Leu Pro
                85                  90                  95
Gln Ile Glu Pro Glu Ser Glu Gln Ser Ser Leu Ser Pro Ser Pro Gln
            100                 105                 110
Pro Pro Glu Gln Gln Asp Leu Pro Leu Gln Ile Asp Pro Leu Pro Asp
        115                 120                 125

Pro Glu Ser Leu Pro Glu Val Ser Leu Ala Asp Leu Thr Thr Pro Pro
    130                 135                 140
Glu Glu Leu Thr Ala Ile Thr Val Thr Pro Gly Tyr Glu Ala Leu Leu
145                 150                 155                 160
Glu Gln Asn Trp Asp Leu Leu Pro Ser Leu Ala Ala Val Asp Pro Ser
                165                 170                 175
Phe Thr Thr Glu Thr Pro Gln Gln Pro Cys Phe Ile Trp Lys Leu Lys
            180                 185                 190
Asp Ser Lys Leu Ile Phe Ile Ser Thr Ser Gly Asp Ile Ala Val Pro
        195                 200                 205
Arg Ile Lys Thr Gln Gly Arg Val Met Ile Val Asn Ala Ala Asn Glu
    210                 215                 220
Asn Ile Ser Arg Glu Gly Gly Gly Thr Asn Lys Ala Leu Ser Leu Ala
225                 230                 235                 240
Thr Ser Leu Gln Cys Trp Asn Ala Ser Arg Leu Pro Arg Ala His Ser
                245                 250                 255
Arg Ser Gly Ser Gln Leu Gln Pro Gly Glu Cys Arg Ser Ala Lys Trp
            260                 265                 270
Glu Asn Ser Asp His Thr Ser Asn Asp His Val Pro Gly Lys Ala His
        275                 280                 285
Phe Leu Ala Gln Leu Leu Gly Pro Glu Ala Ala Lys Cys Asn Asn Asp
    290                 295                 300
Pro Lys Gln Ala Phe Glu Val Ser Lys Lys Ala Phe His Asn Leu Phe
305                 310                 315                 320
Gln Glu Ala Glu Ile Ile Gly Val Asp Val Ile Gln Leu Pro Leu Ile
                325                 330                 335
Gly Cys Asn Leu Phe Ala Pro Ser Arg Leu Leu Asn Leu Gly Lys Thr
            340                 345                 350
Arg Ala Glu Trp Ile Glu Ala Ile Lys Leu Ala Leu Ile Thr Ser Leu
        355                 360                 365
Gln Asp Phe Gly Trp Glu Gln Asp Asn Gln Glu Glu Gln Lys Ile Ile
    370                 375                 380
Ile Leu Thr Asp Lys Asp Gln Pro Pro Ile Ile Pro Pro Arg Phe Asp
385                 390                 395                 400
Leu Thr Thr Pro

<210> 27
<211> 963
<212> DNA
<213> Chlamydia pneumoniae

<220>
<221> CDS
<222> (1)..(963)

<400> 27

gtg cgg ctc tta tct ata ctt aag ctg cat ctc ttc tcg cta cga tct 48
Val Arg Leu Leu Ser Ile Leu Lys Leu His Leu Phe Ser Leu Arg Ser
1               5                   10                  15

tcc agt tca tta tcc cct cac tat tat cat tca tgc tcc cgt tcc atg 96
Ser Ser Ser Leu Ser Pro His Tyr Tyr His Ser Cys Ser Arg Ser Met
            20                  25                  30

ctt cat ttg ctt tgt cgc tgg aaa gat gct gat att atg gaa tgg cag 144
Leu His Leu Leu Cys Arg Trp Lys Asp Ala Asp Ile Met Glu Trp Gln
        35                  40                  45

cag att tgc aat att ctt tca gga gtc tgt agc aga atg agt gga aag 192
Gln Ile Cys Asn Ile Leu Ser Gly Val Cys Ser Arg Met Ser Gly Lys
    50                  55                  60

ttg gtt tct ttg cag aag gaa acc caa gac tct tgt cat cag gag cat 240
Leu Val Ser Leu Gln Lys Glu Thr Gln Asp Ser Cys His Gln Glu His
65                  70                  75                  80

gaa cgt att cac tta cag tat cgg gaa cag cta tct gct ttg gaa gaa 288
Glu Arg Ile His Leu Gln Tyr Arg Glu Gln Leu Ser Ala Leu Glu Glu
                85                  90                  95

gaa tat cgc cgt cgt gaa gaa gct aaa aat caa gat tta gaa aag tta 336
Glu Tyr Arg Arg Arg Glu Glu Ala Lys Asn Gln Asp Leu Glu Lys Leu
            100                 105                 110

caa caa gaa aat acc tgg ttg caa aat cgc ctt gct gag aaa tta caa 384
Gln Gln Glu Asn Thr Trp Leu Gln Asn Arg Leu Ala Glu Lys Leu Gln
        115                 120                 125

caa att cga cac caa agt gat att atc gac gag ata aaa aaa gaa ctg 432
Gln Ile Arg His Gln Ser Asp Ile Ile Asp Glu Ile Lys Lys Glu Leu
    130                 135                 140

ctt caa agt gta cag cga aca gaa att agt gaa ggg cgt cgt tta tgc 480
Leu Gln Ser Val Gln Arg Thr Glu Ile Ser Glu Gly Arg Arg Leu Cys
145                 150                 155                 160

tat gag cat aaa att aaa cag ctg gaa gaa caa cta cag cgt tat gtt 528
Tyr Glu His Lys Ile Lys Gln Leu Glu Glu Gln Leu Gln Arg Tyr Val
                165                 170                 175

tcg caa cac gga gcc ccc tct ata gag ata gaa gaa gac aaa tct tca 576
Ser Gln His Gly Ala Pro Ser Ile Glu Ile Glu Glu Asp Lys Ser Ser
            180                 185                 190

gcc gca tat gca gag atc aac cgt ttg aag aaa agt ctt ata gat ttg 624
Ala Ala Tyr Ala Glu Ile Asn Arg Leu Lys Lys Ser Leu Ile Asp Leu
        195                 200                 205

caa caa gaa aaa gat att tat ata aaa aca tat cat tca gag att gct 672
Gln Gln Glu Lys Asp Ile Tyr Ile Lys Thr Tyr His Ser Glu Ile Ala
    210                 215                 220

aag tta aga gaa aaa tta cag cga caa gaa gga gct caa act agc tct 720
Lys Leu Arg Glu Lys Leu Gln Arg Gln Glu Gly Ala Gln Thr Ser Ser
225                 230                 235                 240

gag gtt tgt tct att gag aag tta aca gag gtg caa acc gat cta gct 768
Glu Val Cys Ser Ile Glu Lys Leu Thr Glu Val Gln Thr Asp Leu Ala
                245                 250                 255

gaa aaa aag aaa gcc ata gcc tta ctc caa gat att gtt gag gat caa 816
Glu Lys Lys Lys Ala Ile Ala Leu Leu Gln Asp Ile Val Glu Asp Gln
            260                 265                 270

tat tgt cag ctt cga gat cta cat aaa gag aaa ggt atg gct atg cct 864
Tyr Cys Gln Leu Arg Asp Leu His Lys Glu Lys Gly Met Ala Met Pro
        275                 280                 285

tca aat aca aag tta gat cat ttg aaa gga ctt tta ggc aaa gaa cct 912
Ser Asn Thr Lys Leu Asp His Leu Lys Gly Leu Leu Gly Lys Glu Pro
    290                 295                 300

gag agt gaa gta gac gta gta ttc tca gaa tct aaa tct tta ggg agt 960
Glu Ser Glu Val Asp Val Val Phe Ser Glu Ser Lys Ser Leu Gly Ser
305                 310                 315                 320

taa 963

<210> 28
<211> 320
<212> PRT
<213> Chlamydia pneumoniae

<400> 28
Val Arg Leu Leu Ser Ile Leu Lys Leu His Leu Phe Ser Leu Arg Ser
1               5                   10                  15
Ser Ser Ser Leu Ser Pro His Tyr Tyr His Ser Cys Ser Arg Ser Met
            20                  25                  30
Leu His Leu Leu Cys Arg Trp Lys Asp Ala Asp Ile Met Glu Trp Gln
        35                  40                  45

Gln Ile Cys Asn Ile Leu Ser Gly Val Cys Ser Arg Met Ser Gly Lys
    50                  55                  60
Leu Val Ser Leu Gln Lys Glu Thr Gln Asp Ser Cys His Gln Glu His
65                  70                  75                  80
Glu Arg Ile His Leu Gln Tyr Arg Glu Gln Leu Ser Ala Leu Glu Glu
                85                  90                  95
Glu Tyr Arg Arg Arg Glu Glu Ala Lys Asn Gln Asp Leu Glu Lys Leu
            100                 105                 110
Gln Gln Glu Asn Thr Trp Leu Gln Asn Arg Leu Ala Glu Lys Leu Gln
        115                 120                 125
Gln Ile Arg His Gln Ser Asp Ile Ile Asp Glu Ile Lys Lys Glu Leu
    130                 135                 140
Leu Gln Ser Val Gln Arg Thr Glu Ile Ser Glu Gly Arg Arg Leu Cys
145                 150                 155                 160
Tyr Glu His Lys Ile Lys Gln Leu Glu Glu Gln Leu Gln Arg Tyr Val
                165                 170                 175
Ser Gln His Gly Ala Pro Ser Ile Glu Ile Glu Glu Asp Lys Ser Ser
            180                 185                 190
Ala Ala Tyr Ala Glu Ile Asn Arg Leu Lys Lys Ser Leu Ile Asp Leu
        195                 200                 205
Gln Gln Glu Lys Asp Ile Tyr Ile Lys Thr Tyr His Ser Glu Ile Ala
    210                 215                 220
Lys Leu Arg Glu Lys Leu Gln Arg Gln Glu Gly Ala Gln Thr Ser Ser
225                 230                 235                 240
Glu Val Cys Ser Ile Glu Lys Leu Thr Glu Val Gln Thr Asp Leu Ala
                245                 250                 255
Glu Lys Lys Lys Ala Ile Ala Leu Leu Gln Asp Ile Val Glu Asp Gln
            260                 265                 270
Tyr Cys Gln Leu Arg Asp Leu His Lys Glu Lys Gly Met Ala Met Pro
        275                 280                 285
Ser Asn Thr Lys Leu Asp His Leu Lys Gly Leu Leu Gly Lys Glu Pro
    290                 295                 300
Glu Ser Glu Val Asp Val Val Phe Ser Glu Ser Lys Ser Leu Gly Ser
305                 310                 315                 320

<210> 29
<211> 3258
<212> DNA
<213> Chlamydia pneumoniae

<220>
<221> CDS
<222> (1)..(3258)

<400> 29
atg gca atg gat ttc aac cca gtt aat tta gat ttt tct att tcc aaa    48

Met Ala Met Asp Phe Asn Pro Val Asn Leu Asp Phe Ser Ile Ser Lys
1               5                   10                  15

gaa ttc aaa gag gaa aca ctt cct tta cta tta gaa aat att cat cca     96
Glu Phe Lys Glu Glu Thr Leu Pro Leu Leu Leu Glu Asn Ile His Pro
            20                  25                  30

gga gcc acg gca ttc ctt gca gca aag atg ttt cat gac tgt cgt gct     144
Gly Ala Thr Ala Phe Leu Ala Ala Lys Met Phe His Asp Cys Arg Ala
        35                  40                  45

tct gta att atg att acg aca ccc gca cgt ctt gat gat ctc ttt gaa     192
Ser Val Ile Met Ile Thr Thr Pro Ala Arg Leu Asp Asp Leu Phe Glu
    50                  55                  60

aat tta agg acc ttt tta gac caa gct cct gta gaa ttt ccc tct tct     240
Asn Leu Arg Thr Phe Leu Asp Gln Ala Pro Val Glu Phe Pro Ser Ser
65                  70                  75                  80

gaa att gat ctc tct cca aaa tta gtg aac ata gat gct gtg ggg aag     288
Glu Ile Asp Leu Ser Pro Lys Leu Val Asn Ile Asp Ala Val Gly Lys
                85                  90                  95

cga gat cat ctt ctt tac agc ttg aat cag cac agg gct cct ata ttc     336
Arg Asp His Leu Leu Tyr Ser Leu Asn Gln His Arg Ala Pro Ile Phe
            100                 105                 110

tgt gtc act aca tta aaa gct ctt tta gaa aaa acc cgt tct cca caa     384
Cys Val Thr Thr Leu Lys Ala Leu Leu Glu Lys Thr Arg Ser Pro Gln
        115                 120                 125

gct aca agt caa caa cat ctt gat ctc gca gtt gga gat gtc tta gat     432
Ala Thr Ser Gln Gln His Leu Asp Leu Ala Val Gly Asp Val Leu Asp
    130                 135                 140

cca gaa gca act acg gaa ctc tgt aaa agt tta gga tat tct cag gtc     480
Pro Glu Ala Thr Thr Glu Leu Cys Lys Ser Leu Gly Tyr Ser Gln Val
145                 150                 155                 160

atg cta act agc gaa aag gga gaa ttt tct tgt cgc gga gga att gtt     528
Met Leu Thr Ser Glu Lys Gly Glu Phe Ser Cys Arg Gly Gly Ile Val
                165                 170                 175

gat att ttc ccg tta tcc tcg cca gag cct ttt agg ata gaa ttt tgg     576
Asp Ile Phe Pro Leu Ser Ser Pro Glu Pro Phe Arg Ile Glu Phe Trp
            180                 185                 190

gga gag aag atc att tct atc aga tct tac aat cct tcg gat cag cta     624

Gly Glu Lys Ile Ile Ser Ile Arg Ser Tyr Asn Pro Ser Asp Gln Leu
        195                 200                 205

tcg aca gga aaa gtc tct aaa att tct atc tct cca gcg tac aca gaa 672
Ser Thr Gly Lys Val Ser Lys Ile Ser Ile Ser Pro Ala Tyr Thr Glu
    210                 215                 220

gag gcc tct gga gga aac tat tct cat tca cta tta gac tat ttc agc 720
Glu Ala Ser Gly Gly Asn Tyr Ser His Ser Leu Leu Asp Tyr Phe Ser
225                 230                 235                 240

acc cct cct ctc tat ctc ttt gat aac tta gaa att cta gaa gat gac 768
Thr Pro Pro Leu Tyr Leu Phe Asp Asn Leu Glu Ile Leu Glu Asp Asp
                245                 250                 255

ttt gcg gat att tct gga aca ctt tcg tcc ctt cca gat aga ttt ttc 816
Phe Ala Asp Ile Ser Gly Thr Leu Ser Ser Leu Pro Asp Arg Phe Phe
            260                 265                 270

tct att ggc act ctc tac gat cgc att tca acg tct aat caa gtt tat 864
Ser Ile Gly Thr Leu Tyr Asp Arg Ile Ser Thr Ser Asn Gln Val Tyr
        275                 280                 285

ttc tcg gag act ccg ttt cct aac gtc aag aat ctc aag gaa aac cgc 912
Phe Ser Glu Thr Pro Phe Pro Asn Val Lys Asn Leu Lys Glu Asn Arg
    290                 295                 300

gtc atc atc gaa gca ttt cac cga aac atg gaa gcg agt cgc caa gcg 960
Val Ile Ile Glu Ala Phe His Arg Asn Met Glu Ala Ser Arg Gln Ala
305                 310                 315                 320

att cct ata ctg tat cct gag caa att att cag aac gat gag aat ccc 1008
Ile Pro Ile Leu Tyr Pro Glu Gln Ile Ile Gln Asn Asp Glu Asn Pro
                325                 330                 335

cta ctt gct ttc cta cag cat ctt caa gaa tat atg cct cct cat gga 1056
Leu Leu Ala Phe Leu Gln His Leu Gln Glu Tyr Met Pro Pro His Gly
            340                 345                 350

aag ccc tta aaa tta gcc atc tac agc aca aaa acg aaa tct tta aaa 1104
Lys Pro Leu Lys Leu Ala Ile Tyr Ser Thr Lys Thr Lys Ser Leu Lys
        355                 360                 365

gag gcc cgt gct cta gca gag act gta gct cgt ggg gat gtg gaa atc 1152
Glu Ala Arg Ala Leu Ala Glu Thr Val Ala Arg Gly Asp Val Glu Ile
    370                 375                 380

tat gaa aaa aca ggg aat cta act tcc agc ttt gca tta gta aac gaa 1200

Tyr Glu Lys Thr Gly Asn Leu Thr Ser Ser Phe Ala Leu Val Asn Glu
385                 390                 395                 400

gcc ttt gca gcg att tcc cta tcc gag ttt gct tct aca aaa gta ttg     1248
Ala Phe Ala Ala Ile Ser Leu Ser Glu Phe Ala Ser Thr Lys Val Leu
                405                 410                 415

cgt agg caa aaa caa cgc act cac ttt tca gtg act act gaa gaa gtt     1296
Arg Arg Gln Lys Gln Arg Thr His Phe Ser Val Thr Thr Glu Glu Val
            420                 425                 430

ttt gtt ccg att cca gga gag act gtt gtc cat att cat aat gga att     1344
Phe Val Pro Ile Pro Gly Glu Thr Val Val His Ile His Asn Gly Ile
        435                 440                 445

gga aaa ttc cta gga ata gaa aaa aaa ccg aac cat ctg aat att gaa     1392
Gly Lys Phe Leu Gly Ile Glu Lys Lys Pro Asn His Leu Asn Ile Glu
    450                 455                 460

acg gat tat ctt gtt tta gaa tat gca gat aaa gct cgg ctt tat gtt     1440
Thr Asp Tyr Leu Val Leu Glu Tyr Ala Asp Lys Ala Arg Leu Tyr Val
465                 470                 475                 480

cct tcg aac caa gcc tat ctg atc tct cgg tat gtt ggg act tct gat     1488
Pro Ser Asn Gln Ala Tyr Leu Ile Ser Arg Tyr Val Gly Thr Ser Asp
                485                 490                 495

aaa gct gcc gat ctc cat cat tta aat agt tcg aaa tgg aag cgc tct     1536
Lys Ala Ala Asp Leu His His Leu Asn Ser Ser Lys Trp Lys Arg Ser
            500                 505                 510

aga gat ctt act gaa aaa tct ttg att gtc tac gca gag aag ctc tta     1584
Arg Asp Leu Thr Glu Lys Ser Leu Ile Val Tyr Ala Glu Lys Leu Leu
        515                 520                 525

cag tta gaa gca caa cgt tcg aca act cct gct ttt gtg tac cct cct     1632
Gln Leu Glu Ala Gln Arg Ser Thr Thr Pro Ala Phe Val Tyr Pro Pro
    530                 535                 540

cac gga gag tcc gta atc aag ttt gcg gaa acg ttt ccc tat gaa gaa     1680
His Gly Glu Ser Val Ile Lys Phe Ala Glu Thr Phe Pro Tyr Glu Glu
545                 550                 555                 560

acc ccc gat cag tta aag act att gat cag att tac aat gac atg atg     1728
Thr Pro Asp Gln Leu Lys Thr Ile Asp Gln Ile Tyr Asn Asp Met Met
                565                 570                 575

tct cca aaa ctc atg gat aga tta att tgc gga gat gct ggc ttt ggg     1776

Ser Pro Lys Leu Met Asp Arg Leu Ile Cys Gly Asp Ala Gly Phe Gly
            580                 585                 590

aaa act gaa gtc atc atg cgg gct gct gtc aag gct gtt tgc gat ggc 1824
Lys Thr Glu Val Ile Met Arg Ala Ala Val Lys Ala Val Cys Asp Gly
        595                 600                 605

cat cga caa gtc att gtt atg gtt ccc aca acg att tta gca act cag 1872
His Arg Gln Val Ile Val Met Val Pro Thr Thr Ile Leu Ala Thr Gln
    610                 615                 620

cac tat gaa act ttt aaa gaa aga atg gcg gga ttg ccg atc gaa att 1920
His Tyr Glu Thr Phe Lys Glu Arg Met Ala Gly Leu Pro Ile Glu Ile
625                 630                 635                 640

gct gtg ctt tca cgt ttc tcc caa gcc aaa gtg caa aaa ctc atc tgt 1968
Ala Val Leu Ser Arg Phe Ser Gln Ala Lys Val Gln Lys Leu Ile Cys
                645                 650                 655

gag caa gta gct tca gga caa att gac att atc att gga act cac aaa 2016
Glu Gln Val Ala Ser Gly Gln Ile Asp Ile Ile Ile Gly Thr His Lys
            660                 665                 670

ctc att aac aaa agc cta gag ttt aag aac cct ggt tta tta att att 2064
Leu Ile Asn Lys Ser Leu Glu Phe Lys Asn Pro Gly Leu Leu Ile Ile
        675                 680                 685

gat gaa gaa caa cgc ttt gga gtt aaa gtt aag gac aat ctg aag gag 2112
Asp Glu Glu Gln Arg Phe Gly Val Lys Val Lys Asp Asn Leu Lys Glu
    690                 695                 700

cgc tat ccc atg att gac tgt ctt aca gta tct gcg act ccc atc cca 2160
Arg Tyr Pro Met Ile Asp Cys Leu Thr Val Ser Ala Thr Pro Ile Pro
705                 710                 715                 720

agg aca ttg cac atg tct cta tca gga gct cgt gat cta tct gtg att 2208
Arg Thr Leu His Met Ser Leu Ser Gly Ala Arg Asp Leu Ser Val Ile
                725                 730                 735

gcc atg cct ccc ttg gat agg ttg cct gta agt act ttt gtc atg gag 2256
Ala Met Pro Pro Leu Asp Arg Leu Pro Val Ser Thr Phe Val Met Glu
            740                 745                 750

cat aat aca gaa aca ttg aca gcg gct tta agg cac gag ctc ctt cga 2304
His Asn Thr Glu Thr Leu Thr Ala Ala Leu Arg His Glu Leu Leu Arg
        755                 760                 765

gga gga caa gcc tat gtc att cat aat cgg att gag agc atc tat act 2352

Gly Gly Gln Ala Tyr Val Ile His Asn Arg Ile Glu Ser Ile Tyr Thr
    770                 775                 780

ctt gct gag acc att cgc aat ctg att cct gag gct cgt att ggc gta 2400
Leu Ala Glu Thr Ile Arg Asn Leu Ile Pro Glu Ala Arg Ile Gly Val
785                 790                 795                 800

gct cat ggt caa atg gga gct gag gac ctc tct aat atc ttt acg aaa 2448
Ala His Gly Gln Met Gly Ala Glu Asp Leu Ser Asn Ile Phe Thr Lys
                805                 810                 815

ttc aaa aat cag aaa acc gac atc ctc gtt gct act gca ctg ata gaa 2496
Phe Lys Asn Gln Lys Thr Asp Ile Leu Val Ala Thr Ala Leu Ile Glu
            820                 825                 830

aac ggg att gat att cca aac gcc aat acc att ttg ata gat cat gcc 2544
Asn Gly Ile Asp Ile Pro Asn Ala Asn Thr Ile Leu Ile Asp His Ala
        835                 840                 845

gat aag ttt gga atg gcg gat tta tat caa atg aag gga cgt gtc ggg 2592
Asp Lys Phe Gly Met Ala Asp Leu Tyr Gln Met Lys Gly Arg Val Gly
    850                 855                 860

cga tgg aat aaa aag gcc tat tgt tat ttt cta gtt cct cac tta gac 2640
Arg Trp Asn Lys Lys Ala Tyr Cys Tyr Phe Leu Val Pro His Leu Asp
865                 870                 875                 880

agg ttg tct ggg cca gca gcg aag cga ctc gct gct tta aat aag cag 2688
Arg Leu Ser Gly Pro Ala Ala Lys Arg Leu Ala Ala Leu Asn Lys Gln
                885                 890                 895

gaa tat gga ggg gga atg aag att gcc ctc cat gat tta gaa atc cgc 2736
Glu Tyr Gly Gly Gly Met Lys Ile Ala Leu His Asp Leu Glu Ile Arg
            900                 905                 910

ggt gca ggg aat att cta gga acc gat cag tcg gga cat atc gga act 2784
Gly Ala Gly Asn Ile Leu Gly Thr Asp Gln Ser Gly His Ile Gly Thr
        915                 920                 925

ata ggg ttt aat ttg tat tgc aaa tta cta aaa aaa gct gtt tca gct 2832
Ile Gly Phe Asn Leu Tyr Cys Lys Leu Leu Lys Lys Ala Val Ser Ala
    930                 935                 940

tta aaa aaa cac acg tcc ccc cta ctt ttc aac gac gat gtg aaa ata 2880
Leu Lys Lys His Thr Ser Pro Leu Leu Phe Asn Asp Asp Val Lys Ile
945                 950                 955                 960

gaa ttt cct tac aat tcg cgt att cct gat act tac atc gaa act gga 2928

Glu Phe Pro Tyr Asn Ser Arg Ile Pro Asp Thr Tyr Ile Glu Thr Gly
                965                 970                 975

tcg atg cgc att gag ttt tac caa aag att ggt aat gct gaa agc tct 2976
Ser Met Arg Ile Glu Phe Tyr Gln Lys Ile Gly Asn Ala Glu Ser Ser
            980                 985                 990

gaa gag ctt acc gca ata caa gaa gaa atg cga gat cgc ttt ggc cca 3024
Glu Glu Leu Thr Ala Ile Gln Glu Glu Met Arg Asp Arg Phe Gly Pro
        995                 1000                1005

tta cct caa gag atc tgc tgg ctt ttt gcc ctt gct gaa ata cgc cta 3072
Leu Pro Gln Glu Ile Cys Trp Leu Phe Ala Leu Ala Glu Ile Arg Leu
    1010                1015                1020

ttt gct ttg cag cat ggc att tct agc att aag gga act gcg aat gct 3120
Phe Ala Leu Gln His Gly Ile Ser Ser Ile Lys Gly Thr Ala Asn Ala
1025                1030                1035                1040

tta tat gtg caa aaa tgc ctt agc aaa tct gaa cag aca aag aaa acc 3168
Leu Tyr Val Gln Lys Cys Leu Ser Lys Ser Glu Gln Thr Lys Lys Thr
                1045                1050                1055

ttg ccc tat gct cta tct cca act cct gaa ctt cta gtc aaa gaa gtc 3216
Leu Pro Tyr Ala Leu Ser Pro Thr Pro Glu Leu Leu Val Lys Glu Val
            1060                1065                1070

att gag tcc ata gaa agg ggc ttc tta atc aac gct tca taa 3258
Ile Glu Ser Ile Glu Arg Gly Phe Leu Ile Asn Ala Ser
        1075                1080                1085

<210> 30
<211> 1085
<212> PRT
<213> Chlamydia pneumoniae

<400> 30
Met Ala Met Asp Phe Asn Pro Val Asn Leu Asp Phe Ser Ile Ser Lys
1               5                   10                  15
Glu Phe Lys Glu Glu Thr Leu Pro Leu Leu Leu Glu Asn Ile His Pro
            20                  25                  30
Gly Ala Thr Ala Phe Leu Ala Ala Lys Met Phe His Asp Cys Arg Ala
        35                  40                  45
Ser Val Ile Met Ile Thr Thr Pro Ala Arg Leu Asp Asp Leu Phe Glu
    50                  55                  60
Asn Leu Arg Thr Phe Leu Asp Gln Ala Pro Val Glu Phe Pro Ser Ser
65                  70                  75                  80

Glu Ile Asp Leu Ser Pro Lys Leu Val Asn Ile Asp Ala Val Gly Lys
                85                  90                  95
Arg Asp His Leu Leu Tyr Ser Leu Asn Gln His Arg Ala Pro Ile Phe
            100                 105                 110
Cys Val Thr Thr Leu Lys Ala Leu Leu Glu Lys Thr Arg Ser Pro Gln
        115                 120                 125
Ala Thr Ser Gln Gln His Leu Asp Leu Ala Val Gly Asp Val Leu Asp
    130                 135                 140
Pro Glu Ala Thr Thr Glu Leu Cys Lys Ser Leu Gly Tyr Ser Gln Val
145                 150                 155                 160
Met Leu Thr Ser Glu Lys Gly Glu Phe Ser Cys Arg Gly Gly Ile Val
                165                 170                 175
Asp Ile Phe Pro Leu Ser Ser Pro Glu Pro Phe Arg Ile Glu Phe Trp
            180                 185                 190
Gly Glu Lys Ile Ile Ser Ile Arg Ser Tyr Asn Pro Ser Asp Gln Leu
        195                 200                 205
Ser Thr Gly Lys Val Ser Lys Ile Ser Ile Ser Pro Ala Tyr Thr Glu
    210                 215                 220
Glu Ala Ser Gly Gly Asn Tyr Ser His Ser Leu Leu Asp Tyr Phe Ser
225                 230                 235                 240
Thr Pro Pro Leu Tyr Leu Phe Asp Asn Leu Glu Ile Leu Glu Asp Asp
                245                 250                 255
Phe Ala Asp Ile Ser Gly Thr Leu Ser Ser Leu Pro Asp Arg Phe Phe
            260                 265                 270
Ser Ile Gly Thr Leu Tyr Asp Arg Ile Ser Thr Ser Asn Gln Val Tyr
        275                 280                 285
Phe Ser Glu Thr Pro Phe Pro Asn Val Lys Asn Leu Lys Glu Asn Arg
    290                 295                 300
Val Ile Ile Glu Ala Phe His Arg Asn Met Glu Ala Ser Arg Gln Ala
305                 310                 315                 320
Ile Pro Ile Leu Tyr Pro Glu Gln Ile Ile Gln Asn Asp Glu Asn Pro
                325                 330                 335
Leu Leu Ala Phe Leu Gln His Leu Gln Glu Tyr Met Pro Pro His Gly
            340                 345                 350
Lys Pro Leu Lys Leu Ala Ile Tyr Ser Thr Lys Thr Lys Ser Leu Lys
        355                 360                 365
Glu Ala Arg Ala Leu Ala Glu Thr Val Ala Arg Gly Asp Val Glu Ile
    370                 375                 380
Tyr Glu Lys Thr Gly Asn Leu Thr Ser Ser Phe Ala Leu Val Asn Glu
385                 390                 395                 400
Ala Phe Ala Ala Ile Ser Leu Ser Glu Phe Ala Ser Thr Lys Val Leu
                405                 410                 415
Arg Arg Gln Lys Gln Arg Thr His Phe Ser Val Thr Thr Glu Glu Val
            420                 425                 430
Phe Val Pro Ile Pro Gly Glu Thr Val Val His Ile His Asn Gly Ile
        435                 440                 445
Gly Lys Phe Leu Gly Ile Glu Lys Lys Pro Asn His Leu Asn Ile Glu
    450                 455                 460

Thr Asp Tyr Leu Val Leu Glu Tyr Ala Asp Lys Ala Arg Leu Tyr Val
465                 470                 475                 480
Pro Ser Asn Gln Ala Tyr Leu Ile Ser Arg Tyr Val Gly Thr Ser Asp
                485                 490                 495
Lys Ala Ala Asp Leu His His Leu Asn Ser Ser Lys Trp Lys Arg Ser
            500                 505                 510
Arg Asp Leu Thr Glu Lys Ser Leu Ile Val Tyr Ala Glu Lys Leu Leu
        515                 520                 525
Gln Leu Glu Ala Gln Arg Ser Thr Thr Pro Ala Phe Val Tyr Pro Pro
    530                 535                 540
His Gly Glu Ser Val Ile Lys Phe Ala Glu Thr Phe Pro Tyr Glu Glu
545                 550                 555                 560
Thr Pro Asp Gln Leu Lys Thr Ile Asp Gln Ile Tyr Asn Asp Met Met
                565                 570                 575
Ser Pro Lys Leu Met Asp Arg Leu Ile Cys Gly Asp Ala Gly Phe Gly
            580                 585                 590
Lys Thr Glu Val Ile Met Arg Ala Ala Val Lys Ala Val Cys Asp Gly
        595                 600                 605
His Arg Gln Val Ile Val Met Val Pro Thr Thr Ile Leu Ala Thr Gln
    610                 615                 620
His Tyr Glu Thr Phe Lys Glu Arg Met Ala Gly Leu Pro Ile Glu Ile
625                 630                 635                 640
Ala Val Leu Ser Arg Phe Ser Gln Ala Lys Val Gln Lys Leu Ile Cys
                645                 650                 655
Glu Gln Val Ala Ser Gly Gln Ile Asp Ile Ile Ile Gly Thr His Lys
            660                 665                 670
Leu Ile Asn Lys Ser Leu Glu Phe Lys Asn Pro Gly Leu Leu Ile Ile
        675                 680                 685
Asp Glu Glu Gln Arg Phe Gly Val Lys Val Lys Asp Asn Leu Lys Glu
    690                 695                 700
Arg Tyr Pro Met Ile Asp Cys Leu Thr Val Ser Ala Thr Pro Ile Pro
705                 710                 715                 720
Arg Thr Leu His Met Ser Leu Ser Gly Ala Arg Asp Leu Ser Val Ile
                725                 730                 735
Ala Met Pro Pro Leu Asp Arg Leu Pro Val Ser Thr Phe Val Met Glu
            740                 745                 750
His Asn Thr Glu Thr Leu Thr Ala Ala Leu Arg His Glu Leu Leu Arg
        755                 760                 765
Gly Gly Gln Ala Tyr Val Ile His Asn Arg Ile Glu Ser Ile Tyr Thr
    770                 775                 780
Leu Ala Glu Thr Ile Arg Asn Leu Ile Pro Glu Ala Arg Ile Gly Val
785                 790                 795                 800
Ala His Gly Gln Met Gly Ala Glu Asp Leu Ser Asn Ile Phe Thr Lys
                805                 810                 815
Phe Lys Asn Gln Lys Thr Asp Ile Leu Val Ala Thr Ala Leu Ile Glu
            820                 825                 830
Asn Gly Ile Asp Ile Pro Asn Ala Asn Thr Ile Leu Ile Asp His Ala
        835                 840                 845

Asp Lys Phe Gly Met Ala Asp Leu Tyr Gln Met Lys Gly Arg Val Gly
    850                 855                 860
Arg Trp Asn Lys Lys Ala Tyr Cys Tyr Phe Leu Val Pro His Leu Asp
865                 870                 875                 880
Arg Leu Ser Gly Pro Ala Ala Lys Arg Leu Ala Ala Leu Asn Lys Gln
                885                 890                 895
Glu Tyr Gly Gly Gly Met Lys Ile Ala Leu His Asp Leu Glu Ile Arg
            900                 905                 910
Gly Ala Gly Asn Ile Leu Gly Thr Asp Gln Ser Gly His Ile Gly Thr
        915                 920                 925
Ile Gly Phe Asn Leu Tyr Cys Lys Leu Leu Lys Lys Ala Val Ser Ala
    930                 935                 940
Leu Lys Lys His Thr Ser Pro Leu Leu Phe Asn Asp Asp Val Lys Ile
945                 950                 955                 960
Glu Phe Pro Tyr Asn Ser Arg Ile Pro Asp Thr Tyr Ile Glu Thr Gly
                965                 970                 975
Ser Met Arg Ile Glu Phe Tyr Gln Lys Ile Gly Asn Ala Glu Ser Ser
            980                 985                 990
Glu Glu Leu Thr Ala Ile Gln Glu Glu Met Arg Asp Arg Phe Gly Pro
        995                 1000                1005
Leu Pro Gln Glu Ile Cys Trp Leu Phe Ala Leu Ala Glu Ile Arg Leu
    1010                1015                1020
Phe Ala Leu Gln His Gly Ile Ser Ser Ile Lys Gly Thr Ala Asn Ala
1025                1030                1035                1040
Leu Tyr Val Gln Lys Cys Leu Ser Lys Ser Glu Gln Thr Lys Lys Thr
                1045                1050                1055
Leu Pro Tyr Ala Leu Ser Pro Thr Pro Glu Leu Leu Val Lys Glu Val
            1060                1065                1070
Ile Glu Ser Ile Glu Arg Gly Phe Leu Ile Asn Ala Ser
        1075                1080                1085

<210> 31
<211> 1947
<212> DNA
<213> Chlamydia pneumoniae

<220>
<221> CDS
<222> (1)..(1947)

<400> 31
atg ttc gta atg aaa aaa ctt gtc cgt cta tgc gta gtt ctt ctt tct    48
Met Phe Val Met Lys Lys Leu Val Arg Leu Cys Val Val Leu Leu Ser
  1               5                  10                  15

tta ctt ccg aat gta tta ttt tct tcg gat ctt tta cga gaa gag ggc    96

Leu Leu Pro Asn Val Leu Phe Ser Ser Asp Leu Leu Arg Glu Glu Gly
            20                  25                  30

atc aaa aag atg atg gac aag ctg atc gag tat cat gtc gat gct caa 144
Ile Lys Lys Met Met Asp Lys Leu Ile Glu Tyr His Val Asp Ala Gln
        35                  40                  45

gag gtt tct acg gat ata ctc tcg cgt tct tta tct agt tac att caa 192
Glu Val Ser Thr Asp Ile Leu Ser Arg Ser Leu Ser Ser Tyr Ile Gln
    50                  55                  60

tct ttt gat cct cat aaa tct tat ctt tca aac caa gag gtt gca gtt 240
Ser Phe Asp Pro His Lys Ser Tyr Leu Ser Asn Gln Glu Val Ala Val
65                  70                  75                  80

ttt cta cag tct ccg gaa aca aag aaa cgt ctc tta aag aat tat aag 288
Phe Leu Gln Ser Pro Glu Thr Lys Lys Arg Leu Leu Lys Asn Tyr Lys
                85                  90                  95

gca ggc aac ttt gct att tat cgc aac atc aat caa tta att cat gag 336
Ala Gly Asn Phe Ala Ile Tyr Arg Asn Ile Asn Gln Leu Ile His Glu
            100                 105                 110

agt att ctt cgt gcc agg cag tgg aga aac gaa tgg gtt aag aat cca 384
Ser Ile Leu Arg Ala Arg Gln Trp Arg Asn Glu Trp Val Lys Asn Pro
        115                 120                 125

aaa gag ctt gta ttg gag gca tcc tca tat cag ata tcg aag caa cct 432
Lys Glu Leu Val Leu Glu Ala Ser Ser Tyr Gln Ile Ser Lys Gln Pro
    130                 135                 140

atg caa tgg agc aaa tct tta gac gaa gtg aag cag aga caa cgc gct 480
Met Gln Trp Ser Lys Ser Leu Asp Glu Val Lys Gln Arg Gln Arg Ala
145                 150                 155                 160

cta ctc ctt tcc tat ctt tct tta cat ctt gct gga gct tct tcc tct 528
Leu Leu Leu Ser Tyr Leu Ser Leu His Leu Ala Gly Ala Ser Ser Ser
                165                 170                 175

cgt tat gag ggt aaa gaa gag cag ctt gct gct ctg tgt cta cgt caa 576
Arg Tyr Glu Gly Lys Glu Glu Gln Leu Ala Ala Leu Cys Leu Arg Gln
            180                 185                 190

atc gag aac cat gag aat gta tat tta ggt atc aac gat cat ggt gtt 624
Ile Glu Asn His Glu Asn Val Tyr Leu Gly Ile Asn Asp His Gly Val
        195                 200                 205

gct atg gat cgg gat gaa gaa gcc tac caa ttc cat atc cgt gtt gtt 672

Ala Met Asp Arg Asp Glu Glu Ala Tyr Gln Phe His Ile Arg Val Val
    210                 215                 220

aaa gct tta gct cat agc tta gat gca cat acg gcg tat ttc agt aag     720
Lys Ala Leu Ala His Ser Leu Asp Ala His Thr Ala Tyr Phe Ser Lys
225                 230                 235                 240

gac gaa gcg ttg gcg atg cga atc caa cta gaa aaa ggc atg tgt gga     768
Asp Glu Ala Leu Ala Met Arg Ile Gln Leu Glu Lys Gly Met Cys Gly
                245                 250                 255

att ggt gtt gtt ctg aag gaa gat att gat gga gtt gtt gtt aga gaa     816
Ile Gly Val Val Leu Lys Glu Asp Ile Asp Gly Val Val Val Arg Glu
            260                 265                 270

atc att cct ggg gga cct gcg gct aaa tct ggg gat ctt cag ctt gga     864
Ile Ile Pro Gly Gly Pro Ala Ala Lys Ser Gly Asp Leu Gln Leu Gly
        275                 280                 285

gat atc atc tat cgg gtg gat ggc aag gat atc gag cat ctt tct ttc     912
Asp Ile Ile Tyr Arg Val Asp Gly Lys Asp Ile Glu His Leu Ser Phe
    290                 295                 300

cgc ggt gtt tta gat tgt tta cgt gga ggt cat ggc tct act gta gtc     960
Arg Gly Val Leu Asp Cys Leu Arg Gly Gly His Gly Ser Thr Val Val
305                 310                 315                 320

tta gat atc cat cgt ggg gag agc gat cat acg atc gcc ttg aga agg    1008
Leu Asp Ile His Arg Gly Glu Ser Asp His Thr Ile Ala Leu Arg Arg
                325                 330                 335

gag aaa atc ctt tta gaa gac cgt cgt gtg gat gtt tcc tat gag cct    1056
Glu Lys Ile Leu Leu Glu Asp Arg Arg Val Asp Val Ser Tyr Glu Pro
            340                 345                 350

tat gga gat ggt gtg att ggg aaa gtt acg tta cat tct ttt tat gaa    1104
Tyr Gly Asp Gly Val Ile Gly Lys Val Thr Leu His Ser Phe Tyr Glu
        355                 360                 365

gga gaa aat cag gtt tct agt gaa caa gat cta cgt cga gcg att cag    1152
Gly Glu Asn Gln Val Ser Ser Glu Gln Asp Leu Arg Arg Ala Ile Gln
    370                 375                 380

gga tta aag gag aag aac ctt ctt gga tta gtt tta gat atc cga gaa    1200
Gly Leu Lys Glu Lys Asn Leu Leu Gly Leu Val Leu Asp Ile Arg Glu
385                 390                 395                 400

aat acg ggt gga ttt tta tct caa gcg atc aaa gtt tct ggt tta ttt    1248

Asn Thr Gly Gly Phe Leu Ser Gln Ala Ile Lys Val Ser Gly Leu Phe
                405                 410                 415

atg acc aat ggc gtt gtg gtt gta tct cgc tat gct gat ggt acc atg 1296
Met Thr Asn Gly Val Val Val Val Ser Arg Tyr Ala Asp Gly Thr Met
            420                 425                 430

aag tgc tac cgc aca gta tct cct aaa aaa ttc tat gat ggt cct ttg 1344
Lys Cys Tyr Arg Thr Val Ser Pro Lys Lys Phe Tyr Asp Gly Pro Leu
        435                 440                 445

gct att tta gta tct aaa agt tcc gca tca gca gcg gag att gta gca 1392
Ala Ile Leu Val Ser Lys Ser Ser Ala Ser Ala Ala Glu Ile Val Ala
    450                 455                 460

caa act ctc caa gat tat gga gtt gct tta gtt gtt gga gat gag cag 1440
Gln Thr Leu Gln Asp Tyr Gly Val Ala Leu Val Val Gly Asp Glu Gln
465                 470                 475                 480

acc tat ggg aag gga acg att cag cat caa aca att act gga gat gcc 1488
Thr Tyr Gly Lys Gly Thr Ile Gln His Gln Thr Ile Thr Gly Asp Ala
                485                 490                 495

tct cag gac gat tgt ttt aag gtt act gta ggg aaa tat tat tcc cct 1536
Ser Gln Asp Asp Cys Phe Lys Val Thr Val Gly Lys Tyr Tyr Ser Pro
            500                 505                 510

tct ggg aaa tcg act caa ctt cag gga gta aaa tcc gat att tta att 1584
Ser Gly Lys Ser Thr Gln Leu Gln Gly Val Lys Ser Asp Ile Leu Ile
        515                 520                 525

cct tct ctc tat gct gaa gat cgt cta gga gag cgt ttt cta gag cat 1632
Pro Ser Leu Tyr Ala Glu Asp Arg Leu Gly Glu Arg Phe Leu Glu His
    530                 535                 540

ccc tta cct gca gat tgc tgt gat aat gta ctt cac gat cct ctc acg 1680
Pro Leu Pro Ala Asp Cys Cys Asp Asn Val Leu His Asp Pro Leu Thr
545                 550                 555                 560

gac ttg gat act caa aca cgt cct tgg ttt caa aaa tac tat ctt cct 1728
Asp Leu Asp Thr Gln Thr Arg Pro Trp Phe Gln Lys Tyr Tyr Leu Pro
                565                 570                 575

aat cta caa aag caa gag act ctt tgg aga gag atg cta cct cag ctt 1776
Asn Leu Gln Lys Gln Glu Thr Leu Trp Arg Glu Met Leu Pro Gln Leu
            580                 585                 590

acg aaa aac agt gag caa agg ctt tct gag aat tcg aat ttt cag gca 1824

Thr Lys Asn Ser Glu Gln Arg Leu Ser Glu Asn Ser Asn Phe Gln Ala
        595                 600                 605

ttt ttg tcg cag ata aaa tca tct gaa aaa acg gac cta tcc tat ggt     1872
Phe Leu Ser Gln Ile Lys Ser Ser Glu Lys Thr Asp Leu Ser Tyr Gly
    610                 615                 620

tcc aat gat tta caa ttg gaa gag tcg ata aac att ttg aag gac atg     1920
Ser Asn Asp Leu Gln Leu Glu Glu Ser Ile Asn Ile Leu Lys Asp Met
625                 630                 635                 640

att tta tta caa cag tgt aga aaa taa                                 1947
Ile Leu Leu Gln Gln Cys Arg Lys
                645

<210> 32
<211> 648
<212> PRT
<213> Chlamydia pneumoniae

<400> 32
Met Phe Val Met Lys Lys Leu Val Arg Leu Cys Val Val Leu Leu Ser
  1               5                  10                  15
Leu Leu Pro Asn Val Leu Phe Ser Ser Asp Leu Leu Arg Glu Glu Gly
             20                  25                  30
Ile Lys Lys Met Met Asp Lys Leu Ile Glu Tyr His Val Asp Ala Gln
         35                  40                  45
Glu Val Ser Thr Asp Ile Leu Ser Arg Ser Leu Ser Ser Tyr Ile Gln
     50                  55                  60
Ser Phe Asp Pro His Lys Ser Tyr Leu Ser Asn Gln Glu Val Ala Val
 65                  70                  75                  80
Phe Leu Gln Ser Pro Glu Thr Lys Lys Arg Leu Leu Lys Asn Tyr Lys
                 85                  90                  95
Ala Gly Asn Phe Ala Ile Tyr Arg Asn Ile Asn Gln Leu Ile His Glu
            100                 105                 110
Ser Ile Leu Arg Ala Arg Gln Trp Arg Asn Glu Trp Val Lys Asn Pro
        115                 120                 125
Lys Glu Leu Val Leu Glu Ala Ser Ser Tyr Gln Ile Ser Lys Gln Pro
    130                 135                 140
Met Gln Trp Ser Lys Ser Leu Asp Glu Val Lys Gln Arg Gln Arg Ala
145                 150                 155                 160
Leu Leu Leu Ser Tyr Leu Ser Leu His Leu Ala Gly Ala Ser Ser Ser
                165                 170                 175
Arg Tyr Glu Gly Lys Glu Glu Gln Leu Ala Ala Leu Cys Leu Arg Gln
            180                 185                 190
Ile Glu Asn His Glu Asn Val Tyr Leu Gly Ile Asn Asp His Gly Val
        195                 200                 205

Ala Met Asp Arg Asp Glu Glu Ala Tyr Gln Phe His Ile Arg Val Val
    210                 215                 220
Lys Ala Leu Ala His Ser Leu Asp Ala His Thr Ala Tyr Phe Ser Lys
225                 230                 235                 240
Asp Glu Ala Leu Ala Met Arg Ile Gln Leu Glu Lys Gly Met Cys Gly
                245                 250                 255
Ile Gly Val Val Leu Lys Glu Asp Ile Asp Gly Val Val Val Arg Glu
            260                 265                 270
Ile Ile Pro Gly Gly Pro Ala Ala Lys Ser Gly Asp Leu Gln Leu Gly
        275                 280                 285
Asp Ile Ile Tyr Arg Val Asp Gly Lys Asp Ile Glu His Leu Ser Phe
    290                 295                 300
Arg Gly Val Leu Asp Cys Leu Arg Gly Gly His Gly Ser Thr Val Val
305                 310                 315                 320
Leu Asp Ile His Arg Gly Glu Ser Asp His Thr Ile Ala Leu Arg Arg
                325                 330                 335
Glu Lys Ile Leu Leu Glu Asp Arg Arg Val Asp Val Ser Tyr Glu Pro
            340                 345                 350
Tyr Gly Asp Gly Val Ile Gly Lys Val Thr Leu His Ser Phe Tyr Glu
        355                 360                 365
Gly Glu Asn Gln Val Ser Ser Glu Gln Asp Leu Arg Arg Ala Ile Gln
    370                 375                 380
Gly Leu Lys Glu Lys Asn Leu Leu Gly Leu Val Leu Asp Ile Arg Glu
385                 390                 395                 400
Asn Thr Gly Gly Phe Leu Ser Gln Ala Ile Lys Val Ser Gly Leu Phe
                405                 410                 415
Met Thr Asn Gly Val Val Val Val Ser Arg Tyr Ala Asp Gly Thr Met
            420                 425                 430
Lys Cys Tyr Arg Thr Val Ser Pro Lys Lys Phe Tyr Asp Gly Pro Leu
        435                 440                 445
Ala Ile Leu Val Ser Lys Ser Ser Ala Ser Ala Ala Glu Ile Val Ala
    450                 455                 460
Gln Thr Leu Gln Asp Tyr Gly Val Ala Leu Val Val Gly Asp Glu Gln
465                 470                 475                 480
Thr Tyr Gly Lys Gly Thr Ile Gln His Gln Thr Ile Thr Gly Asp Ala
                485                 490                 495
Ser Gln Asp Asp Cys Phe Lys Val Thr Val Gly Lys Tyr Tyr Ser Pro
            500                 505                 510
Ser Gly Lys Ser Thr Gln Leu Gln Gly Val Lys Ser Asp Ile Leu Ile
        515                 520                 525
Pro Ser Leu Tyr Ala Glu Asp Arg Leu Gly Glu Arg Phe Leu Glu His
    530                 535                 540
Pro Leu Pro Ala Asp Cys Cys Asp Asn Val Leu His Asp Pro Leu Thr
545                 550                 555                 560
Asp Leu Asp Thr Gln Thr Arg Pro Trp Phe Gln Lys Tyr Tyr Leu Pro
                565                 570                 575
Asn Leu Gln Lys Gln Glu Thr Leu Trp Arg Glu Met Leu Pro Gln Leu
            580                 585                 590

Thr Lys Asn Ser Glu Gln Arg Leu Ser Glu Asn Ser Asn Phe Gln Ala
        595                 600                 605
Phe Leu Ser Gln Ile Lys Ser Ser Glu Lys Thr Asp Leu Ser Tyr Gly
    610                 615                 620
Ser Asn Asp Leu Gln Leu Glu Glu Ser Ile Asn Ile Leu Lys Asp Met
625                 630                 635                 640
Ile Leu Leu Gln Gln Cys Arg Lys
                645

<210> 33
<211> 1323
<212> DNA
<213> Chlamydia pneumoniae

<220>
<221> CDS
<222> (1)..(1323)

<400> 33
gtg tct aag ata gtt tat aaa ttt ggt ggc act agc tta gca act gct      48
Val Ser Lys Ile Val Tyr Lys Phe Gly Gly Thr Ser Leu Ala Thr Ala
  1               5                  10                  15

gag aat atc tgt ttg gtt tgt gat atc att tgc aaa gat aag cct tct      96
Glu Asn Ile Cys Leu Val Cys Asp Ile Ile Cys Lys Asp Lys Pro Ser
             20                  25                  30

ttt gtt gtt gta agc gca atc gcc ggt gtg acg gac ctc ctt gta gac     144
Phe Val Val Val Ser Ala Ile Ala Gly Val Thr Asp Leu Leu Val Asp
         35                  40                  45

ttc tgc tcg tct tct tta aga gaa cga gag gag gtc tta aga aaa ata     192
Phe Cys Ser Ser Ser Leu Arg Glu Arg Glu Glu Val Leu Arg Lys Ile
     50                  55                  60

gag gga aaa cat gag gag att gta aaa aac cta gcg att cct ttt cct     240
Glu Gly Lys His Glu Glu Ile Val Lys Asn Leu Ala Ile Pro Phe Pro
 65                  70                  75                  80

gtc tct aca tgg acg tct cga ctc ctt ccc tat cta caa cat ctg gag     288
Val Ser Thr Trp Thr Ser Arg Leu Leu Pro Tyr Leu Gln His Leu Glu
                 85                  90                  95

atc tca gat ctc gat ttt gct cgt att ttg tct tta gga gaa gat att     336
Ile Ser Asp Leu Asp Phe Ala Arg Ile Leu Ser Leu Gly Glu Asp Ile
            100                 105                 110

tca gct tcc cta gtt cgt gct gtt tgt agc acc cgt ggt tgg gat tta 384
Ser Ala Ser Leu Val Arg Ala Val Cys Ser Thr Arg Gly Trp Asp Leu
        115                 120                 125

gga ttt ctc gag gca cgt agt gtc atc tta aca gac gat agc tac cga 432
Gly Phe Leu Glu Ala Arg Ser Val Ile Leu Thr Asp Asp Ser Tyr Arg
    130                 135                 140

cgt gcc tct cca aac cta gat ctt atg aaa gca cat tgg cat cag ctc 480
Arg Ala Ser Pro Asn Leu Asp Leu Met Lys Ala His Trp His Gln Leu
145                 150                 155                 160

gaa cta aat cag cct tcg tat att atc cag ggg ttc atc gga tct aat 528
Glu Leu Asn Gln Pro Ser Tyr Ile Ile Gln Gly Phe Ile Gly Ser Asn
                165                 170                 175

ggt ttg gga gaa aca gtt ctt ctt ggg cgc gga ggc agt gat tat tca 576
Gly Leu Gly Glu Thr Val Leu Leu Gly Arg Gly Gly Ser Asp Tyr Ser
            180                 185                 190

gca act ttg atc gca gag ctt gca aga gca aca gaa gtg cgt att tat 624
Ala Thr Leu Ile Ala Glu Leu Ala Arg Ala Thr Glu Val Arg Ile Tyr
        195                 200                 205

acc gat gtt aat ggg atc tat acc atg gat cct aaa gtg att tcc gat 672
Thr Asp Val Asn Gly Ile Tyr Thr Met Asp Pro Lys Val Ile Ser Asp
    210                 215                 220

gca cag cgc att cct gag ctc agt ttc gaa gag atg cag aat tta gca 720
Ala Gln Arg Ile Pro Glu Leu Ser Phe Glu Glu Met Gln Asn Leu Ala
225                 230                 235                 240

agt ttt ggt gct aaa gtc ctt tat cct cct atg ctc ttt cct tgt atg 768
Ser Phe Gly Ala Lys Val Leu Tyr Pro Pro Met Leu Phe Pro Cys Met
                245                 250                 255

cgt gcg gga att cct att ttt gtg aca tca aca ttt gac cct gaa aaa 816
Arg Ala Gly Ile Pro Ile Phe Val Thr Ser Thr Phe Asp Pro Glu Lys
            260                 265                 270

gga gga aca tgg gtc tat gct gtc gat aag tct gtg agt tat gaa cca 864
Gly Gly Thr Trp Val Tyr Ala Val Asp Lys Ser Val Ser Tyr Glu Pro
        275                 280                 285

aga ata aaa gct tta tcc tta agt caa tac caa agc ttc tgt tct gta 912
Arg Ile Lys Ala Leu Ser Leu Ser Gln Tyr Gln Ser Phe Cys Ser Val
    290                 295                 300

gac tat act gtc cta gga tgt ggg gga tta gaa gag att tta ggc att     960
Asp Tyr Thr Val Leu Gly Cys Gly Gly Leu Glu Glu Ile Leu Gly Ile
305                 310                 315                 320

tta gaa tcc cat ggg ata gat cct gaa ttg atg ata gca caa aac aac     1008
Leu Glu Ser His Gly Ile Asp Pro Glu Leu Met Ile Ala Gln Asn Asn
                325                 330                 335

gtc gtt gga ttt gta atg gat gat gat atc att tct caa gaa gct caa     1056
Val Val Gly Phe Val Met Asp Asp Asp Ile Ile Ser Gln Glu Ala Gln
            340                 345                 350

gag cac ctt gta gat gtt tta tcg cta tct agt gtc aca cgc ttg cat     1104
Glu His Leu Val Asp Val Leu Ser Leu Ser Ser Val Thr Arg Leu His
        355                 360                 365

cat agt gtt gca ttg att acc atg atc gga gat aat ctt tct tct cca     1152
His Ser Val Ala Leu Ile Thr Met Ile Gly Asp Asn Leu Ser Ser Pro
    370                 375                 380

aaa gtt gtc tca aca att acg gag aaa ctc aga ggt ttt caa gga cct     1200
Lys Val Val Ser Thr Ile Thr Glu Lys Leu Arg Gly Phe Gln Gly Pro
385                 390                 395                 400

gta ttt tgt ttt tgc caa agt tca atg gca tta agc ttt gtt gtt gcc     1248
Val Phe Cys Phe Cys Gln Ser Ser Met Ala Leu Ser Phe Val Val Ala
                405                 410                 415

tca gag ttg gca gag ggt att ata gaa gaa tta cat aat gat tat gta     1296
Ser Glu Leu Ala Glu Gly Ile Ile Glu Glu Leu His Asn Asp Tyr Val
            420                 425                 430

aaa caa aaa gct ata gtc gcc acg tag                                 1323
Lys Gln Lys Ala Ile Val Ala Thr
        435                 440

<210> 34
<211> 440
<212> PRT
<213> Chlamydia pneumoniae

<400> 34
Val Ser Lys Ile Val Tyr Lys Phe Gly Gly Thr Ser Leu Ala Thr Ala
 1               5                  10                  15
Glu Asn Ile Cys Leu Val Cys Asp Ile Ile Cys Lys Asp Lys Pro Ser
            20                  25                  30

Phe Val Val Val Ser Ala Ile Ala Gly Val Thr Asp Leu Leu Val Asp
        35                  40                  45
Phe Cys Ser Ser Ser Leu Arg Glu Arg Glu Glu Val Leu Arg Lys Ile
    50                  55                  60
Glu Gly Lys His Glu Glu Ile Val Lys Asn Leu Ala Ile Pro Phe Pro
65                  70                  75                  80
Val Ser Thr Trp Thr Ser Arg Leu Leu Pro Tyr Leu Gln His Leu Glu
                85                  90                  95
Ile Ser Asp Leu Asp Phe Ala Arg Ile Leu Ser Leu Gly Glu Asp Ile
            100                 105                 110
Ser Ala Ser Leu Val Arg Ala Val Cys Ser Thr Arg Gly Trp Asp Leu
        115                 120                 125
Gly Phe Leu Glu Ala Arg Ser Val Ile Leu Thr Asp Asp Ser Tyr Arg
    130                 135                 140
Arg Ala Ser Pro Asn Leu Asp Leu Met Lys Ala His Trp His Gln Leu
145                 150                 155                 160
Glu Leu Asn Gln Pro Ser Tyr Ile Ile Gln Gly Phe Ile Gly Ser Asn
                165                 170                 175
Gly Leu Gly Glu Thr Val Leu Leu Gly Arg Gly Gly Ser Asp Tyr Ser
            180                 185                 190
Ala Thr Leu Ile Ala Glu Leu Ala Arg Ala Thr Glu Val Arg Ile Tyr
        195                 200                 205
Thr Asp Val Asn Gly Ile Tyr Thr Met Asp Pro Lys Val Ile Ser Asp
    210                 215                 220
Ala Gln Arg Ile Pro Glu Leu Ser Phe Glu Glu Met Gln Asn Leu Ala
225                 230                 235                 240
Ser Phe Gly Ala Lys Val Leu Tyr Pro Pro Met Leu Phe Pro Cys Met
                245                 250                 255
Arg Ala Gly Ile Pro Ile Phe Val Thr Ser Thr Phe Asp Pro Glu Lys
            260                 265                 270
Gly Gly Thr Trp Val Tyr Ala Val Asp Lys Ser Val Ser Tyr Glu Pro
        275                 280                 285
Arg Ile Lys Ala Leu Ser Leu Ser Gln Tyr Gln Ser Phe Cys Ser Val
    290                 295                 300
Asp Tyr Thr Val Leu Gly Cys Gly Gly Leu Glu Glu Ile Leu Gly Ile
305                 310                 315                 320
Leu Glu Ser His Gly Ile Asp Pro Glu Leu Met Ile Ala Gln Asn Asn
                325                 330                 335
Val Val Gly Phe Val Met Asp Asp Asp Ile Ile Ser Gln Glu Ala Gln
            340                 345                 350
Glu His Leu Val Asp Val Leu Ser Leu Ser Ser Val Thr Arg Leu His
        355                 360                 365
His Ser Val Ala Leu Ile Thr Met Ile Gly Asp Asn Leu Ser Ser Pro
    370                 375                 380
Lys Val Val Ser Thr Ile Thr Glu Lys Leu Arg Gly Phe Gln Gly Pro
385                 390                 395                 400
Val Phe Cys Phe Cys Gln Ser Ser Met Ala Leu Ser Phe Val Val Ala
                405                 410                 415

Ser Glu Leu Ala Glu Gly Ile Ile Glu Glu Leu His Asn Asp Tyr Val
            420                 425                 430
Lys Gln Lys Ala Ile Val Ala Thr
        435                 440

<210> 35
<211> 933
<212> DNA
<213> Chlamydia pneumoniae

<220>
<221> CDS
<222> (1)..(933)

<400> 35
atg aga aaa ctt att tta tgc aat cct aga gga ttt tgc tct gga gtt      48
Met Arg Lys Leu Ile Leu Cys Asn Pro Arg Gly Phe Cys Ser Gly Val
  1               5                  10                  15

gtg cgc gct att caa gtt gta gag gtt gct tta gaa aag tgg gga gct      96
Val Arg Ala Ile Gln Val Val Glu Val Ala Leu Glu Lys Trp Gly Ala
             20                  25                  30

cct atc tat gta aaa cat gag att gtt cac aat cgc cat gtt gtt aat     144
Pro Ile Tyr Val Lys His Glu Ile Val His Asn Arg His Val Val Asn
         35                  40                  45

gct tta cga gcc aag gga gcg atc ttt gtt gaa gaa ctt gtt gat gtt     192
Ala Leu Arg Ala Lys Gly Ala Ile Phe Val Glu Glu Leu Val Asp Val
     50                  55                  60

cct gaa ggt gag aga gtc att tat tca gct cat gga att cct cct tca     240
Pro Glu Gly Glu Arg Val Ile Tyr Ser Ala His Gly Ile Pro Pro Ser
 65                  70                  75                  80

gtt aga gct gaa gca aaa gcc cgt aag ctt att gat att gat gct acc     288
Val Arg Ala Glu Ala Lys Ala Arg Lys Leu Ile Asp Ile Asp Ala Thr
                 85                  90                  95

tgt ggt ttg gtt act aag gtg cat tct gct gcg aag tta tac gca agt     336
Cys Gly Leu Val Thr Lys Val His Ser Ala Ala Lys Leu Tyr Ala Ser
            100                 105                 110

aaa gga tac aaa atc ata ctg atc ggc cat aag aag cac gtt gag gtg     384
Lys Gly Tyr Lys Ile Ile Leu Ile Gly His Lys Lys His Val Glu Val
        115                 120                 125

att ggt att gtt gga gaa gtt cct gaa cac att act gtt gtc gag aag 432
Ile Gly Ile Val Gly Glu Val Pro Glu His Ile Thr Val Val Glu Lys
    130                 135                 140

gtt gct gac gtc gag gcc tta cct ttt agt tct gat aca cct tta ttt 480
Val Ala Asp Val Glu Ala Leu Pro Phe Ser Ser Asp Thr Pro Leu Phe
145                 150                 155                 160

tat att act caa acg acg ttg agt ttg gat gat gtt cag gag atc tca 528
Tyr Ile Thr Gln Thr Thr Leu Ser Leu Asp Asp Val Gln Glu Ile Ser
                165                 170                 175

tcg gct ttg cta aag cga tat ccc tct atc att act ctg cct agt tct 576
Ser Ala Leu Leu Lys Arg Tyr Pro Ser Ile Ile Thr Leu Pro Ser Ser
            180                 185                 190

tcg att tgt tat gca acc acg aac cgt caa aaa gca ttg cgt tct gtt 624
Ser Ile Cys Tyr Ala Thr Thr Asn Arg Gln Lys Ala Leu Arg Ser Val
        195                 200                 205

tta tct cgc gtg aat tac gtc tat gtg gtt gga gat gtc aac agc tcg 672
Leu Ser Arg Val Asn Tyr Val Tyr Val Val Gly Asp Val Asn Ser Ser
    210                 215                 220

aat tcc aat cgt ctt cgc gaa gtg gct ttg aga agg gga gtt ccc gct 720
Asn Ser Asn Arg Leu Arg Glu Val Ala Leu Arg Arg Gly Val Pro Ala
225                 230                 235                 240

gat ttg atc aac aat ccc gag gat att gat acg aac atc gta aat cat 768
Asp Leu Ile Asn Asn Pro Glu Asp Ile Asp Thr Asn Ile Val Asn His
                245                 250                 255

tct gga gat ata gca atg act gca gga gcc tca act ccc gaa gac gta 816
Ser Gly Asp Ile Ala Met Thr Ala Gly Ala Ser Thr Pro Glu Asp Val
            260                 265                 270

gtt caa gct tgc att cga aag cta tca tca ctt atc cct ggt tta caa 864
Val Gln Ala Cys Ile Arg Lys Leu Ser Ser Leu Ile Pro Gly Leu Gln
        275                 280                 285

gtg gaa aat gat ata ttt gct gta gag gat gtc gta ttt caa tta cca 912
Val Glu Asn Asp Ile Phe Ala Val Glu Asp Val Val Phe Gln Leu Pro
    290                 295                 300

aaa gaa ctc cgt tgt tct tag 933
Lys Glu Leu Arg Cys Ser
305                 310

<210> 36
<211> 310
<212> PRT
<213> Chlamydia pneumoniae

<400> 36
Met Arg Lys Leu Ile Leu Cys Asn Pro Arg Gly Phe Cys Ser Gly Val
1               5                   10                  15
Val Arg Ala Ile Gln Val Val Glu Val Ala Leu Glu Lys Trp Gly Ala
            20                  25                  30
Pro Ile Tyr Val Lys His Glu Ile Val His Asn Arg His Val Val Asn
        35                  40                  45
Ala Leu Arg Ala Lys Gly Ala Ile Phe Val Glu Glu Leu Val Asp Val
    50                  55                  60
Pro Glu Gly Glu Arg Val Ile Tyr Ser Ala His Gly Ile Pro Pro Ser
65                  70                  75                  80
Val Arg Ala Glu Ala Lys Ala Arg Lys Leu Ile Asp Ile Asp Ala Thr
                85                  90                  95
Cys Gly Leu Val Thr Lys Val His Ser Ala Ala Lys Leu Tyr Ala Ser
            100                 105                 110
Lys Gly Tyr Lys Ile Ile Leu Ile Gly His Lys Lys His Val Glu Val
        115                 120                 125
Ile Gly Ile Val Gly Glu Val Pro Glu His Ile Thr Val Val Glu Lys
    130                 135                 140
Val Ala Asp Val Glu Ala Leu Pro Phe Ser Ser Asp Thr Pro Leu Phe
145                 150                 155                 160
Tyr Ile Thr Gln Thr Thr Leu Ser Leu Asp Asp Val Gln Glu Ile Ser
                165                 170                 175
Ser Ala Leu Leu Lys Arg Tyr Pro Ser Ile Ile Thr Leu Pro Ser Ser
            180                 185                 190
Ser Ile Cys Tyr Ala Thr Thr Asn Arg Gln Lys Ala Leu Arg Ser Val
        195                 200                 205
Leu Ser Arg Val Asn Tyr Val Tyr Val Val Gly Asp Val Asn Ser Ser
    210                 215                 220
Asn Ser Asn Arg Leu Arg Glu Val Ala Leu Arg Arg Gly Val Pro Ala
225                 230                 235                 240
Asp Leu Ile Asn Asn Pro Glu Asp Ile Asp Thr Asn Ile Val Asn His
                245                 250                 255
Ser Gly Asp Ile Ala Met Thr Ala Gly Ala Ser Thr Pro Glu Asp Val
            260                 265                 270
Val Gln Ala Cys Ile Arg Lys Leu Ser Ser Leu Ile Pro Gly Leu Gln
        275                 280                 285
Val Glu Asn Asp Ile Phe Ala Val Glu Asp Val Val Phe Gln Leu Pro
    290                 295                 300
Lys Glu Leu Arg Cys Ser
305                 310

<210> 37
<211> 882
<212> DNA
<213> Chlamydia pneumoniae

<220>
<221> CDS
<222> (1)..(882)

<400> 37
atg ctc tcc tca cta atc cgt gat tca ttt ccc ctt ctt att tta ctt 48
Met Leu Ser Ser Leu Ile Arg Asp Ser Phe Pro Leu Leu Ile Leu Leu
1               5                   10                  15

ccc aca ttc cta gcg gca tta gga gcc tcc gta gct ggc ggc gtt atg 96
Pro Thr Phe Leu Ala Ala Leu Gly Ala Ser Val Ala Gly Gly Val Met
            20                  25                  30

gga acc tat atc gtt gta aaa cgt att gtt tca att agt gga agt ata 144
Gly Thr Tyr Ile Val Val Lys Arg Ile Val Ser Ile Ser Gly Ser Ile
        35                  40                  45

tct cat gca att cta gga gga att ggc ctc acc cta tgg ata caa tat 192
Ser His Ala Ile Leu Gly Gly Ile Gly Leu Thr Leu Trp Ile Gln Tyr
    50                  55                  60

aag ctt cat ctc tct ttt ttc cct atg tat gga gct att gta gga gct 240
Lys Leu His Leu Ser Phe Phe Pro Met Tyr Gly Ala Ile Val Gly Ala
65                  70                  75                  80

att ttt cta gct ctt tgc atc ggc aag atc cac ctg aaa tac caa gaa 288
Ile Phe Leu Ala Leu Cys Ile Gly Lys Ile His Leu Lys Tyr Gln Glu
                85                  90                  95

agg gaa gac tct ttg att gcg atg att tgg tct gtg ggc atg gca att 336
Arg Glu Asp Ser Leu Ile Ala Met Ile Trp Ser Val Gly Met Ala Ile
            100                 105                 110

gga att ata ttc att tcc agg ctt ccc acc ttt aat gga gag ctc atc 384
Gly Ile Ile Phe Ile Ser Arg Leu Pro Thr Phe Asn Gly Glu Leu Ile
        115                 120                 125

aat ttt cta ttt ggg aac att ctc tgg gtc acc cct tca gac ctc tat 432
Asn Phe Leu Phe Gly Asn Ile Leu Trp Val Thr Pro Ser Asp Leu Tyr
    130                 135                 140

agc tta gga atc ttt gat ctt ctt gtt tta gga att gtg gtc ctt tgc     480
Ser Leu Gly Ile Phe Asp Leu Leu Val Leu Gly Ile Val Val Leu Cys
145                 150                 155                 160

cac acc cgg ttc ctt gct ctt tgc ttt gat gag agg tac acg gct tta     528
His Thr Arg Phe Leu Ala Leu Cys Phe Asp Glu Arg Tyr Thr Ala Leu
                165                 170                 175

aac cat tgt tct gta cag ctg tgg tat ttc cta ctt ctt gtt ctg aca     576
Asn His Cys Ser Val Gln Leu Trp Tyr Phe Leu Leu Leu Val Leu Thr
            180                 185                 190

gca atc acg att gtg atg ttg att tat gtg atg gga acg att ctg atg     624
Ala Ile Thr Ile Val Met Leu Ile Tyr Val Met Gly Thr Ile Leu Met
        195                 200                 205

ctt agc atg ctc gtc tta cct gtt gct ata gcg tgt aga ttt tcg tac     672
Leu Ser Met Leu Val Leu Pro Val Ala Ile Ala Cys Arg Phe Ser Tyr
    210                 215                 220

aag atg aca cga att atg ttc atc tcg gtc ctc ttg aat atc tta tgt     720
Lys Met Thr Arg Ile Met Phe Ile Ser Val Leu Leu Asn Ile Leu Cys
225                 230                 235                 240

tct ttt tct gga att tgc atc gcc tac tgt tta gat ttc cca gta ggt     768
Ser Phe Ser Gly Ile Cys Ile Ala Tyr Cys Leu Asp Phe Pro Val Gly
                245                 250                 255

cct acg ata tca ttg ctg atg ggg tta ggt tat aca gcg agt ctt tgt     816
Pro Thr Ile Ser Leu Leu Met Gly Leu Gly Tyr Thr Ala Ser Leu Cys
            260                 265                 270

gtg aag aag cgg tac aat ccg tcg acg cct tct cct gta agt cct gaa     864
Val Lys Lys Arg Tyr Asn Pro Ser Thr Pro Ser Pro Val Ser Pro Glu
        275                 280                 285

atc aat aca aat gta tag                                             882
Ile Asn Thr Asn Val
    290

<210> 38
<211> 293
<212> PRT
<213> Chlamydia pneumoniae

<400> 38

Met Leu Ser Ser Leu Ile Arg Asp Ser Phe Pro Leu Leu Ile Leu Leu
1               5                   10                  15
Pro Thr Phe Leu Ala Ala Leu Gly Ala Ser Val Ala Gly Gly Val Met
            20                  25                  30
Gly Thr Tyr Ile Val Val Lys Arg Ile Val Ser Ile Ser Gly Ser Ile
        35                  40                  45
Ser His Ala Ile Leu Gly Gly Ile Gly Leu Thr Leu Trp Ile Gln Tyr
    50                  55                  60
Lys Leu His Leu Ser Phe Phe Pro Met Tyr Gly Ala Ile Val Gly Ala
65                  70                  75                  80
Ile Phe Leu Ala Leu Cys Ile Gly Lys Ile His Leu Lys Tyr Gln Glu
                85                  90                  95
Arg Glu Asp Ser Leu Ile Ala Met Ile Trp Ser Val Gly Met Ala Ile
            100                 105                 110
Gly Ile Ile Phe Ile Ser Arg Leu Pro Thr Phe Asn Gly Glu Leu Ile
        115                 120                 125
Asn Phe Leu Phe Gly Asn Ile Leu Trp Val Thr Pro Ser Asp Leu Tyr
    130                 135                 140
Ser Leu Gly Ile Phe Asp Leu Leu Val Leu Gly Ile Val Val Leu Cys
145                 150                 155                 160
His Thr Arg Phe Leu Ala Leu Cys Phe Asp Glu Arg Tyr Thr Ala Leu
                165                 170                 175
Asn His Cys Ser Val Gln Leu Trp Tyr Phe Leu Leu Leu Val Leu Thr
            180                 185                 190
Ala Ile Thr Ile Val Met Leu Ile Tyr Val Met Gly Thr Ile Leu Met
        195                 200                 205
Leu Ser Met Leu Val Leu Pro Val Ala Ile Ala Cys Arg Phe Ser Tyr
    210                 215                 220
Lys Met Thr Arg Ile Met Phe Ile Ser Val Leu Leu Asn Ile Leu Cys
225                 230                 235                 240
Ser Phe Ser Gly Ile Cys Ile Ala Tyr Cys Leu Asp Phe Pro Val Gly
                245                 250                 255
Pro Thr Ile Ser Leu Leu Met Gly Leu Gly Tyr Thr Ala Ser Leu Cys
            260                 265                 270
Val Lys Lys Arg Tyr Asn Pro Ser Thr Pro Ser Pro Val Ser Pro Glu
        275                 280                 285
Ile Asn Thr Asn Val
    290

<210> 39
<211> 34
<212> DNA
<213> Artificial Sequence

<220>
<223> Description of Artificial Sequence:

oligonucleotide primer

<400> 39
ctctccgtcc atatgtgtga cgtacggtct aagg                                34

<210> 40
<211> 46
<212> DNA
<213> Artificial Sequence

<220>
<223> Description of Artificial Sequence:
      oligonucleotide primer

<400> 40
tcaggaggat ccacttactt agtcattcac cttgatttcc ttcttg                   46

<210> 41
<211> 37
<212> DNA
<213> Artificial Sequence

<220>
<223> Description of Artificial Sequence:
      oligonucleotide primer

<400> 41
cacaacgtgt tcatatgaaa atcaccacag tcaaaac                             37

<210> 42
<211> 42
<212> DNA
<213> Artificial Sequence

<220>
<223> Description of Artificial Sequence:
      oligonucleotide primer

<400> 42
ctatccggat ccacttactt agtcaggagg ttggtgcggg ag                       42

<210> 43
<211> 30
<212> DNA

<213> Artificial Sequence

<220>
<223> Description of Artificial Sequence:
      oligonucleotide primer

<400> 43
gggaattcca tatgctccgt ttcttcgctg                                        30


<210> 44
<211> 40
<212> DNA
<213> Artificial Sequence

<220>
<223> Description of Artificial Sequence:
      oligonucleotide primer

<400> 44
ggtcctcgag ttacttagct atagtttttt ctataaaacg                             40


<210> 45
<211> 27
<212> DNA
<213> Artificial Sequence

<220>
<223> Description of Artificial Sequence:
      oligonucleotide primer

<400> 45
ccgctaccgc catatgtcat ctcctgt                                           27


<210> 46
<211> 37
<212> DNA
<213> Artificial Sequence

<220>
<223> Description of Artificial Sequence:
      oligonucleotide primer

<400> 46
gcccgctcga gctacttact tactgaccat ctcctgt                                37

<210> 47
<211> 35
<212> DNA
<213> Artificial Sequence

<220>
<223> Description of Artificial Sequence:
      oligonucleotide primer

<400> 47
ctctataggt ccatatgaaa ctacttctga aagcg                                35


<210> 48
<211> 29
<212> DNA
<213> Artificial Sequence

<220>
<223> Description of Artificial Sequence:
      oligonucleotide primer

<400> 48
gatccctcga gagttgttat tgccatgtc                                       29


<210> 49
<211> 29
<212> DNA
<213> Artificial Sequence

<220>
<223> Description of Artificial Sequence:
      oligonucleotide primer

<400> 49
catgggggac atatggctgt tcaatctat                                       29


<210> 50
<211> 34
<212> DNA
<213> Artificial Sequence

<220>
<223> Description of Artificial Sequence:
      oligonucleotide primer

<400> 50
gtcgctcgag tacttaccta acttcccgcc cctg                                34

<210> 51
<211> 31
<212> DNA
<213> Artificial Sequence

<220>
<223> Description of Artificial Sequence:
      oligonucleotide primer

<400> 51
gttggaattc atatgagcag ttcggaagtt g                                   31

<210> 52
<211> 32
<212> DNA
<213> Artificial Sequence

<220>
<223> Description of Artificial Sequence:
      oligonucleotide primer

<400> 52
gggcctcgag ttactcaatc atctgacatc tg                                  32

<210> 53
<211> 31
<212> DNA
<213> Artificial Sequence

<220>
<223> Description of Artificial Sequence:
      oligonucleotide primer

<400> 53
gtagttaatc catgggaact cctatatctg g                                   31

<210> 54
<211> 33
<212> DNA
<213> Artificial Sequence

<220>
<223> Description of Artificial Sequence:
      oligonucleotide primer

<400> 54
gtcctgactc gaggtctttg ggctctattg atg                                33


<210> 55
<211> 31
<212> DNA
<213> Artificial Sequence

<220>
<223> Description of Artificial Sequence:
      oligonucleotide primer

<400> 55
ggctacgcgc atatgctcac cctaggcttg g                                  31


<210> 56
<211> 34
<212> DNA
<213> Artificial Sequence

<220>
<223> Description of Artificial Sequence:
      oligonucleotide primer

<400> 56
gcgcggatcc tcacgctacg gagaggctaa ggag                               34


<210> 57
<211> 33
<212> DNA
<213> Artificial Sequence

<220>
<223> Description of Artificial Sequence:
      oligonucleotide primer

<400> 57
ctcggctgct gccatatggg gctgcaatcc agg                                33

<210> 58
<211> 42
<212> DNA
<213> Artificial Sequence

<220>
<223> Description of Artificial Sequence:
      oligonucleotide primer

<400> 58
gcccgctcga gctacttact taattcatct tcgtaaagaa tc                42

<210> 59
<211> 31
<212> DNA
<213> Artificial Sequence

<220>
<223> Description of Artificial Sequence:
      oligonucleotide primer

<400> 59
cgcgagaatt catatgggaa tcaatccttc g                            31

<210> 60
<211> 29
<212> DNA
<213> Artificial Sequence

<220>
<223> Description of Artificial Sequence:
      oligonucleotide primer

<400> 60
ggcgctcgag ttattcaaaa agatccccg                               29

<210> 61
<211> 33
<212> DNA
<213> Artificial Sequence

<220>
<223> Description of Artificial Sequence:
      oligonucleotide primer

<400> 61
ggaagcatgc catggcaata gctattgcaa ggg                                33

<210> 62
<211> 43
<212> DNA
<213> Artificial Sequence

<220>
<223> Description of Artificial Sequence:
      oligonucleotide primer

<400> 62
catttttggg gatccactta ccttagctaa ttatcgaaat gtc                      43

<210> 63
<211> 38
<212> DNA
<213> Artificial Sequence

<220>
<223> Description of Artificial Sequence:
      oligonucleotide primer

<400> 63
ctattaataa aatcatatga cagattctaa tcccctac                           38

<210> 64
<211> 39
<212> DNA
<213> Artificial Sequence

<220>
<223> Description of Artificial Sequence:
      oligonucleotide primer

<400> 64
gctttccgac atctcgaggg gagtcgttag atcgaaacg                          39

<210> 65
<211> 35
<212> DNA
<213> Artificial Sequence

<220>
<223> Description of Artificial Sequence:
      oligonucleotide primer

<400> 65
attttagagg tccatggtgc ggctcttatc tatac                                35

<210> 66
<211> 37
<212> DNA
<213> Artificial Sequence

<220>
<223> Description of Artificial Sequence:
      oligonucleotide primer

<400> 66
ctacgtagga ctcgagactc cctaaagatt tagattc                              37

<210> 67
<211> 39
<212> DNA
<213> Artificial Sequence

<220>
<223> Description of Artificial Sequence:
      oligonucleotide primer

<400> 67
gtattaaatg aaaccatatg gcaatggatt tcaacccag                            39

<210> 68
<211> 36
<212> DNA
<213> Artificial Sequence

<220>
<223> Description of Artificial Sequence:
      oligonucleotide primer

<400> 68
catagagtcc ctcgagtgaa gcgttgatta agaagc                               36

<210> 69

<211> 41
<212> DNA
<213> Artificial Sequence

<220>
<223> Description of Artificial Sequence:
      oligonucleotide primer

<400> 69
cattaacatt aaacatatgt tcgtaatgaa aaaacttgtc c                41


<210> 70
<211> 34
<212> DNA
<213> Artificial Sequence

<220>
<223> Description of Artificial Sequence:
      oligonucleotide primer

<400> 70
gagcaacagc tcgagttttc tacactgttg taat                        34


<210> 71
<211> 39
<212> DNA
<213> Artificial Sequence

<220>
<223> Description of Artificial Sequence:
      oligonucleotide primer

<400> 71
cttaagaggg acatatgtct aagatagttt ataaatttg                   39


<210> 72
<211> 36
<212> DNA
<213> Artificial Sequence

<220>
<223> Description of Artificial Sequence:
      oligonucleotide primer

<400> 72

gtaaatgcat gatgcggccg ccgtggcgac tatagc                                36

<210> 73
<211> 36
<212> DNA
<213> Artificial Sequence

<220>
<223> Description of Artificial Sequence:
      oligonucleotide primer

<400> 73
ctttaggtaa ttcatatgag aaaacttatt ttatgc                                36

<210> 74
<211> 37
<212> DNA
<213> Artificial Sequence

<220>
<223> Description of Artificial Sequence:
      oligonucleotide primer

<400> 74
gtaagcctaa actcgagaga acaacggagt tcttttg                               37

<210> 75
<211> 37
<212> DNA
<213> Artificial Sequence

<220>
<223> Description of Artificial Sequence:
      oligonucleotide primer

<400> 75
gcctccgtag ctcatatggt tatgggaacc tatatcg                               37

<210> 76
<211> 35
<212> DNA
<213> Artificial Sequence

<220>

<223> Description of Artificial Sequence:
      oligonucleotide primer

<400> 76
gcgcttcccc tcgagtacat ttgtattgat ttcag                                35

## Description

### FIELD OF THE INVENTION

The present invention is related to Chlamydia polypeptides and corresponding DNA fragments, which may be used to prevent, diagnose and/or treat Chlamydia infections.

### BACKGROUND OF THE INVENTION

Chlamydiae are obligate intracellular bacteria that cause a wide variety of diseases in man, animals and birds. Despite many differences (e.g. epidemiology, transmission routes, overall DNA homology, antigenic analysis), Chlamydiae share common biological and immunological features. The organism only grows into a specialized vacuole in the post-Golgi exocytic vesicular compartment of the eukaryotic cell. They undergo a distinct developmental cycle that alternates between an extracellular transmission cell, termed elementary body (EB) and an intracellular replicating cell, termed the reticulate body (RB). The organisms are capable of persisting in cells of an immune host presumably due to its evolved capabilities for immune evasion. Infection causes host cells to produce a variety of proinflammatory cytokines which likely contribute to disease pathogenesis (Rasmussen S.J., Eckmann L., Quayle A.J., Shen L., Zhang, Y.X., Anderson D.J., Fierer J., Stephens R.S. and Kagnoff M.F. (1997) Secretion of proinflammatory cytokines by epithelial cells in response to Chlamydia infection suggests a central role for epithelial cells in chlamydial pathogenesis. J. Clin. Invest. 99: 77-87).

Four chlamydial species are currently known: Chlamydia trachomatis, Chlamydia pneumoniae, Chlamydia pecorum and Chlamydia psittaci. C. trachomatis and C. pneumoniae are two Chlamydia species that are natural human pathogen. C. psittaci strains are highly diverse, having been isolated from human and a very large number of avian and mammalian species. They have been grouped into eight biovars which produce a broad spectrum of diseases (Storz J., (1988), Overview of Animal Diseases Induced by Chlamydial Infections, In «Microbiology of Chlamydia» Ed. AL. Baron, CRC Press Inc. Boka Raton, Florida, pp. 167-192). C. pecorum has been isolated only from mammals.

In general, all chlamydiae appear to share a common immunobiology characterized by acute infection followed by immunity or by persistent or repeated infection that is associated with tissue damage and disease sequelae. Genome analysis shows that over 80% of C. pneumoniae and C. trachomatis protein-coding genes are orthologs that share a similar organization (Kalman S.,Mitchell W., Marathe R., Lammel C., Fan J., Hyman R.W., Olinger L., Grinwood J., Davis R.W., Stephens R.S. (1999) Comparative genomes of Chlamydia pneumoniae and C. trachomatis. Nat Genet. 21: 385-389). C. trachomatis causes blinding trachoma and is a major cause of sexually transmitted infections. C. pneumoniae infects the mucosal surfaces of respiratory tract causing sinusitis, pharyngitis, bronchitis and pneumonitis. As approximately, 10% of community-acquired pneumonia and 5% of bronchitis and sinusitis cases are caused by C. pneumoniae (Grayston, J.T. (2000) Background and current knowledge of Chlamydia pneumoniae and atherosclerosis. J. Infect. Dis. 181(Suppl 3): S402-410), prevention of them would already be beneficial. All the more that infection has been associated with other respiratory tract diseases, such as asthmatic bronchitis, adult-onset asthma, and chronic obstructive pulmonary diseases (COPD). Furthermore, numerous sero-epidemiological and pathological studies suggest that C. pneumoniae may disseminate from the respiratory tract to produce vascular infection and contribute to atherogenesis. Although this association has not been proven to be causal, it is becoming recognized as potentially highly important. In fact, C. pneumoniae is capable to surviving and replicating remarkably inside human mononuclear cells, a cell type that usually kills invading microbes. This probably provides them with a way to disseminate from the respiratory tract to elsewhere in the body, including artery walls (Gaydos C.A., Summersgill J.T., Sahney N.N., Ramirez J.A. and Quinn T.C. (1996) Replication of Chlamydia pneumoniae *in vitro* in human macrophages, endothelial cells and aortic artery smooth muscle cells. Infect. Immun. 64: 1614-1620). Consequently, infections may indirectly accelerate or enhance atherosclerosis in the presence of other risk factors by several mechanisms or they may induce atherosclerosis through local infection, inflammation or autoimmune reaction (Fong I.W. (2000) Emerging relations between infectious diseases and coronary artery disease and atherosclerosis. CMAJ 163: 49-56).

From seroprevalence studies, C. pneumoniae infections usually happen between the ages of 5 and 20 years. In population, prevalence antibody rates reach 50% by age 20 and continue to rise in adults, reaching in old age about 80% in men and 70% in women (Wang S.P., and Grayston J.T. (1990) Population prevalence antibody to Chlamydia pneumoniae strain TWAR. In : Bowie W.R., Caldwell H.D., Jones R.P. *et al*., Eds. Chlamydial infections. Cambridge, UK : Cambridge University Press, pp 402-405; Wang S.P., and Grayston J.T. (1998) Chlamydia pneumoniae (TWAR) microimmunofluorescence studies - 1998 update. In: Stephens R.S., Byrne B.I., Christiansen G. *et al*., Eds. Chlamydial infections. Berkely, CA: University of california, pp 155-158).
Frequent reinfection and persistent chronic infection can best explain this prevalence. So, the great majority of the adult population has antibodies to C. pneumoniae, indicating past infection. But, protection conferred by natural chlamydial infection, is usually incomplete, transient (3-5 years)(Padnode D., Wang S.P. and Grayston J.T. (1990) Persistence of Chlamydia pneumoniae, strain TWAR micro-immunofluorescent antibody. In: Bowie W.R., Caldwell H.D., Jones R.P. *et al*., Eds. Chlamydial infections. Cambridge, UK: Cambridge University Press, pp 406-409) and strain specific. Until now, all tentatives have failed to result in an effective vaccine for human use. Therefore, there remains an unmet need for Chlamydia antigens that may be used as vaccine components for the prophylaxis and/or therapy of Chlamydia infection.

### SUMMARY OF THE INVENTION

According to one aspect, the present invention provides an isolated polynucleotide encoding a polypeptide having at least 70% identity to a second polypeptide comprising a sequence chosen from SEQ ID Nos: 2,4,6,8,10,12,14,16,18,20, 22,24,26,28,30,32,34,36 and 38 or fragments or analogs thereof.

According to one aspect, the present invention relates to polypeptides which comprise an amino acid sequence chosen from SEQ ID Nos: 2, 4, 6, 8, 10, 12, 14, 16, 18, 20, 22, 24, 26, 28, 30, 32, 34, 36 and 38 or fragments or analogs thereof.

In other aspects, there are provided polypeptides encoded by polynucleotides of the invention, pharmaceutical compositions, vectors comprising polynucleotides of the invention operably linked to an expression control region, as well as host cells transfected with said vectors and processes for producing polypeptides comprising culturing said host cells under conditions suitable for expression.

### BRIEF DESCRIPTION OF THE DRAWINGS

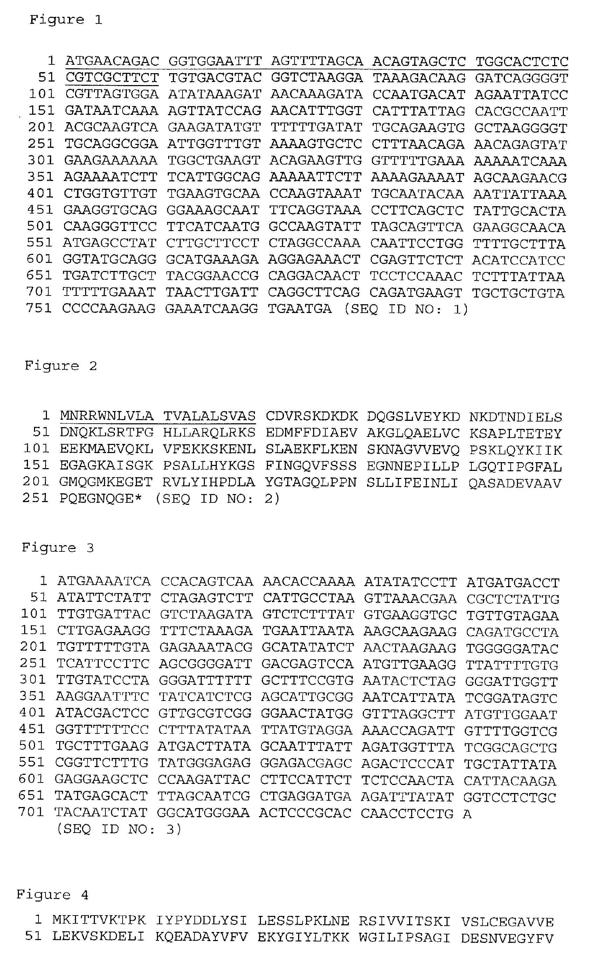

Figure 1 is the DNA sequence of BVH-CPN1 gene from C. pneumoniae strain CWL-029; SEQ ID NO: 1. The underlined portion of the sequence represents the region coding for the leader peptide.
Figure 2 is the deduced amino acid sequence of the full-length BVH-CPN1 from C. pneumoniae strain CWL-029; SEQ ID NO: 2. The underlined portion of the sequence represents the leader peptide.
Figure 3 is the DNA sequence of BVH-CPN2 gene from C. pneumoniae strain CWL-029; SEQ ID NO: 3.
Figure 4 is the deduced amino acid sequence of the full-length BVH-CPN2 from C. pneumoniae strain CWL-029; SEQ ID NO: 4.

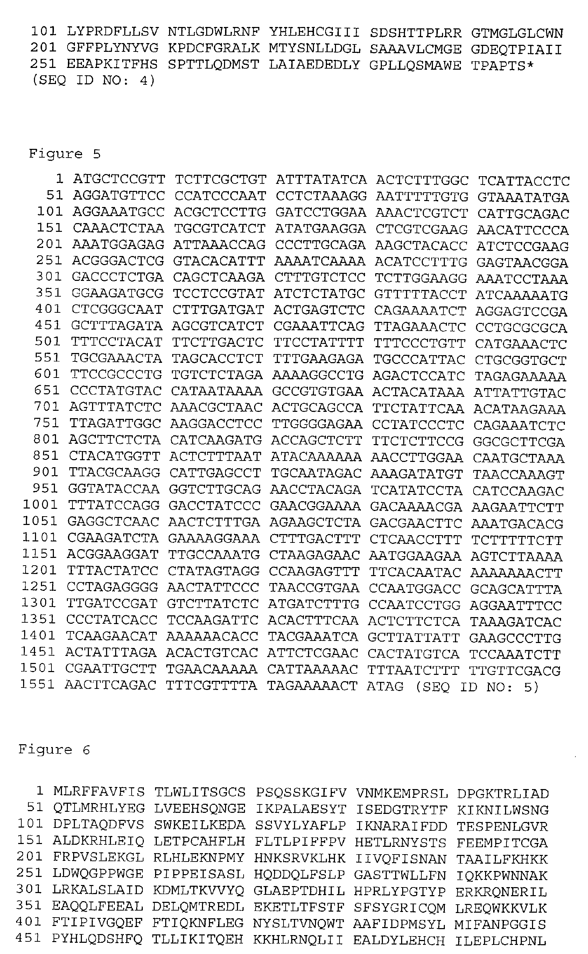

Figure 5 is the DNA sequence of BVH-CPN3 gene from C. pneumoniae strain CWL-029; SEQ ID NO: 5.
Figure 6 is the deduced amino acid sequence of the full-length BVH-CPN3 from C. pneumoniae strain CWL-029; SEQ ID NO: 6.

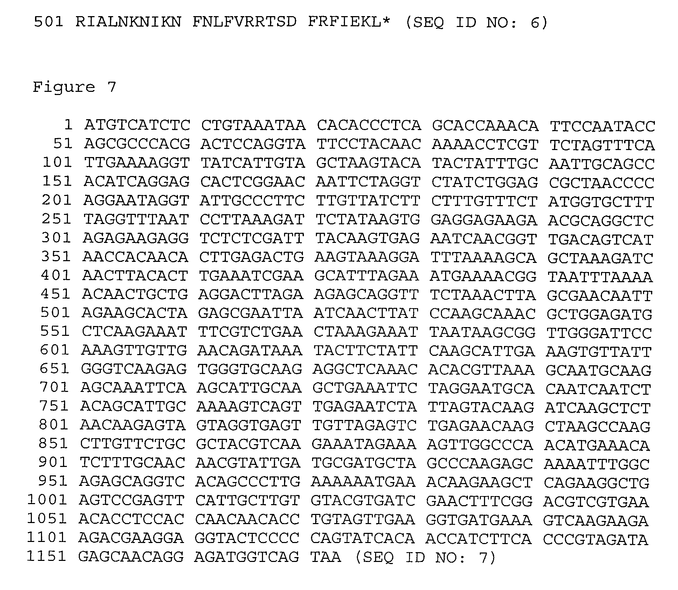

Figure 7 is the DNA sequence of BVH-CPN4 gene from C. pneumoniae strain CWL-029; SEQ ID NO: 7.

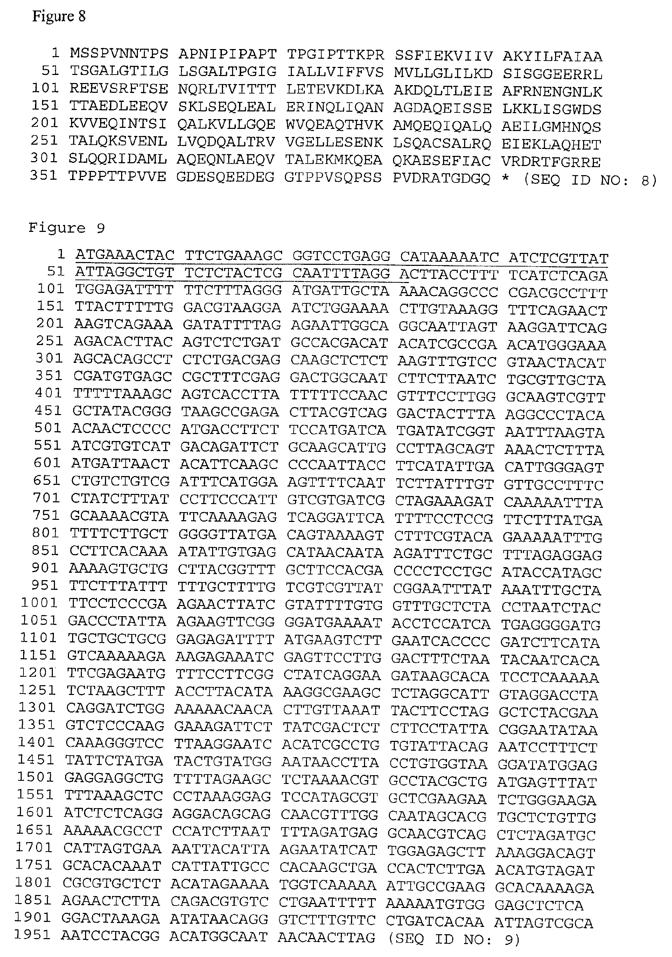

Figure 8 is the deduced amino acid sequence of the full-length BVH-CPN4 from C. pneumoniae strain CWL-029; SEQ ID NO: 8.
Figure 9 is the DNA sequence of BVH-CPN5 gene from C. pneumoniae strain CWL-029; SEQ ID NO: 9. The underlined portion of the sequence represents the region coding for the leader peptide.

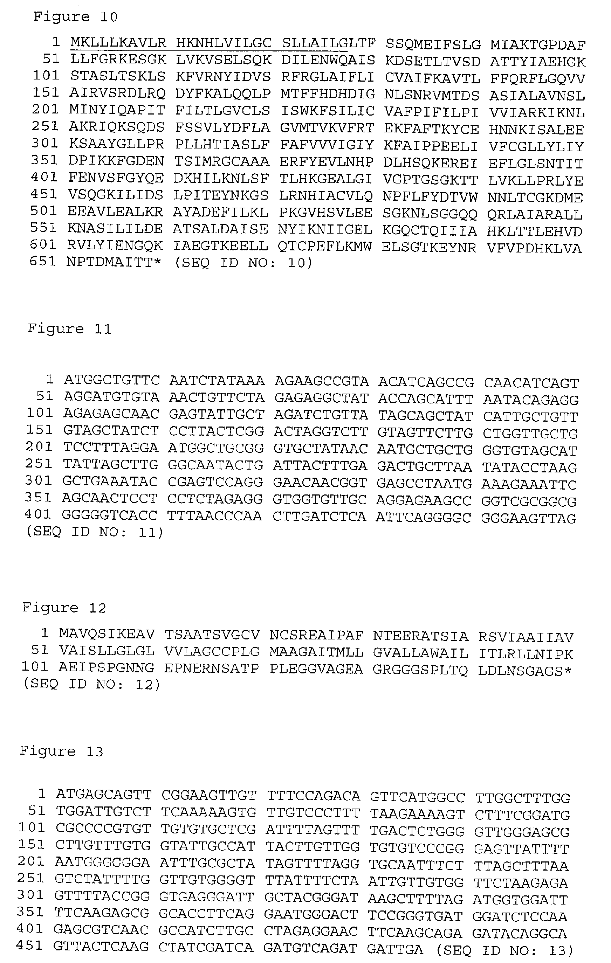

Figure 10 is the deduced amino acid sequence of the full-length BVH-CPN5 from C. pneumoniae strain CWL-029; SEQ ID NO: 10. The underlined portion of the sequence represents the leader peptide.
Figure 11 is the DNA sequence of BVH-CPN6 gene from C. pneumoniae strain CWL-029; SEQ ID NO: 11.
Figure 12 is the deduced amino acid sequence of the full-length BVH-CPN6 from C. pneumoniae strain CWL-029; SEQ ID NO: 12.

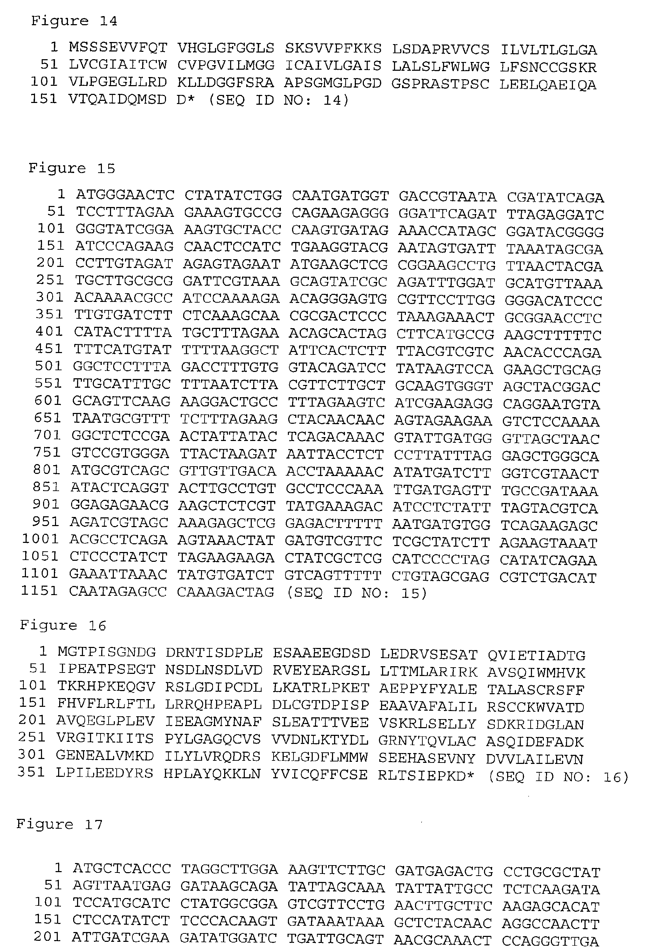

Figure 13 is the DNA sequence of BVH-CPN7 gene from C. pneumoniae strain CWL-029; SEQ ID NO: 13.
Figure 14 is the deduced amino acid sequence of the full-length BVH-CPN7 from C. pneumoniae strain CWL-029; SEQ ID NO: 14.
Figure 15 is the DNA sequence of BVH-CPN8 gene from C. pneumoniae strain CWL-029; SEQ ID NO: 15.
Figure 16 is the deduced amino acid sequence of the full-length BVH-CPN8 from C. pneumoniae strain CWL-029; SEQ ID NO: 16.
Figure 17 is the DNA sequence of BVH-CPN9 gene from C. pneumoniae strain CWL-029; SEQ ID NO: 17.

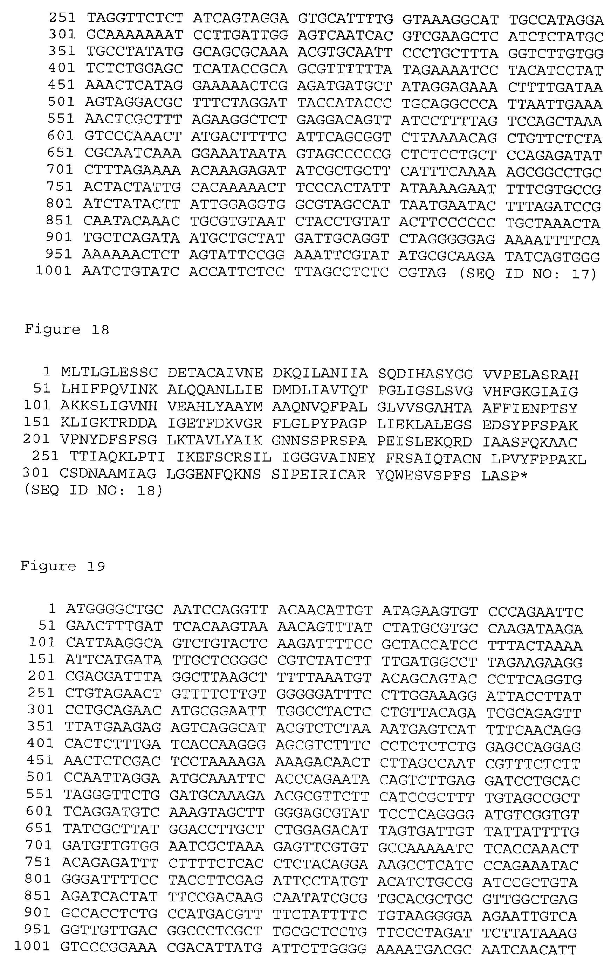

Figure 18 is the deduced amino acid sequence of the full-length BVH-CPN9 from C. pneumoniae strain CWL-029; SEQ ID NO: 18.
Figure 19 is the DNA sequence of BVH-CPN10 gene from C. pneumoniae strain CWL-029; SEQ ID NO: 19.

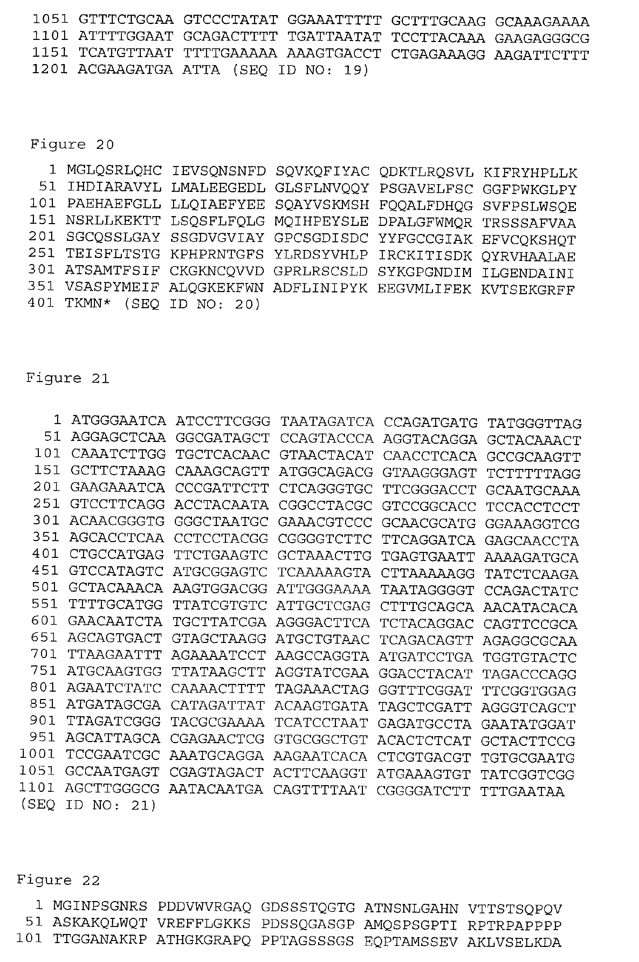

Figure 20 is the deduced amino acid sequence of the full-length BVH-CPN10 from C. pneumoniae strain CWL-029; SEQ ID NO: 20.
Figure 21 is the DNA sequence of BVH-CPN11 gene from C. pneumoniae strain CWL-029; SEQ ID NO: 21.
Figure 22 is the deduced amino acid sequence of the full-length BVH-CPN11 from C. pneumoniae strain CWL-029; SEQ ID NO: 22.

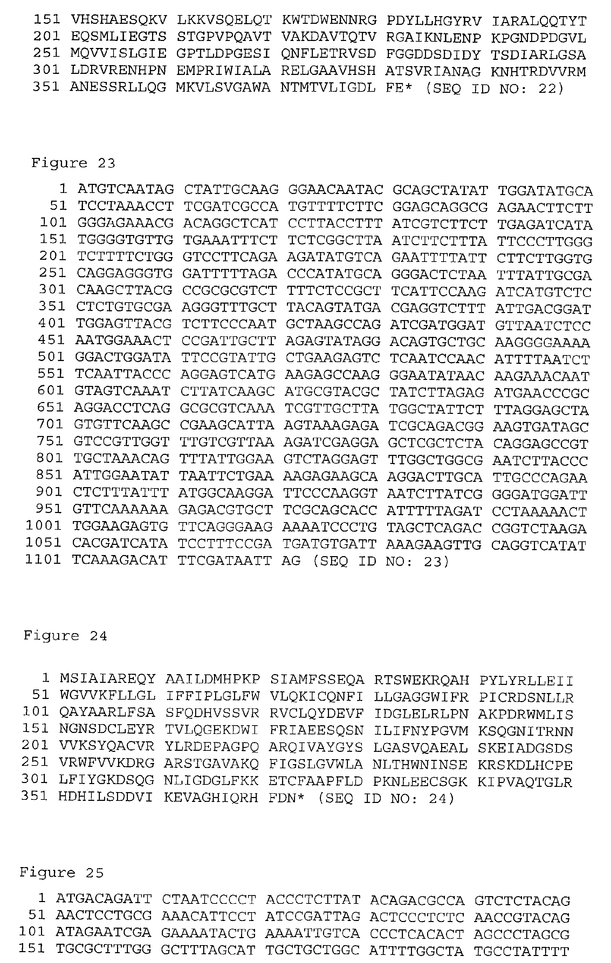

Figure 23 is the DNA sequence of BVH-CPN12 gene from C. pneumoniae strain CWL-029; SEQ ID NO: 23.
Figure 24 is the deduced amino acid sequence of the full-length BVH-CPN12 from C. pneumoniae strain CWL-029; SEQ ID NO: 24.
Figure 25 is the DNA sequence of BVH-CPN13 gene from C. pneumoniae strain CWL-029; SEQ ID NO: 25.

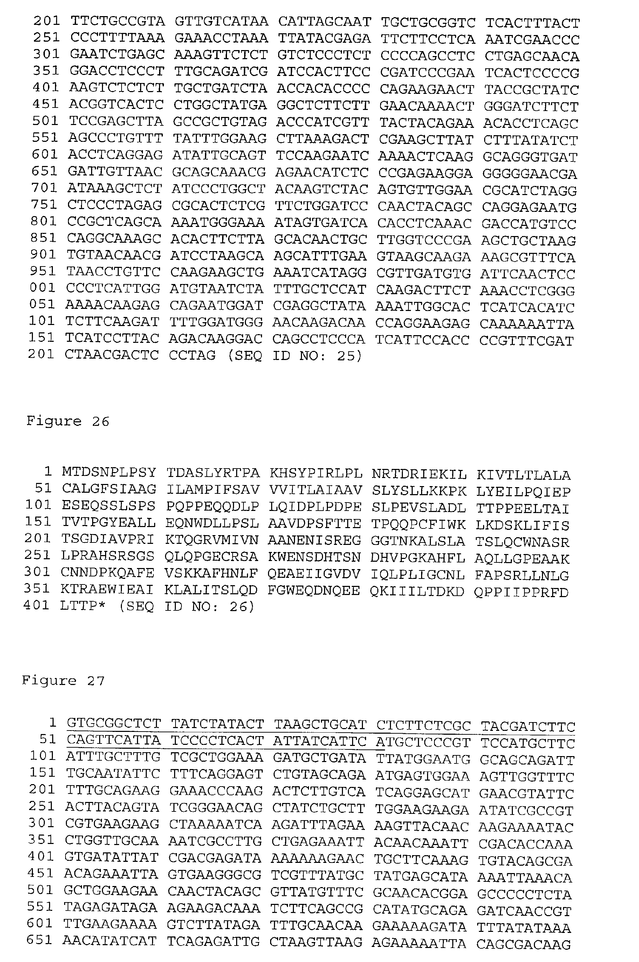

Figure 26 is the deduced amino acid sequence of the full-length BVH-CPN13 from C. pneumoniae strain CWL-029; SEQ ID NO: 26.
Figure 27 is the DNA sequence of BVH-CPN14 gene from C. pneumoniae strain CWL-029; SEQ ID NO: 27. The underlined portion of the sequence represents the region coding for the leader peptide.

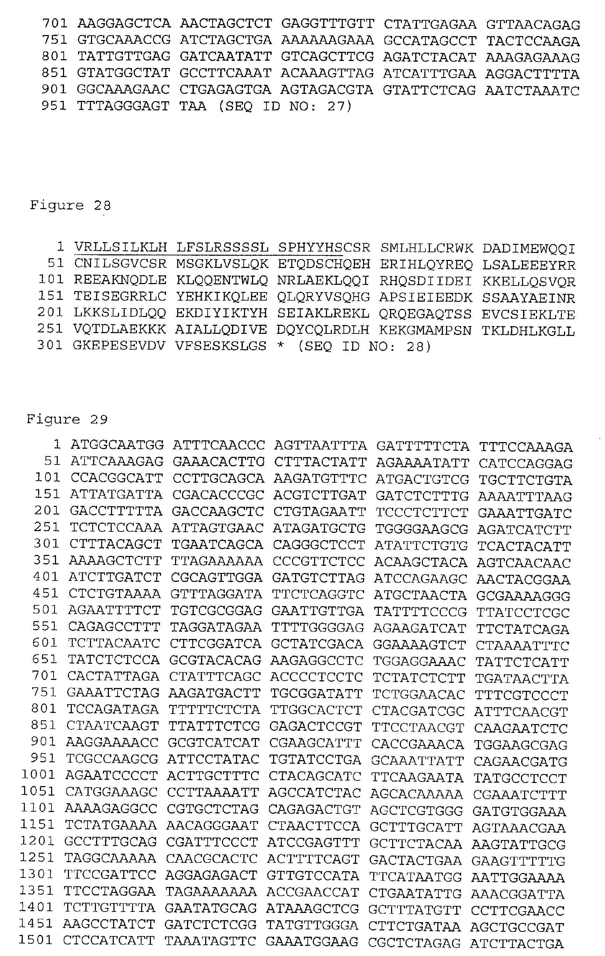

Figure 28 is the deduced amino acid sequence of the full-length BVH-CPN14 from C. pneumoniae strain CWL-029; SEQ ID NO: 28. The underlined portion of the sequence represents the leader peptide.
Figure 29 is the DNA sequence of BVH-CPN15 gene from C. pneumoniae strain CWL-029; SEQ ID NO: 29.

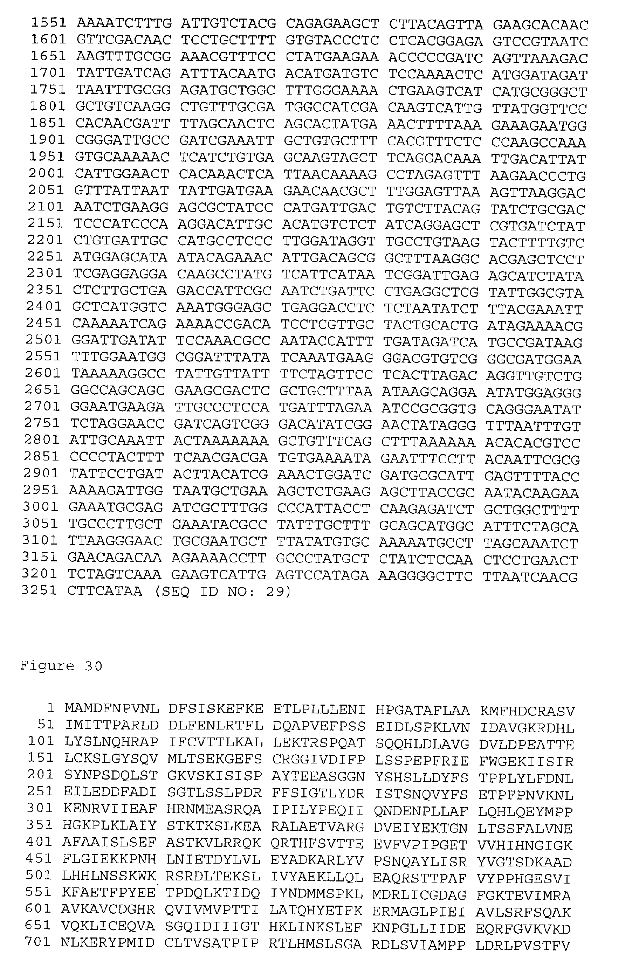

Figure 30 is the deduced amino acid sequence of the full-length BVH-CPN15 from C. pneumoniae strain CWL-029; SEQ ID NO: 30.

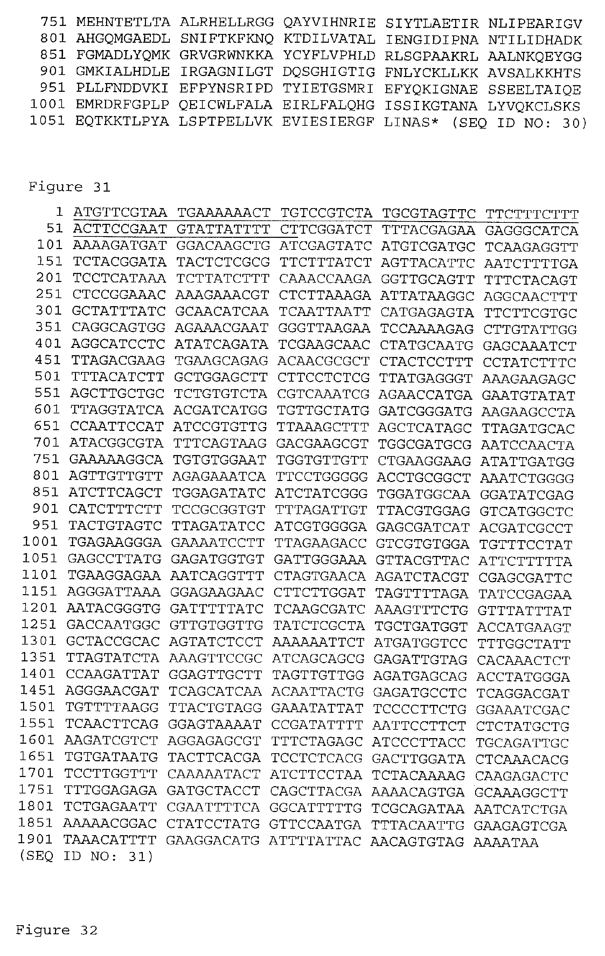

Figure 31 is the DNA sequence of BVH-CPN16 gene from C. pneumoniae strain CWL-029; SEQ ID NO: 31. The underlined portion of the sequence represents the region coding for the leader peptide.
Figure 32 is the deduced amino acid sequence of the full-length BVH-CPN16 from C. pneumoniae strain CWL-029; SEQ ID NO: 32. The underlined portion of the sequence represents the leader peptide.

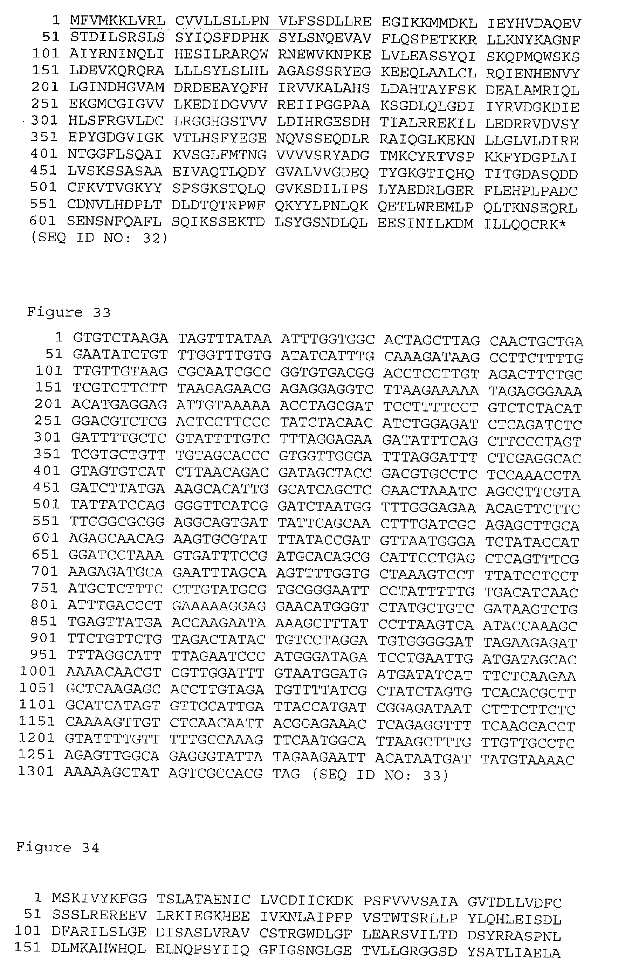

Figure 33 is the DNA sequence of BVH-CPN17 gene from C. pneumoniae strain CWL-029; SEQ ID NO: 33.
Figure 34 is the deduced amino acid sequence of the full-length BVH-CPN17 from C. pneumoniae strain CWL-029; SEQ ID NO: 34.

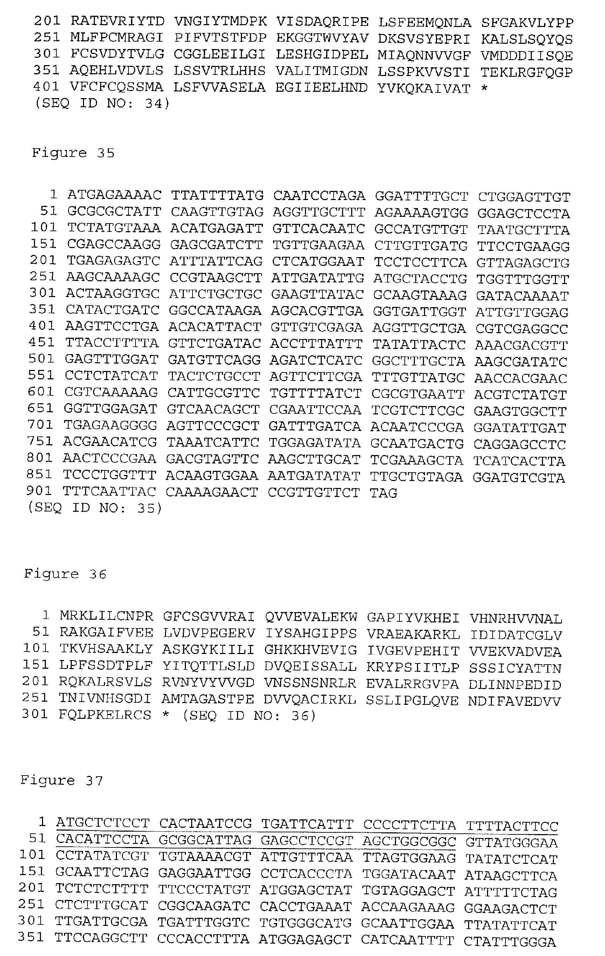

Figure 35 is the DNA sequence of BVH-CPN18 gene from C. pneumoniae strain CWL-029; SEQ ID NO: 35.
Figure 36 is the deduced amino acid sequence of the full-length BVH-CPN18 from C. pneumoniae strain CWL-029; SEQ ID NO: 36.
Figure 37 is the DNA sequence of BVH-CPN19 gene from C. pneumoniae strain CWL-029; SEQ ID NO: 37. The underlined portion of the sequence represents the region coding for the leader peptide.

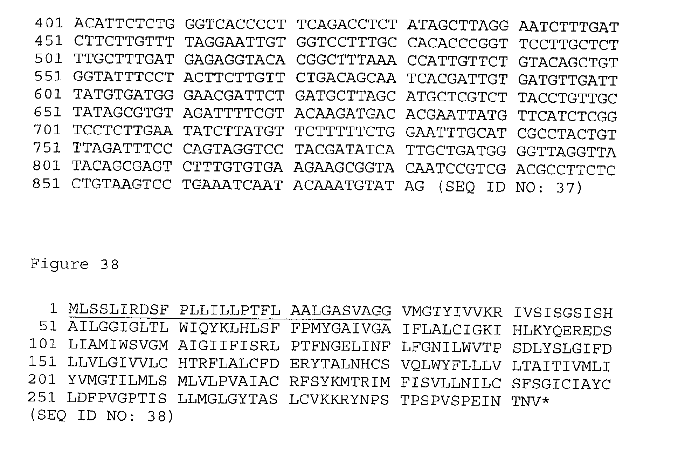

Figure 38 is the deduced amino acid sequence of the full-length BVH-CPN19 from C. pneumoniae strain CWL-029; SEQ ID NO: 38. The underlined portion of the sequence represents the leader peptide.
Figure 39 having Panels A to C, shows the anti-BVH-CPN1 immune response following intranasal immunization of BVH-CPN1 polypeptide in presence of the choleric toxin (CT; 1 µg) (A), the E. coli labile toxin (LT; 1 µg) (B) and subcutaneous immunization in presence of QuilA (15 µg) (C).
Figure 40 shows the protection of the BVH-CPN1 polypeptide in the mouse model of pulmonary infection following intranasal immunization with 20 µg of BVH-CPN1 in presence of choleric toxin (CT; 1 µg) and the E. coli heat labile toxin (LT; 1 µg) and subcutaneous immunization with 20 µg of BVH-CPN1 in presence of QuilA (15 µg). Data corresponding to animals immunized with adjuvant only were compared to those immunized with the antigen in combination with the adjuvant according to the unpaired t test with Welch's correction.

### DETAILED DESCRIPTION OF THE INVENTION

The present invention provides purified and isolated DNA molecules, which encode Chlamydial polypeptides that can be used to prevent, treat, and/or diagnose Chlamydial infection.

According to one aspect, the present invention provides an isolated polynucleotide encoding a polypeptide having at least 70% identity to a second polypeptide comprising a sequence chosen from SEQ ID Nos: 2, 4, 6, 8, 10, 12, 14, 16, 18, 20, 22, 24, 26, 28, 30, 32, 34, 36 and 38 or fragments or analogs thereof.

According to one aspect, the present invention provides an isolated polynucleotide encoding a polypeptide having at least 80% identity to a second polypeptide comprising a sequence chosen from SEQ ID Nos: 2, 4, 6, 8, 10, 12, 14, 16, 18, 20, 22, 24, 26, 28, 30, 32, 34, 36 and 38 or fragments or analogs thereof.

According to one aspect, the present invention provides an isolated polynucleotide encoding a polypeptide having at least 90% identity to a second polypeptide comprising a sequence chosen from SEQ ID Nos: 2, 4, 6, 8, 10, 12, 14, 16, 18, 20, 22, 24, 26, 28, 30, 32, 34, 36 and 38 or fragments or analogs thereof.

According to one aspect, the present invention provides an isolated polynucleotide encoding a polypeptide having at least 95% identity to a second polypeptide comprising a sequence chosen from SEQ ID Nos: 2, 4, 6, 8, 10, 12, 14, 16, 18, 20, 22, 24, 26, 28, 30, 32, 34, 36 and 38 or fragments or analogs thereof.

According to one aspect, the present invention provides a polynucleotide encoding a polypeptide comprising a sequence chosen from: SEQ ID NOs: 2, 4, 6, 8, 10, 12, 14, 16, 18, 20, 22, 24, 26, 28, 30, 32, 34, 36 and 38 or fragments or analogs thereof.

According to one aspect, the present invention provides a polynucleotide encoding a polypeptide capable of generating antibodies having binding specificity for a polypeptide having a sequence chosen from SEQ ID NOs: 2, 4, 6, 8, 10, 12, 14, 16, 18, 20, 22, 24, 26, 28, 30, 32, 34, 36 and 38 or fragments or analogs thereof.

According to one aspect, the present invention relates to polynucleotides encoding an epitope bearing portion of a polypeptide having a sequence chosen from SEQ ID NOS: 2, 4, 6, 8, 10, 12, 14, 16, 18, 20, 22, 24, 26, 28, 30, 32, 34, 36 and 38 or fragments or analogs thereof.

According to one aspect, the present invention relates to epitope bearing portions of a polypeptide having a sequence chosen from SEQ ID NOS: 2, 4, 6, 8, 10, 12, 14, 16, 18, 20, 22, 24, 26, 28, 30, 32, 34, 36 and 38 or fragments or analogs thereof.

According to one aspect, the present invention provides an isolated polynucleotide encoding a polypeptide having at least 70% identity to a second polypeptide comprising a sequence chosen from SEQ ID Nos: 2, 4, 6, 8, 10, 12, 14, 16, 18, 20, 22, 24, 26, 28, 30, 32, 34, 36 and 38.

According to one aspect, the present invention provides an isolated polynucleotide encoding a polypeptide having at least 80% identity to a second polypeptide comprising a sequence chosen from SEQ ID Nos: 2, 4, 6, 8, 10, 12, 14, 16, 18, 20, 22, 24, 26, 28, 30, 32, 34, 36 and 38.

According to one aspect, the present invention provides an isolated polynucleotide encoding a polypeptide having at least 90% identity to a second polypeptide comprising a sequence chosen from SEQ ID Nos: 2, 4, 6, 8, 10, 12, 14, 16, 18, 20, 22, 24, 26, 28, 30, 32, 34, 36 and 38.

According to one aspect, the present invention provides an isolated polynucleotide encoding a polypeptide having at least 95% identity to a second polypeptide comprising a sequence chosen from SEQ ID Nos: 2, 4, 6, 8, 10, 12, 14, 16, 18, 20, 22, 24, 26, 28, 30, 32, 34, 36 and 38.

According to one aspect, the present invention provides a polynucleotide encoding a polypeptide capable of generating antibodies having binding specificity for a polypeptide having a sequence chosen from SEQ ID NOs: 2, 4, 6, 8, 10, 12, 14, 16, 18, 20, 22, 24, 26, 28, 30, 32, 34, 36 and 38.

According to one aspect, the present invention provides a polynucleotide encoding an epitope bearing portion of a polypeptide having a sequence chosen from: SEQ ID NOs: 2, 4, 6, 8, 10, 12, 14, 16, 18, 20, 22, 24, 26, 28, 30, 32, 34, 36 and 38.

According to one aspect, the present invention relates to polynucleotides encoding an epitope bearing portion of a polypeptide having a sequence chosen from SEQ ID NOS: 2, 4, 6, 8, 10, 12, 14, 16, 18, 20, 22, 24, 26, 28, 30, 32, 34, 36 and 38.

According to one aspect, the present invention relates to epitope bearing portions of a polypeptide having a sequence chosen from SEQ ID NOS: 2, 4, 6, 8, 10, 12, 14, 16, 18, 20, 22, 24, 26, 28, 30, 32, 34, 36 and 38.

In accordance with the present invention, all polynucleotides encoding polypeptides are within the scope of the present invention.

Those skilled in the art will appreciate that the invention includes DNA molecules that encode analogs such as mutants, variants, homologues and derivatives of such polypeptides, as described herein in the present patent application. The invention also includes RNA molecules corresponding to the DNA molecules of the invention. In addition to the DNA and RNA molecules, the invention includes the corresponding polypeptides and monospecific antibodies that specifically bind to such polypeptides.

According to one aspect, the present invention relates to polypeptides having at least 70% identity to a second polypeptide having an amino acid sequence chosen from: SEQ ID NOs: 2, 4, 6, 8, 10, 12, 14, 16, 18, 20, 22, 24, 26, 28, 30, 32, 34, 36,38 or fragments or analogs thereof.

According to one aspect, the present invention relates to polypeptides having at least 95% identity to a second polypeptide having an amino acid sequence chosen from: SEQ ID NOs: 2, 4, 6, 8, 10, 12, 14, 16, 18, 20, 22, 24, 26, 28, 30, 32, 34, 36, 38 or fragments or analogs thereof.

According to one aspect, the present invention relates to polypeptides characterized by the amino acid sequence comprising sequences from SEQ ID Nos: 2, 4, 6, 8, 10, 12, 14, 16, 18, 20, 22, 24, 26, 28, 30, 32, 34, 36, 38 or fragments or analogs thereof.

According to one aspect, the present invention relates to polypeptides capable of generating antibodies having binding specificity for a polypeptide having a sequence chosen from SEQ ID NOs: 2, 4, 6, 8, 10, 12, 14, 16, 18, 20, 22, 24, 26, 28, 30, 32, 34, 36, 38 or fragments or analogs thereof.

According to one aspect, the present invention relates to epitope bearing portions of a polypeptide having a sequence chosen from SEQ ID NOs: 2, 4, 6, 8, 10, 12, 14, 16, 18, 20, 22, 24, 26, 28, 30, 32, 34, 36, 38 or fragments or analogs thereof.

According to one aspect, the present invention relates to polypeptides having at least 70% identity to a second polypeptide having an amino acid sequence chosen from: SEQ ID NOs: 2, 4, 6, 8, 10, 12, 14, 16, 18, 20, 22, 24, 26, 28, 30, 32, 34, 36 or 38.

According to one aspect, the present invention relates to polypeptides having at least 95% identity to a second polypeptide having an amino acid sequence chosen from: SEQ ID NOs: 2, 4, 6, 8, 10, 12, 14, 16, 18, 20, 22, 24, 26, 28, 30, 32, 34, 36 or 38.

According to one aspect, the present invention relates to polypeptides characterized by the amino acid sequence comprising sequences from SEQ ID Nos: 2,4,6,8,10,12,14,16,18,20,22,24,26, 28,30,32,34,36 or 38.

According to one aspect, the present invention relates to polypeptides capable of generating antibodies having binding specificity for a polypeptide having a sequence chosen from SEQ ID NOs: 2, 4, 6, 8, 10, 12, 14, 16, 18, 20, 22, 24, 26, 28, 30, 32, 34, 36 or 38.

According to one aspect, the present invention relates to epitope bearing portions of a polypeptide having a sequence chosen from SEQ ID NOs: 2, 4, 6, 8, 10, 12, 14, 16, 18, 20, 22, 24, 26, 28, 30, 32, 34, 36, 38 or fragments or analogs thereof.

In a further embodiment, the polypeptides in accordance with the present invention are antigenic.

In a further embodiment, the polypeptides in accordance with the present invention are immunogenic.

In a further embodiment, the polypeptides in accordance with the present invention can elicit an immune response in a host.

In a further embodiment, the present invention also relates to polypeptides which are able to raise antibodies having binding specificity to the polypeptides of the present invention as defined above.

An antibody that « has binding specificity» is an antibody that recognizes and binds the selected polypeptide but which does not substantially recognize and bind other molecules in a sample, e.g., a biological sample. Specific binding can be measured using an ELISA assay in which the selected polypeptide is used as an antigen.

In accordance with the present invention, "protection" in the biological studies is defined by a significant increase in the survival curve, rate or period. Statistical analysis using the Log rank test to compare survival curves, and Fisher exact test to compare survival rates and numbers of days to death, respectively, might be useful to calculate P values and determine whether the difference between the two groups is statistically significant. P values of 0.05 are regarded as not significant.

In an additional aspect of the invention there are provided antigenic/immunogenic fragments of the polypeptides of the invention, or of analogs thereof.

The fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a polypeptide or analog thereof as described herein. The present invention further provides fragments having at least 10 contiguous amino acid residues from the polypeptide sequences of the present invention. In one embodiment, at least 15 contiguous amino acid residues. In one embodiment, at least 20 contiguous amino acid residues.

The skilled person will appreciate that analogs of the polypeptides of the invention will also find use in the context of the present invention, i.e. as antigenic/immunogenic material. Thus, for instance polypeptides which include one or more additions, deletions, substitutions or the like are encompassed by the present invention.

As used herein, "fragments", "analogs" or "derivatives" of the polypeptides of the invention include those polypeptides in which one or more of the amino acid residues are substituted with a conserved or non-conserved amino acid residue (preferably conserved) and which may be natural or unnatural. In one embodiment, derivatives and analogs of polypeptides of the invention will have about 70% identity with those sequences illustrated in the figures or fragments thereof. That is, 70% of the residues are the same. In a further embodiment, polypeptides will have greater than 80% identity. In a further embodiment, polypeptides will have greater than 85% identity. In a further embodiment, polypeptides will have greater than 90% identity. In a further embodiment, polypeptides will have greater than 95% identity. In a further embodiment, polypeptides will have greater than 99% identity. In a further embodiment, analogs of polypeptides of the invention will have fewer than about 20 amino acid residue substitutions, modifications or deletions and more preferably less than 10.

In a further embodiment, polypeptides will have greater than 70% homology. In a further embodiment, polypeptides will have greater than 75% homology. In a further embodiment, polypeptides will have greater than 80% homology. In a further embodiment, polypeptides will have greater than 85% homology. In a further embodiment, polypeptides will have greater than 90% homology. In a further embodiment, polypeptides will have greater than 95% homology. In a further embodiment, polypeptides will have greater than 99% homology. In a further embodiment, derivatives and analogs of polypeptides of the invention will have less than about 20 amino acid residue substitutions, modifications or deletions and more preferably less than 10. Preferred substitutions are those known in the art as conserved i.e. the substituted residues share physical or chemical properties such as hydrophobicity, size, charge or functional groups.

These substitutions are those having a minimal influence on the secondary structure and hydropathic nature of the polypeptide. Preferred substitutions are those known in the art as conserved, i.e. the substituted residues share physical or chemical properties such as hydrophobicity, size, charge or functional groups. These include substitutions such as those described by Dayhoff, M. in Atlas of Protein Sequence and Structure 5, 1978 and by Argos, P. in EMBO J. 8, 779-785, 1989. For example, amino acids, either natural or unnatural, belonging to one of the following groups represent conservative changes:
- ala, pro, gly, gln, asn, ser, thr, val;
- cys, ser, tyr, thr;
- val, ile, leu, met, ala, phe;
- lys, arg, orn, his;
- and phe, tyr, trp, his.

The preferred substitutions also include substitutions of D-enantiomers for the corresponding L-amino acids.

The percentage of homology is defined as the sum of the percentage of identity plus the percentage of similarity or conservation of amino acid type.

In one embodiment, analogs of polypeptides of the invention will have about 70% identity with those sequences illustrated in the figures or fragments thereof. That is, 70% of the residues are the same. In a further embodiment, polypeptides will have greater than 80% homology. In a further embodiment, polypeptides will have greater than 90% homology. In a further embodiment, polypeptides will have greater than 95% homology. In a further embodiment, polypeptides will have greater than 99% homology. In a further embodiment, analogs of polypeptides of the invention will have less than about 20 amino acid residue substitutions, modifications or deletions and more preferably less than 10.

One can use a program such as the CLUSTAL program to compare amino acid sequences. This program compares amino acid sequences and finds the optimal alignment by inserting spaces in either sequence as appropriate. It is possible to calculate amino acid identity or similarity (identity plus conservation of amino acid type) for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In an alternative approach, the analogs could be fusion polypeptides, incorporating moieties which render purification easier, for example by effectively tagging the desired polypeptide. It may be necessary to remove the "tag" or it may be the case that the fusion polypeptide itself retains sufficient antigenicity to be useful.

Thus, what is important for analogs, derivatives and fragments is that they possess at least a degree of the antigenicity/immunogenic of the protein or polypeptide from which they are derived.

Also included are polypeptides which have fused thereto other compounds which alter the biological or pharmacological properties of the polypeptide, i.e., polyethylene glycol (PEG) to increase half-life, leader or secretory amino acid sequences for ease of purification, prepro- and pro- sequences and polysaccharides.

Furthermore, in those situations where amino acid regions are found to be polymorphic, it may be desirable to vary one or more particular amino acids to more effectively mimic the different epitopes of the different chlamydial strains.

Moreover, the polypeptides of the present invention can be modified by terminal -NH₂ acylation (e.g. by acetylation or thioglycolic acid amidation, terminal carboxy amidation, e.g. with ammonia or methylamine) to provide stability, increased hydrophobicity for linking or binding to a support or other molecule.

Also contemplated are hetero and homo polypeptide multimers of the polypeptide fragments and analogs. These polymeric forms include, for example, one or more polypeptides that have been cross-linked with cross-linkers such as avidin/biotin, glutaraldehyde or dimethylsuperimidate. Such polymeric forms also include polypeptides containing two or more tandem or inverted contiguous sequences, produced from multicistronic mRNAs generated by recombinant DNA technology. In a further embodiment, the present invention also relates to chimeric polypeptides which comprise one or more polypeptides or fragments, analogs or derivatives thereof as defined in the figures of the present application.

In a further embodiment, the present invention also relates to chimeric polypeptides comprising two or more polypeptides having a sequence chosen from SEQ ID NOs: 2, 4, 6, 8, 10, 12, 14, 16, 18, 20, 22, 24, 26, 28, 30, 32, 34, 36, 38 or fragments or analogs thereof; provided that the polypeptides are linked as to formed a chimeric polypeptide.

In a further embodiment, the present invention also relates to chimeric polypeptides comprising two or more polypeptides having a sequence chosen from SEQ ID NOs: 2, 4, 6, 8, 10, 12, 14, 16, 18, 20, 22, 24, 26, 28, 30, 32, 34, 36 and 38, provided that the polypeptides are linked as to formed a chimeric polypeptide.

Preferably, a fragment, analog or derivative of a polypeptide of the invention will comprise at least one antigenic region i.e. at least one epitope.

In order to achieve the formation of antigenic polymers (i.e. synthetic multimers), polypeptides may be utilized having bishaloacetyl groups, nitroarylhalides, or the like, where the reagents being specific for thio groups. Therefore, the link between two mercapto groups of the different peptides may be a single bound or may be composed of a linking group of at least two, typically at least four and not more than 16, but usually not more than about 14 carbon atoms.

In a particular embodiment, polypeptide fragments or analogs of the invention do not contain a starting residue, such as methionine (Met) or Valine (Val).

Preferably, polypeptides will not incorporate a leader or secretory sequence (signal sequence). The signal portion of a polypeptide of the invention may be determined according to established molecular biological techniques. The polypeptide of interest may be isolated from Chlamydial culture and subsequently sequenced to determine the initial residue of the mature protein and therefore the sequence of the mature polypeptide.

It is understood that polypeptides can be produced and/or used without their start codon (methionine or valine) and/or without their leader peptide to favor production and purification of recombinant polypeptides. It is known that cloning genes without sequences encoding leader peptides will restrict the polypeptides to the cytoplasm of E. coli and will facilitate their recovery (Glick, B.R. and Pasternak, J.J. (1998) Manipulation of gene expression in prokaryotes. In "Molecular biotechnology: Principles and applications of recombinant DNA", 2nd edition, ASM Press, Washington DC, p.109-143).

The polypeptides may be expressed with or without a leader or secretion sequence. In the former case, the leader may be removed using post-translational processing (see US 4 431 739, US 4 425 437 and US 4 338 397 incorporated herein by reference) or be chemically removed subsequent to purifying the expressed polypeptide.

According to another aspect of the invention, there are also provided (i) a composition of matter containing a polypeptide of the invention, together with a carrier, diluent or adjuvant; (ii) a pharmaceutical composition comprising a polypeptide of the invention and a carrier, diluent or adjuvant; (iii) a vaccine comprising a polypeptide of the invention and a carrier, diluent or adjuvant; (iv) a method for inducing an immune response against Chlamydia, in a host, by administering to the host, an immunogenically effective amount of a polypeptide of the invention to elicit an immune response, e.g., a protective immune response to Chlamydia (e.g. C.pneumoniae, C.trachomatis, C.psitacci or C.pecorum); and particularly, (v) a method for preventing and/or treating a Chlamydia (e.g. C.pneumoniae, C.trachomatis, C.psitacci or C.pecorum) infection, by administering a prophylactic or therapeutic amount of a polypeptide of the invention to a host in need.

According to another aspect of the invention, there are also provided (i) a composition of matter containing a polynucleotide of the invention, together with a carrier, diluent or adjuvant; (ii) a pharmaceutical composition comprising a polynucleotide of the invention and a carrier, diluent or adjuvant; (iii) a method for inducing an immune response against Chlamydia, in a host, by administering to the host, an immunogenically effective amount of a polynucleotide of the invention to elicit an immune response, e.g., a protective immune response to Chlamydia (e.g. C.pneumoniae, C.trachomatis, C.psitacci or C.pecorum); and particularly, (iv) a method for preventing and/or treating a Chlamydia (e.g. C.pneumoniae, C.trachomatis, C.psitacci or C.pecorum) infection, by administering a prophylactic or therapeutic amount of a polynucleotide of the invention to a host in need.

Before immunization, the polypeptides of the invention can also be coupled or conjugated to carrier proteins such as tetanus toxin, diphtheria toxin, hepatitis B virus surface antigen, poliomyelitis virus VP1 antigen or any other viral or bacterial toxin or antigen or any suitable proteins to stimulate the development of a stronger immune response. This coupling or conjugation can be done chemically or genetically. A more detailed description of peptide-carrier conjugation is available in Van Regenmortel, M.H.V., Briand J.P., Muller S., Plaué S., «Synthetic Polypeptides as antigens» in Laboratory Techniques in Biochemistry and Molecular Biology, Vol.19 (ed.) Burdou, R.H. & Van Knippenberg P.H. (1988), Elsevier New York.

According to another aspect, there are provided pharmaceutical compositions comprising one or more Chlamydial polypeptides of the invention in a mixture with a pharmaceutically acceptable carrier, diluent or adjuvant. Suitable adjuvants include (1) oil-in-water emulsion formulations such as MF59™, SAF™, Ribi™ ; (2) Freund's complete or incomplete adjuvant; (3) salts i.e. AlK(SO₄)₂, AlNa(SO₄)₂, AlNH₄(SO₄)₂, Al(OH)₃, AlPO₄, silica, kaolin; (4) saponin derivatives such as Stimulon™ or particles generated therefrom such as ISCOMs (immunostimulating complexes); (5) cytokines such as interleukins, interferons, macrophage colony stimulating factor (M-CSF), tumor necrosis factor (TNF); (6) other substances such as carbon polynucleotides i.e. poly IC and poly AU, detoxified cholera toxin (CTB)and E.coli heat labile toxin for induction of mucosal immunity. A more detailed description of adjuvant is available in a review by M.Z.I Khan et al. in Pharmaceutical Research, vol.11, No.1 (1994) pp2-11, and also in another review by Gupta et al., in Vaccine, Vol.13, Nol4, pp. 1263-1276 (1995) and in WO 99/24578, which are herein incorporated by reference. Preferred adjuvants include QuilA™, QS21™, Alhydrogel™ and Adjuphos™.

In a further embodiment, there is provided a method of manufacturing a pharmaceutical composition comprising admixing a polypeptide of the invention with a pharmaceutically acceptable diluent, excipient or adjuvant.

In a further aspect, the invention provides a method for prophylactic or therapeutic treatment of Chlamydial bacterial infection in a host susceptible to Chlamydiae infection comprising administering to a host a therapeutic or prophylactic amount of a composition of the invention.

In a further aspect, the invention provides a method for prophylactic or therapeutic treatment of Chlamydial bacterial infection wherein said infection causes sinusitis, pharyngitis, bronchitis, pneumonitis, asthmatic bronchitis adult-onset asthma, chronic obstructive pulmonary diseases (CDPD), atherogenesis or atherosclerosis.

Pharmaceutical compositions of the invention may be used for the treatment or prophylaxis of chlamydial infection and/or diseases and symptoms mediated by chlamydial infection, in particular Chlamydia trachomatis , Chlamydia pneumoniae, Chlamydia pecorum or Chlamydia psittaci.

In one embodiment, pharmaceutical compositions of the invention are used for the treatment or prophylaxis of Chlamydia pneumoniae infection and/or diseases and symptoms mediated by Chlamydia pneumoniae infection.

In one embodiment, pharmaceutical compositions of the invention are used for the treatment or prophylaxis of Chlamydia psittaci infections and/or diseases and symptoms mediated by Chlamydia psittaci infection.

In one embodiment, pharmaceutical compositions of the invention are used for the treatment or prophylaxis of Chlamydia trachomatis infection and/or diseases and symptoms mediated by Chlamydia trachomatis infection.

Pharmaceutical compositions of the invention may be administered parenterally by injection, rapid infusion, nasopharyngeal adsorption, dermoadsorption, bucal or oral.

In a particular embodiment, pharmaceutical compositions are administered to those hosts at risk of Chlamydia infection such as infants, elderly and immunocompromised hosts.

In a further aspect, the invention provides the use of pharmaceutical composition of the invention for the prophylactic or therapeutic treatment of Chlamydia bacterial infection in a host susceptible to Chlamydia infection comprising administering to said host a therapeutic or prophylactic amount of a composition of the invention.

According to a further aspect, the Chlamydia polypeptides of the invention may be used in a kit comprising the polypeptides of the invention for detection or diagnosis of Chlamydia infection.

As used in the present application, the term « hosts » include animals. In a further embodiment, the animal is a mammal or a bird. In a further embodiment, the mammal is a human.

Pharmaceutical compositions are preferably in unit dosage form of about 0.001 to 100 µg/kg (antigen/body weight) and more preferably 0.01 to 10 µg/kg and most preferably 0.1 to 1 µg/kg, 1 to 3 times with an interval of about 1 to 6 weeks between immunizations.

Pharmaceutical compositions are preferably in unit dosage form of about 0.1 µg to 10 mg and more preferably 1µg to 1 mg and most preferably 10 to 100 µg 1 to 3 times with an interval of about 1 to 6 week intervals between immunizations.

In one embodiment, polynucleotides are those illustrated in SEQ ID Nos: 1, 3, 5, 7, 9, 11, 13, 15, 17, 19, 21, 23, 25, 27, 29, 31, 33, 35 and 37 which may include the open reading frames (ORF), encoding the polypeptides of the invention.

It will be appreciated that the polynucleotide sequences illustrated in the figures may be altered with degenerated codons yet still encode the polypeptide of the invention. Accordingly the present invention further provides polynucleotide herein above described (or the complement sequence thereof) having 50% identity between sequences. In one embodiment, at least 70% identity between sequences. In one embodiment, at least 75% identity between sequences. In one embodiment, at least 80% identity between sequences. In one embodiment, at least 85% identity between sequences. In one embodiment, at least 90% identity between sequences. In a further embodiment, polynucleotides are hybridizable under stringent conditions, i.e. having at least 95% identity. In a further embodiment, more than 97% identity.

Suitable stringent conditions for hybridation can be readily determined by one of skilled in the art (see for example Sambrook *et al*., (1989) Molecular cloning: A Laboratory Manual, 2^{nd} ed, Cold Spring Harbor, N.Y.; Current Protocols in Molecular Biology, (1999) Edited by Ausubel F.M. *et al*., John Wiley & Sons, Inc., N.Y.).

In a further embodiment, the present invention provides polynucleotides that hybridize under stringent conditions to either
(a) a DNA sequence encoding a polypeptide or
(b) the complement of a DNA sequence encoding a polypeptide;
   wherein said polypeptide comprises SEQ ID NO: 2, 4, 6, 8, 10, 12, 14, 16, 18, 20, 22, 24, 26, 28, 30, 32, 34, 36, 38 or fragments or analogs thereof.
   In a further embodiment, the present invention provides polynucleotides that hybridize under stringent conditions to either
(c) a DNA sequence encoding a polypeptide or
(d) the complement of a DNA sequence encoding a polypeptide;
wherein said polypeptide comprises SEQ ID NO: 2, 4, 6, 8, 10, 12, 14, 16, 18, 20, 22, 24, 26, 28, 30, 32, 34, 36 or 38.

In a further embodiment, the present invention provides polynucleotides that hybridize under stringent conditions to either
(a) a DNA sequence encoding a polypeptide or
(b) the complement of a DNA sequence encoding a polypeptide;
   wherein said polypeptide comprises at least 10 contiguous amino acid residues from a polypeptide comprising SEQ ID NO: 2, 4, 6, 8, 10, 12, 14, 16, 18, 20, 22, 24, 26, 28, 30, 32, 34, 36, 38 or fragments or analogs thereof.
   In a further embodiment, the present invention provides polynucleotides that hybridize under stringent conditions to either
(c) a DNA sequence encoding a polypeptide or
(d) the complement of a DNA sequence encoding a polypeptide;
wherein said polypeptide comprises at least 10 contiguous amino acid residues from a polypeptide comprising SEQ ID NO: 2, 4, 6, 8, 10, 12, 14, 16, 18, 20, 22, 24, 26, 28, 30, 32, 34, 36 or 38.

As will be readily appreciated by one skilled in the art, polynucleotides include both DNA and RNA.

The present invention also includes polynucleotides complementary to the polynucleotides described in the present application.

In a further aspect, polynucleotides encoding polypeptides of the invention, or fragments or analogs thereof, may be used in a DNA immunization method. That is , they can be incorporated into a vector which is replicable and expressible upon injection thereby producing the antigenic polypeptide *in vivo.* For example polynucleotides may be incorporated into a plasmid vector under the control of the CMV promoter which is functional in eukaryotic cells. Preferably, the vector is injected intramuscularly.

According to another aspect, there is provided a process for producing polypeptides of the invention by recombinant techniques by expressing a polynucleotide encoding said polypeptide in a host cell and recovering the expressed polypeptide product. Alternatively, the polypeptides can be produced according to established synthetic chemical techniques, i.e. solution phase or solid phase synthesis of oligopeptides which are ligated to produce the full polypeptide (block ligation).

General methods for obtention and evaluation of polynucleotides and polypeptides are described in the following references: Sambrook *et al*., (1989) Molecular cloning: A Laboratory Manual, 2^{nd} ed, Cold Spring Harbor, N.Y.; Current Protocols in Molecular Biology, (1999) Edited by Ausubel F.M. *et al*., John Wiley & Sons, Inc., N.Y.; PCR Cloning Protocols, from Molecular Cloning to Genetic Engineering, (1997) Edited by White B.A., Humana Press, Totowa, New Jersey, 490 pages; Protein Purification, Principles and Practices, (1993) Scopes R.K., Springer-Verlag, N.Y., 3^{rd} Edition, 380 pages; Current Protocols in Immunology, (1999) Edited by Coligan J.E. *et al*., John Wiley & Sons Inc., N.Y..

For recombinant production, host cells are transfected with vectors which encode the polypeptide, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook *et al*., (1989) Molecular Cloning: A Laboratory manual, 2^{nd} ed., Cold Spring Harbor, N.Y.; Current Protocols in Molecular Biology, (1999) Edited by Ausubel F.M. *et al*., John Wikey & Sons, Inc., N.Y., incorporated herein by reference). Suitable promoters include but are not limited to LTR or SV40 promoter, E. coli lac, tac or trp promoters and the phage lambda P_{L} promoter. Vectors will preferably incorporate an origin of replication as well as selection markers, i.e. antibiotic resistance gene. Suitable bacterial vectors include pET, pQE70, pQE60, pQE-9, pD10 phagescript, PSIX174, pBluescript SK, pbsks, pNH8A, pNH16A, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial (i.e. E. coli, Bacillus subtilis, Streptomyces), fungal (i.e. Aspergillus niger, Aspergillus nidulins), yeast (i.e. Saccharomyces) or eukaryotic (i.e. CHO, COS).

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide: ammonium sulfate or ethanol precipitation, acid extraction, anion or cation exchange chromatography, phosphocellulose chromatography hydrophobic interaction chromatography, hydroxylapatite chromatography and lectin chromatography. Final purification may be achieved using HPLC.

According to a further aspect, the Chlamydial polypeptides of the invention may be used in a diagnostic test for Chlamydial infection, in particular C. pneumoniae infection. Several diagnostic methods are possible, for example detecting Chlamydia organism in a biological sample, the following procedure may be followed:
a) obtaining a biological sample from a host;
b) incubating an antibody or fragment thereof reactive with a Chlamydia polypeptide of the invention with the biological sample to form a mixture, and
c) detecting specifically bound antibody or bound fragment in the mixture which indicates the presence of Chlamydia.

Alternatively, a method for the detection of antibody specific to a Chlamydial antigen in a biological sample containing or suspected of containing said antibody may be performed as follows:
a) obtaining a biological sample from a host;
b) incubating one or more Chlamydial polypeptides of the invention or fragments thereof with the biological sample to form a mixture; and
c) detecting specifically bound antigen or bound fragment in the mixture which indicates the presence of antibody specific to Chlamydia.

One of skill in the art will recognize that this diagnostic test may take several forms, including an immunological test such as an enzyme-linked immunoadsorbent assay (ELISA), a radioimmunoassay or a latex agglutination assay, essentially to determine whether antibodies specific for the protein are present in an organism.

The DNA sequences encoding polypeptides of the invention may also be used to design DNA probes for use in detecting the presence of Chlamydia in a biological sample suspected of containing such bacteria. The detection method of this invention comprises:
a) obtaining the biological sample from a host;
b) incubating one or more DNA probes having a DNA sequence encoding a polypeptide of the invention or fragments thereof with the biological sample to form a mixture; and
c) detecting specifically bound DNA probe in the mixture which indicates the presence of Chlamydia bacteria.

The DNA probes of this invention may also be used for detecting circulating Chlamydia (i.e. Chlamydial nucleic acids)in a sample, for example using a polymerase chain reaction, as a method of diagnosing Chlamydia infections.

The probe may be synthesized using conventional techniques and may be immobilized on a solid phase or may be labelled with a detectable label. A preferred DNA probe for this application is an oligomer having a sequence complementary to at least about 6 contiguous nucleotides of the C. pneumoniae polypeptides of the invention.

Another diagnostic method for the detection of Chlamydia in a host comprises:
a) labelling an antibody reactive with a polypeptide of the invention or fragment thereof with a detectable label;
b) administering the labelled antibody or labelled fragment to the host; and
c) detecting specifically bound labelled antibody or labelled fragment in the host which indicates the presence of Chlamydia.

A further aspect of the invention is the use of the Chlamydia polypeptides of the invention as immunogens for the production of specific antibodies for the diagnosis and in particular the treatment of Chlamydia infection. Suitable antibodies may be determined using appropriate screening methods, for example by measuring the ability of a particular antibody to passively protect against Chlamydia infection in a test model. One example of an animal model is the mouse model described in the example herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal or preferably monoclonal. It may be specific for a number of epitopes associated with the C. pneumoniae polypeptides but is preferably specific for one.

A further aspect of the invention is the use of the antibodies directed to the polypeptides of the invention for passive immunization. One could use the antibodies described in the present application. Suitable antibodies may be determined using appropriate screening methods, for example by measuring the ability of a particular antibody to passively protect against chlamydia infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the chlamydia polypeptides but is preferably specific for one.

According to one aspect, the present invention provides the use of an antibody for treatment and/or prophylaxis of Chlamydial infections.

Unless otherwise defined, all technical and scientific terms used herein have the same meaning as commonly understood by one of ordinary skill in the art to which this invention belong. All publications, patent applications, patents and other references mentioned herein are incorporated by reference in their entirety. In case of conflict, the present specification including definitions will control. In addition, the materials, methods, and examples are illustrative only and not intended to be limiting.

### ISOLATION OF CHLAMYDIA POLYPEPTIDES AND IDENTIFICATION OF GENES ENCODING THESE POLYPEPTIDES

### ISOLATION OF INFECTION SPECIFIC CHLAMYDIA POLYPEPTIDES Bacterial strains

C. pneumoniae CWL-029 (American Culture Collection, Rockville, Maryland) was cultivated in HEp-2 and McCoy cells (American Culture Collection, Rockville, Maryland) using standard methods (Maass M. and Harig U. (1995) Evaluation of culture conditions used for isolation of Chlamydia pneumoniae. Clin. Microbiol. Infect. Dis. 103: 141-148; Roblin P.M., Dumornay W. and Hammerschlag M.R. (1992) Use of HEp-2 cells for improved isolation and passage of Chlamydia pneumoniae. J. Clin. Microbiol. 30: 1968-1971; Wong K.H., Skelton S.K. and Chan Y.K. (1992) Efficient culture of Chlamydia pneumoniae with cell lines derived from the human respiratory tract. J. Clin. Microbiol. 30: 1625-1630). Purified chlamydiae were obtained using Hypaque-M 76% (diatrizoate meglumine and diatrizoate sodium (Sanofi Winthrop, Laval, Quebec) density gradient centrifugation (Caldwell H.D., Kromhout J., and Schachter J. (1981) Purification and partial characterization of the outer membrane protein of Chlamydia trachomatis. Infect. Immun. 31: 1161-1176; Miyashita, M. and Matsumoto, A. (1992) Establishment of a particle-counting method for purified elementary bodies of Chlamydiae and evaluation of sensitivities of the IDEIA Chlamydia kit and DNA probe by using the purified elementary bodies. J. Clin. Microbiol. 30: 2911-2916). Escherichia coli DH5α (GIBCO BRL, Burlington, Ontario) was used as the host for recombinant DNA. E. coli XL1-Blue MRF' (Stratagene, LaJolla, California) was used as the host strain for infection with lambda ZAP Express phage vector. E. coli XLOLR (Stratagene) was used as the host strain for infection with in vivo excised filamentous lambda ZAP Express.

### Antisera

Rabbit and mice antisera against Chlamydia-infected McCoy cells were produced following three-week intervals subcutaneous immunization in presence of QuilA adjuvant (Cedarlane Laboratories Ltd, Hornby, Canada). Sera were collected 28 days after the tertiary immunization.

Four human antisera were selected on the basis of their reactivity by ELISA and Western blotting on heat-killed purified Chlamydiae.

### C. pneumoniae library construction and screening

C. pneumoniae CWL-029 DNA was extracted using the RNA/DNA Midi Kit (Qiagen). DNA was partially digested with *Tsp*509I and ligated to *Eco*RI digested λ-ZAP Express phage arms (Stratagene). The ligation was packaged in vitro with Gigapack extracts according to the manufacturer's recommendations (Stratagene). Recombinant phage were plated on E. coli XL1-Blue MRF' at density of 2.5 x 10⁴ PFU/150 mm (diameter) plate. Following eight hours incubation, the plates were overlaid with nitrocellulose disks and the resulting lifts were processed for immunoblotting with human and rabbit sera. Positive clones were picked and plaque-purified. Recombinant pBK-CMV plasmids coding for the chlamydial genes of interest were recovered from the purified bacteriophage using ExAssist filamentous helper phage and the lambda-resistant strain E. coli XLOLR by in vivo excision.

### Identification of antigens

SDS-PAGE and immunoblot analysis of lysates of these recombinant E. coli showed that each expressed one or more proteins that reacted with human or rabbit antisera.

### DNA sequencing and sequence analysis

The genomic clones were sequenced with the Taq DyeDeoxy Terminator Cycle Sequencing Kit (Applied Biosystem, Foster City, California). Several internal primers were designed to sequence further into the cloned inserts. Sequence assembly was performed using Sequencher software (Gene Codes Corporation, Ann Arbor, Michigan) and sequence analysis was performed with McVector software (Oxford Molecular Ltd., Campbell, California).

### EXAMPLE 1

This example illustrates the cloning of C. pneumoniae genes into expression vector

The coding region of C. pneumoniae genes BVH-CPN1 to BVH-CPN19 (SEQ ID NO:1 to NO: 19) were amplified by PCR (DNA Thermal Cycler GeneAmp PCR system 2400 Perkin Elmer, San Jose, CA) from genomic DNA of C. pneumoniae strain CWL-029 by using the following oligonucleotide primers that contained base extensions for the addition of restriction sites *Bam*HI (GGATCC), *Nco*I (CCATGG), *Nde*I (CATATG), *Not*I (GCGGCCGC) or *Xho*I (CTCGAG) (Table 1). PCR products were purified from agarose gel by using a QIAquick gel extraction kit from QIAgen following the manufacturer's instructions (Chatsworth, CA), and digested with appropriate restriction endonucleases (Pharmacia Canada Inc, Baie d'Urfé, Canada). The pET vectors (Novagen, Madison, Wisconsin) were digested likewise and purified from agarose gel using a QIAquick gel extraction kit from QIAgen (Chatsworth, California). The digested PCR products were ligated to the enzyme restricted pET expression vector. The ligated products were transformed into E. coli strain DH5α [φ80d*lac*ZΔM15 Δ(*lac*ZYA-*arg*F)U169 *end*A1 *rec*A1 *hsd*R17(r_{K}-m_{K}+) *deo*R *thi*-1 *sup*E44 λ⁻ *gyr*A96 *rel*A1] (Gibco BRL, Gaithersburg, Maryland) according to the method of Simanis (Hanahan D. (1985) In: DNA Cloning. D.M. Glover, ed., IRL Press, Washington D.C. vol. 1 pp. 109-135). Recombinant plasmid (rpET) containing genes were purified using a QIAgen kit (Chatsworth, California) and DNA inserts were sequenced (Taq Dye Deoxy Terminator Cycle Sequencing kit, ABI, Foster City, California).

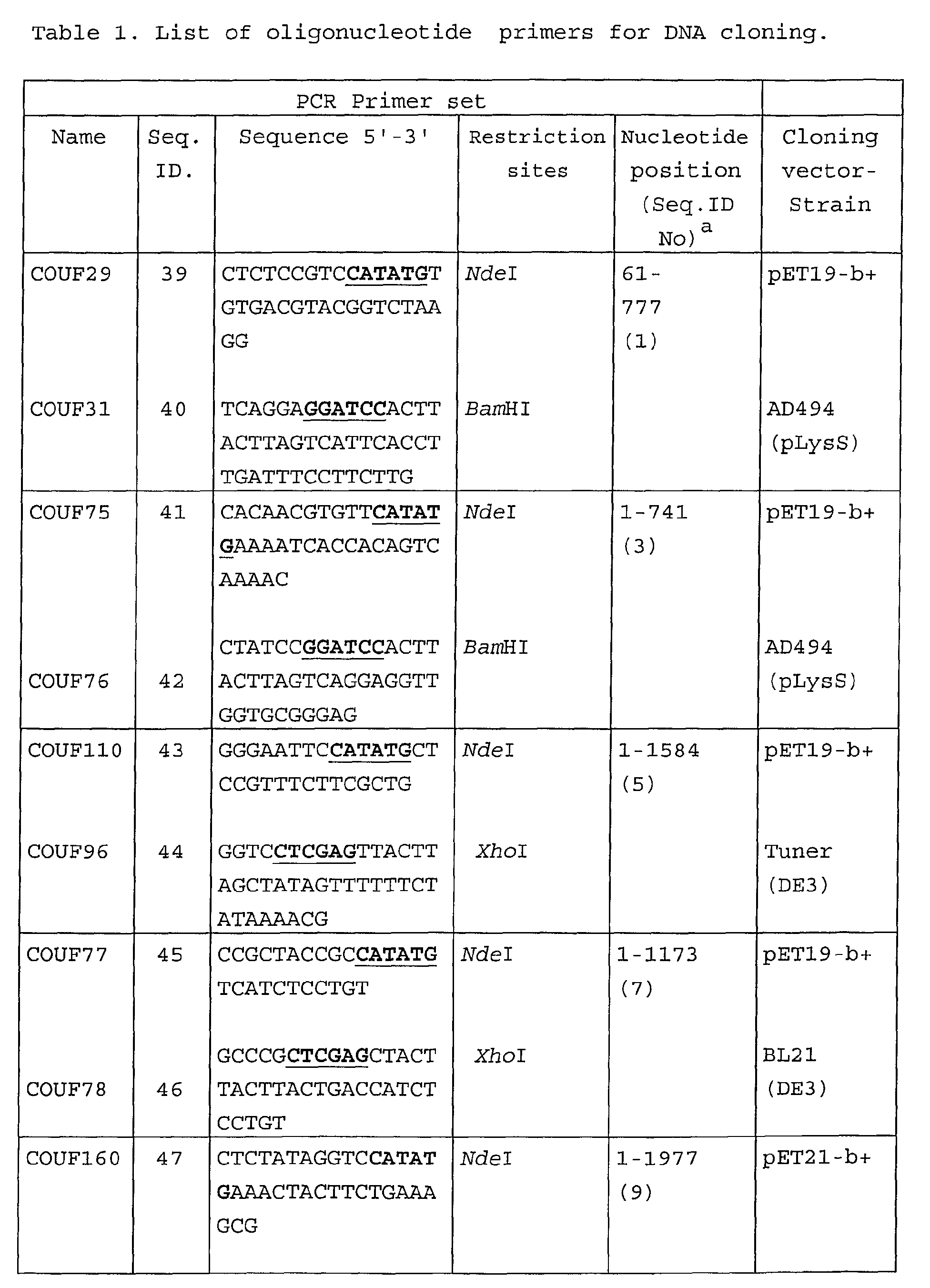

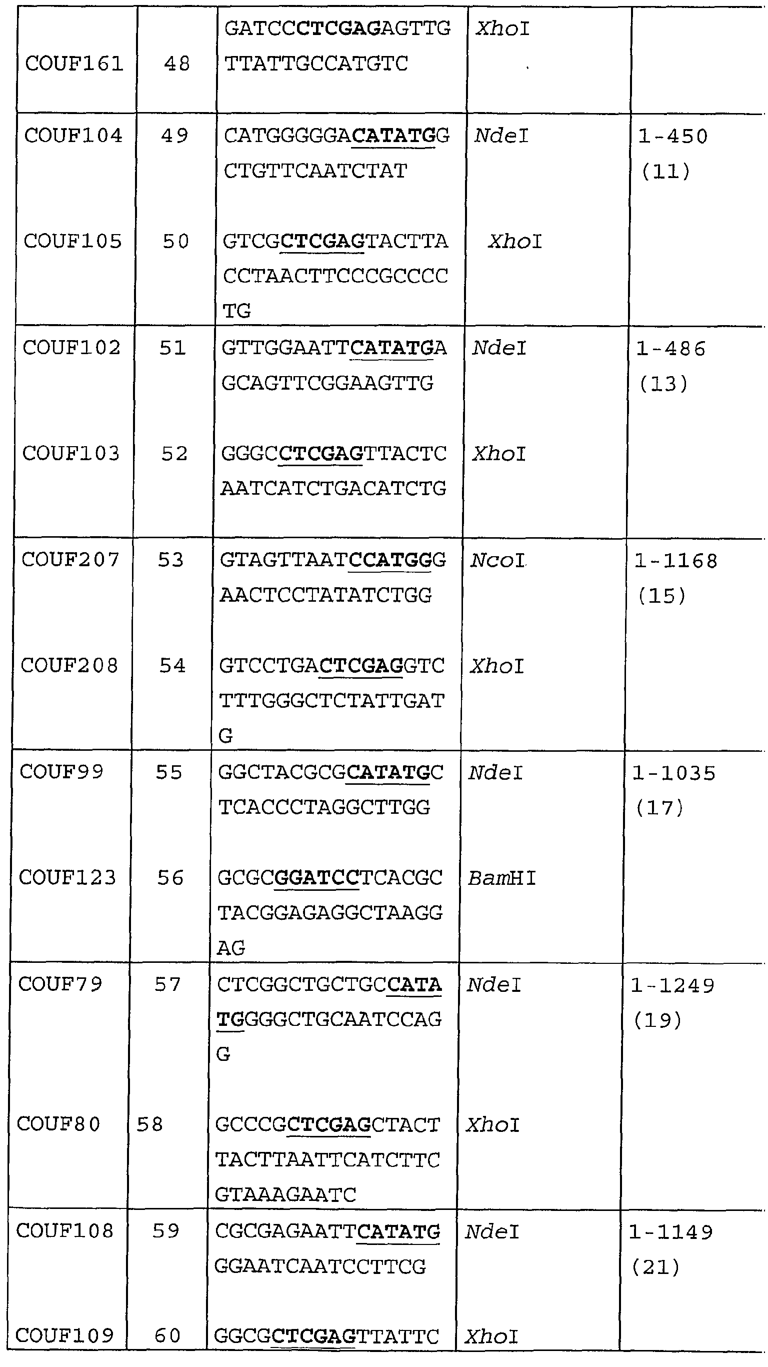

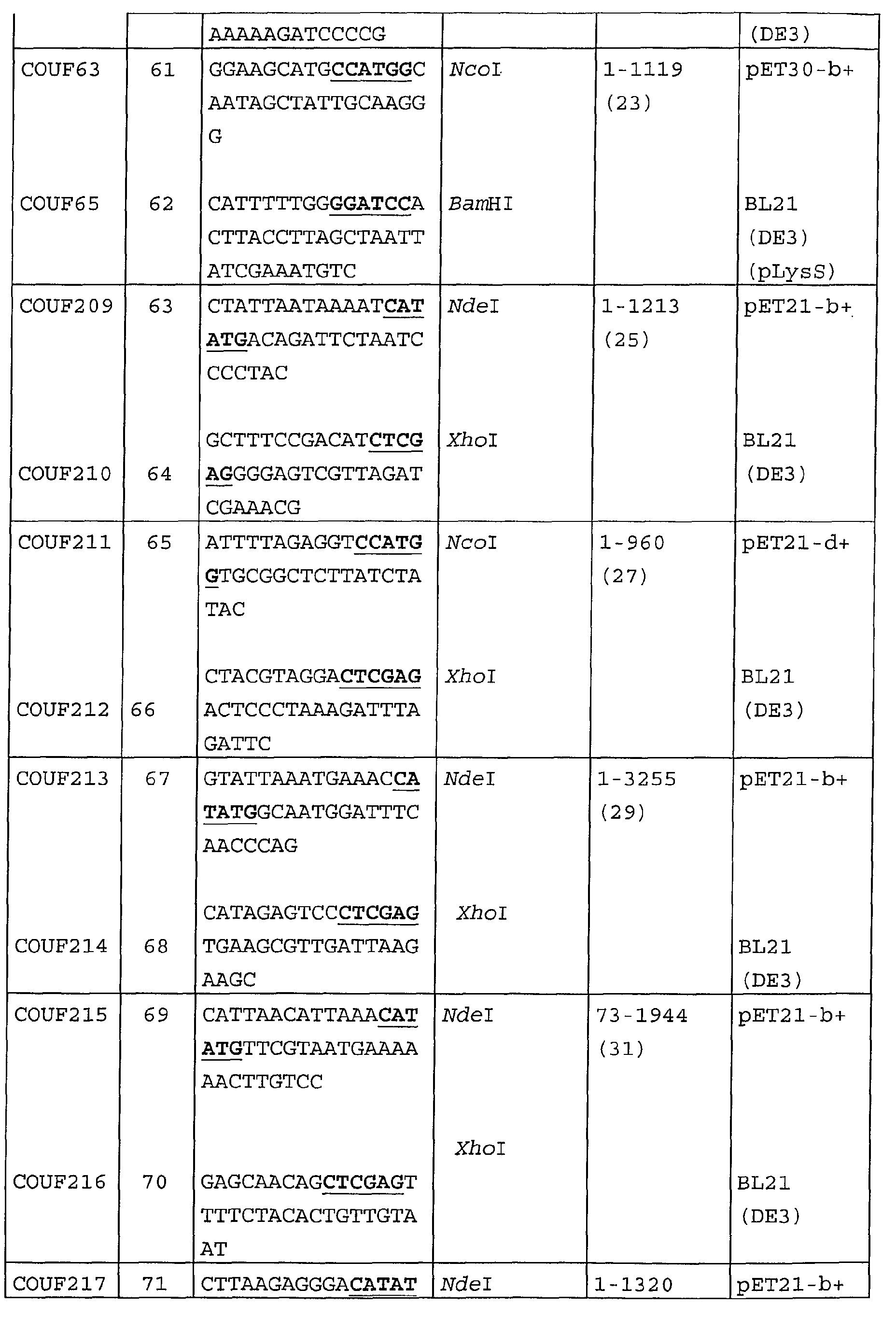

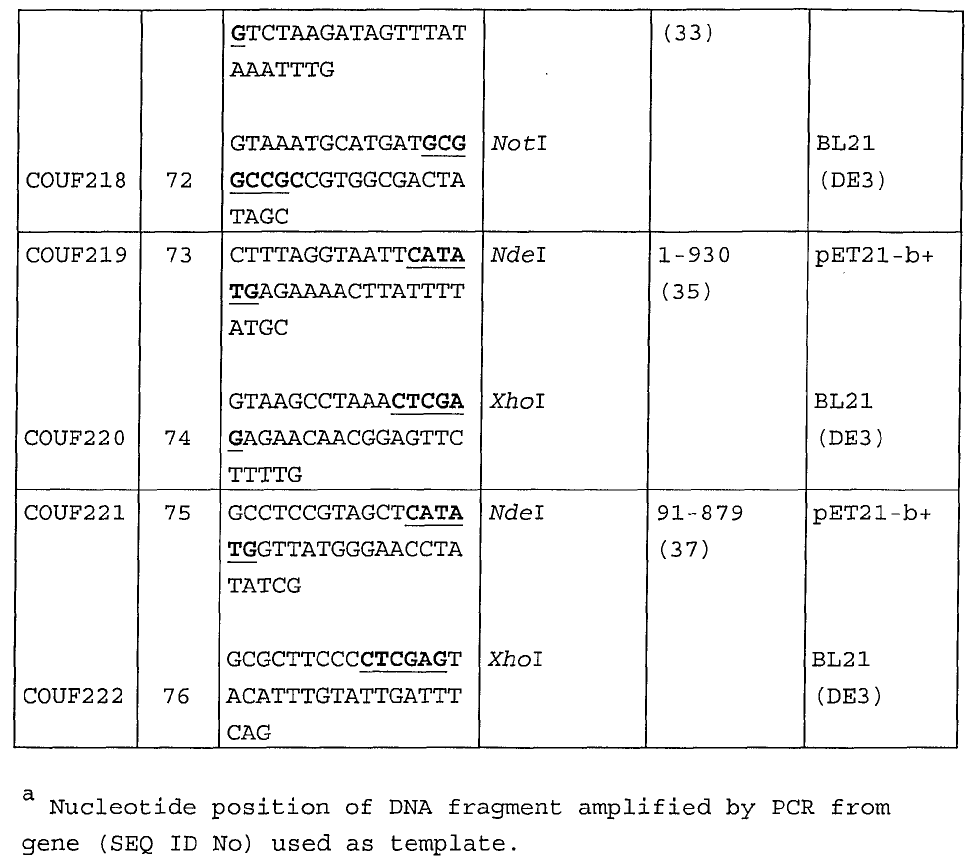

### EXAMPLE 2

This example describes the PCR amplification and sequencing of BVH-CPN1 gene from other C. pneumoniae strains and the evaluation of the level of molecular conservation of this gene.

C. pneumoniae CWL-029 and CM-1 isolates were provided by the American Culture Collection (Rockville, Maryland); C. pneumoniae TW-183 and AR39 isolates were provided by the Washington Research Foundation (Seattle, Washington); C. pneumoniae strains YK-41 and KKpn-1 were provided by Akira Matsumoto (Okayama,Japan); C. pneumoniae strains WI-02 to WI-06 were provided by The Center of Disease Control (Atlanta, Georgia); C. pneumoniae strain UZG-1 was provided by Jacobus M. Ossewaarde (Bilthoven, The Netherlands). The respective coding region of C. pneumoniae gene BVH-CPN1 from these strains were amplified by PCR (DNA Thermal Cycler GeneAmp PCR system 2400 Perkin Elmer, San Jose, California) from genomic DNA using the following oligos: COUF29 (5'-CTCTCCGTCCATATGTGTGACGTACGGTCTAAGG-3'); COUF31 (5'-TCAGGAGGATCCACTTACTTAGTCATTCACCTTGATTTCCTTCTTG -3'). PCR products were purified from agarose gel using a QIAquick gel extraction kit from QIAgen following the manufacturer's instructions (Chatsworth, California) and the DNA insert were sequenced (Taq Dye Deoxy Terminator Cycle Sequencing kit, ABI, Foster City, California). Length of PCR fragments generated for all these strains was identical and pairwise comparisons of the nucleotides and the predicted amino acid sequences from four of these strains (CWL-029, KKpn-1, Wi-04 and UZG-1) revealed 100% identity. (PCR fragments of other strains have not been sequenced).

### EXAMPLE 3

This example illustrates the production and purification of recombinant C. pneumoniae BVH-CPN1 polypeptide.

The recombinant pET19 plasmid with BVH-CPN1 gene corresponding to the SEQ ID NO: 1 was used to transform by electroporation (Gene Pulser II apparatus, BIO-RAD Labs, Mississauga, Canada) E. coli strain AD494 (DE3) pLysS [Δ*araleu7697 ΔlacX74 ΔphoA PvuII phoR ΔmalF3 F'* [*lac*^{*+*} (*lacI*^{*q*}) *pro*] trxB::Kan (DE3) pLysS(Cm^{r})] (Novagen, Madison, Wisconsin). In this strain of E. coli, the T7 promotor controlling expression of the recombinant protein is specifically recognized by the T7 RNA polymerase (present on the λDE3 prophage) whose gene is under the control of the lac promotor which is inducible by isopropyl-β-d-thiogalactopyranoside (IPTG). The transformant AD494(DE3)pLysS/rpET19 was grown at 37°C with agitation at 250 rpm in LB broth (peptone 10g/L, yeast extract 5g/L, NaCl 10g/L) containing 100 µg/ml of carbenicillin (Sigma-Aldrich Canada Ltd., Oakville, Canada), 15 µg/ml of kanamycin (Sigma) and 30 µg/ml of chloramphenicol (Sigma) until the A₆₀₀ reached a value of 0.5. In order to induce the production of C. pneumoniae BVH-His•Tag-CPN1 recombinant protein, the cells were incubated for 3 additional hours at 30 °C in presence of IPTG at a final concentration of 1 mM. Induced cells from a 500 mL culture were pelleted by centrifugation and frozen at -70°C.

The purification of the recombinant proteins from the soluble cytoplasmic fraction of IPTG-induced AD494(DE3)pLysS/rpET19 was done by affinity chromatography based on the properties of the His•Tag sequence (10 consecutive histidine residues) to bind to divalent cations (Ni²⁺) immobilized on the His•Bind metal chelation resin. Briefly, the pelleted cells obtained from a 500 mL culture induced with IPTG was resuspended in lysis buffer (20 mM Tris, 500 mM NaCl, 5 mM imidazole, pH 7.9) containing 1 mM of (Phenylmethylsulfonyl fluoride (PMSF; Sigma), sonicated and centrifuged at 16,000 X g for 30 min to remove debris. The supernatant was deposited on a Ni-NTA agarose column (QIAgen, Mississauga, Ontario, Canada). The C. pneumoniae BVH-His•Tag-CPN1 recombinant protein was eluted with 500 mM imidazole-500mM NaCl-20 mM Tris pH 7.9. The removal of the salt and imidazole from the sample was done by dialysis against PBS at 4°C. The quantity of recombinant protein obtained from the soluble fraction of E. coli was estimated by MicroBCA (Pierce, Rockford, Illinois).

### EXAMPLE 4

This example illustrates the analysis of the immunogenicity and the protective ability of mice against C. pneumoniae infection following immunization with BVH-CPN1.

Groups of 7 female BALB/c mice (Charles River, Ontario, Canada) were immunized intranasally four times at two-week intervals with 20 µg of affinity purified C. pneumoniae His•Tag recombinant protein in presence of choleric toxin (CT; 1 µg) adjuvant or the E. coli heat labile toxin (LT; 1 µg) adjuvant (Cedarlane Laboratories Ltd, Hornby, Canada) or, as control, with adjuvant alone in PBS. Subcutaneous immunizations were done also in presence of QuilA (15 µg) (Cedarlane Laboratories Ltd, Hornby, Canada),three times at two-week intervals. Blood samples were collected from the orbital sinus on day prior to each immunization and 14 days following the third (day 42) or fourth (day 56) injection. Antibody titers were determined by microimmunofluorescence on confluent HEp-2 monolayers cells cultured into microculture plates (96 wells) and infected with 0.5 inclusion-forming unit (IFU) of C. pneumoniae CWL-029 per cell. The secondary antibody used was a goat anti-mouse IgG + IgM (H+L) (Fc specific) conjugated to fluorescein isothiocyanate (FITC) (Jackson Immunoresearch Labs, Mississauga, Ontario). The reactive titer of an antiserum was defined as the reciprocal of the smaller dilution showing fluorescent signal. To investigate whether these immune responses elicited by BVH-CPN1 was functionally significant, in vivo protective efficacy was evaluated 28 days later in mice challenged intranasally with approximately 2 x 10⁶ IFU of the C. pneumoniae strain CWL-029. To measure the effectiveness of vaccination to limit the in vivo growth of Chlamydia, 5 days following the lung infection bacterial, the bacterial clearance is provided on intact lungs following excision and homogenization in a tissue homogeniser in presence of conservation buffer (7.5% sucrose-3.8mM KH₂PO₄-7mM K₂HPO₄-5mM glutamic acid). Serial dilutions of supernatants of these lung homogenates were plated on HEp-2 monolayers cells (Epermidoid carcinoma from human larynx; American Type Culture Collection, Rockville, Maryland) for IFU determination. Mice injected with adjuvant only were used as negative controls.

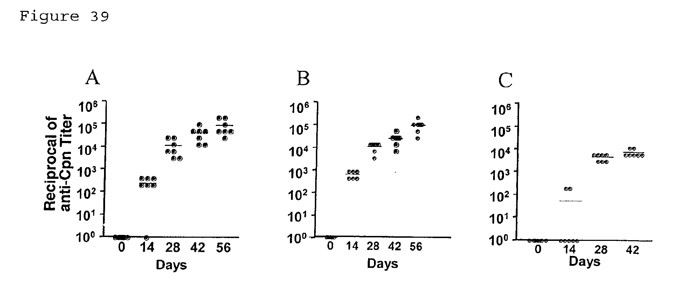

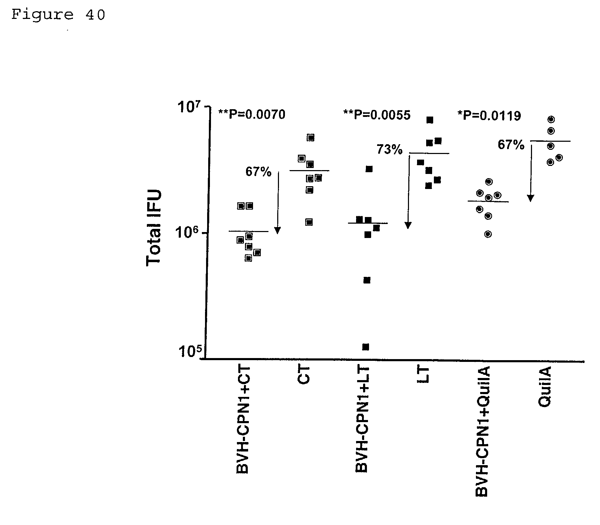

The results of immunogenicity study are illustrated in figure 39. As shown in figure 39, the final bleed sera obtained from mice intranasally immunized with BVH-CPN1 in presence of CT or LT are equivalent. By comparison, subcutaneous immunization in presence of QuilA gave a 10 fold lower titer following the third immunization. However, titer variations did not seem to have an effect on protective ability of mice as shown in figure 40. Mice, intranasally or subcutaneously immunized with BVH-CPN1 had chlamydial lung titers that were over 3 to 3.6-fold lower than those of control mice immunized with adjuvant only, giving a protection level ranged from 67 to 73%.

### EXAMPLE 5

This example illustrates the accessibility to antibodies of the BVH-CPN1 protein at the surface of C. pneumoniae strain CWL-029.

A pellet of purified chlamydiae (1 x 10⁸ IFU) was resuspended in 500 µl of polyvalent sera elicited by mice immunized with the BVH-CPN1 polypeptide. The suspension was incubated while shaking for 6 hours at 4 °C. After two washes with PBS, the chlamydiae were incubated for 6 hours at 4 °C with a goat anti-mouse IgG gold conjugated (12 nm) antibody (Jackson Immunoresearch Labs, Mississauga, Ontario). After two washes with ammonium acetate (0.1 M), the pellet was resuspended in 50 µL of ammonium acetate (0.1 M). Chlamydiae were stained with uranyl acetate (2%) and viewed in an electron microscope (Phillips EM 300, Phillips, Germany) at 60kV. Micrographs were taken at a magnification of x92 400 and x148 500.

The electron micrographs of chlamydiae preparations showed the immunogold labelling of surface antigens on the reticulate forms. No gold particles were seen to be associated with chlamydiae that had been reacted with preimmune sera.

## Claims

1. An isolated polynucleotide comprising a polynucleotide chosen from:
(a) a polynucleotide encoding a polypeptide having at least 70% identity to a second polypeptide comprising a sequence chosen from: SEQ ID NOs: 2, 4, 6, 8, 10, 12, 14, 16, 18, 20, 22, 24, 26, 28, 30, 32, 34, 36 and 38 or fragments or analogs thereof;
(b) a polynucleotide encoding a polypeptide having at least 95% identity to a second polypeptide comprising a sequence chosen from: SEQ ID NOs: 2, 4, 6, 8, 10, 12, 14, 16, 18, 20, 22, 24, 26, 28, 30, 32, 34, 36 and 38 or fragments or analogs thereof;
(c) a polynucleotide encoding a polypeptide comprising a sequence chosen from: SEQ ID NOs: 2, 4, 6, 8, 10, 12, 14, 16, 18, 20, 22, 24, 26, 28, 30, 32, 34, 36 and 38 or fragments or analogs thereof;
(d) a polynucleotide encoding a polypeptide capable of generating antibodies having binding specificity for a polypeptide having a sequence chosen from SEQ ID NOs: 2, 4, 6, 8, 10, 12, 14, 16, 18, 20, 22, 24, 26, 28, 30, 32, 34, 36 and 38 or fragments or analogs thereof;
(e) a polynucleotide encoding an epitope bearing portion of a polypeptide having a sequence chosen from: SEQ ID NOs: 2, 4, 6, 8, 10, 12, 14, 16, 18, 20, 22, 24, 26, 28, 30, 32, 34, 36 and 38 or fragments or analogs thereof;
(f) a polynucleotide that is complementary to a polynucleotide in (a), (b), (c), (d) or (e).

2. The polynucleotide of claim 1, wherein said polynucleotide is DNA.

3. The polynucleotide of claim 1, wherein said polynucleotide is RNA.

4. The polynucleotide of claim 1 that hybridizes under stringent conditions to either
(a) a DNA sequence encoding a polypeptide or
(b) the complement of a DNA sequence encoding a polypeptide;
wherein said polypeptide comprises SEQ ID NO: 2, 4, 6, 8, 10, 12, 14, 16, 18, 20, 22, 24, 26, 28, 30, 32, 34, 36, 38 or fragments or analogs thereof.

5. The polynucleotide of claim 1 that hybridizes under stringent conditions to either
(a) a DNA sequence encoding a polypeptide or
(b) the complement of a DNA sequence encoding a polypeptide;
wherein said polypeptide comprises at least 10 contiguous amino acid residues from a polypeptide comprising SEQ ID NO: 2, 4, 6, 8, 10, 12, 14, 16, 18, 20, 22, 24, 26, 28, 30, 32, 34,36, 38 or fragments or analogs thereof.

6. A vector comprising the polynucleotide of any of claims 1 to 5, wherein said DNA is operably linked to an expression control region.

7. A host cell transfected with the vector of claim 6.

8. A process for producing a polypeptide comprising culturing a host cell according to claim 7 under conditions suitable for expression of said polypeptide.

9. An isolated polypeptide comprising a member chosen from:
(a) a polypeptide having at least 70% identity to a second polypeptide having an amino acid sequence chosen from: SEQ ID NOs: 2, 4, 6, 8, 10, 12, 14, 16, 18, 20, 22, 24, 26, 28, 30, 32, 34, 36,38 or fragments or analogs thereof;
(b) a polypeptide having at least 95% identity to a second polypeptide having an amino acid sequence chosen from: SEQ ID NOs: 2, 4, 6, 8, 10, 12, 14, 16, 18, 20, 22, 24, 26, 28, 30, 32, 34, 36, 38 or fragments or analogs thereof;
(c) a polypeptide comprising a sequence chosen from: SEQ ID NOs: 2, 4, 6, 8, 10, 12, 14, 16, 18, 20, 22, 24, 26, 28, 30, 32, 34, 36, 38 or fragments or analogs thereof;
(d) a polypeptide capable of generating antibodies having binding specificity for a polypeptide having a sequence chosen from SEQ ID NOs: 2, 4, 6, 8, 10, 12, 14, 16, 18, 20, 22, 24, 26, 28, 30, 32, 34, 36, 38 or fragments or analogs thereof;
(e) an epitope bearing portion of a polypeptide having a sequence chosen from SEQ ID NOs: 2, 4, 6, 8, 10, 12, 14, 16, 18, 20, 22, 24, 26, 28, 30, 32, 34, 36, 38 or fragments or analogs thereof;
(f) the polypeptide of (a), (b), (c), (d) or (e) wherein the N-terminal Met residue is deleted;
(g) the polypeptide of (a), (b), (c), (d) or (e) wherein the secretory amino acid sequence is deleted.

10. A chimeric polypeptide comprising two or more polypeptides having a sequence chosen from SEQ ID NOs: 2, 4, 6, 8, 10, 12, 14, 16, 18, 20, 22, 24, 26, 28, 30, 32, 34, 36, 38 or fragments or analogs thereof; provided that the polypeptides are linked as to formed a chimeric polypeptide.

11. A pharmaceutical composition comprising a polypeptide according to claim 9 or 10 and a pharmaceutically acceptable carrier, diluent or adjuvant.

12. Use of a polypeptide according to claim 9 or 10 for the production of a medicament for the prophylactic or therapeutic treatment of Chlamydial bacterial infection in a host susceptible to Chlamydiae infection.

13. Use of a composition according to claim 11 for the production of a medicament for the prophylactic or therapeutic treatment of Chlamydial bacterial infection in a host susceptible to Chlamydiae infection.

14. Use according to claim 12 or 13 wherein the host is an animal.

15. Use according to claim 12 or 13 wherein the host is a human.

16. Use according to claim 12 or 13 wherein said bacterial infection is caused by Chlamydia pneumoniae.

17. Use according to claim 12 or 13 wherein said bacterial infection is caused by Chlamydia psittaci.

18. Use according to claim 12 or 13 wherein said bacterial infection is caused by Chlamydia trachomatis.

19. Use according to claim 12 or 13 wherein said infection causes sinusitis, pharyngitis, bronchitis, pneumonitis, asthmatic bronchitis adult-onset asthma, chronic obstructive pulmonary diseases (CDPD), atherogenesis or atherosclerosis.

20. Use of a polypeptide according to claim 9 or 10 for the production of a diagnostic agent of Chlamydial bacterial infection in a host susceptible to Chlamydiae infection.

21. Use of a composition according to claim 11 for the production of a diagnostic agent of Chlamydial bacterial infection in a host susceptible to Chlamydiae infection.

22. A method for diagnosis of Chlamydia infection in a host susceptible to Chlamydia infection comprising
(a) obtaining a biological sample from a host;
(b) incubating an antibody or fragment thereof reactive with a Chlamydia polypeptide of claim 9 or 10 with the biological sample to form a mixture; and
(c) detecting specifically bound antibody or bound fragment in the mixture which indicates the presence of Chlamydia.

23. A method for diagnosis of Chlamydia infection in a host susceptible to Chlamydia infection comprising
(a) obtaining a biological sample from a host;
(b) incubating one or more Chlamydia polypeptide of claim 9 or 10 or fragments thereof with the biological sample to form a mixture; and
(c) detecting specifically bound antigen or bound fragment in the mixture which indicates the presence of antibody specific to Chlamydia.

24. Kit comprising a polypeptide according to claim 9 or 10 for detection or diagnosis of Chlamydia infection.
